# EUROPEAN PATENT SPECIFICATION

(11) **EP 2 968 612 B1**
(45) Date of publication and mention of the grant of the patent: **14.02.2018**
(21) Application number: 14775545.8
(22) Date of filing: 12.03.2014
(51) Int. Cl.: A61K 48/00, A61P 27/02, C12N 15/86

(54) **MODIFIED SOLUBLE VEGF RECEPTOR-1 GENES AND VECTORS FOR GENE THERAPY**
MODIFIZIERTE LÖSLICHE VEGF-REZEPTOR-1-GENE UND VEKTOREN ZUR GENTHERAPIE
GÈNES DE RECEPTEUR-1 VEGF SOLUBLE MODIFIÉ ET VECTEURS POUR UNE THÉRAPIE GÉNIQUE

(30) Priority: 14.03.2013 US 201361782450 P
(43) Date of publication of application: 20.01.2016
(73) Proprietor: Asklepios Biopharmaceutical, Inc., Chapel Hill, NC 27517 (US)
(72) Inventor: SAMULSKI, Richard, J., Chapel Hill, NC 27514 (US)
(74) Representative: ABG Patentes, S.L.
(86) International application number: PCT/US2014/024119
(87) International publication number: WO 2014/159546

(56) References cited:
- WO-A2-01/58494
- WO-A2-2007/149852
- US-A- 6 051 698
- LAI ET AL: "Long-term Evaluation of AAV-Mediated sFlt-1 Gene Therapy for Ocular Neovascularization in Mice and Monkeys", MOLECULAR THERAPY, NATURE PUBLISHING GROUP, GB, vol. 12, no. 4, 1 October 2005 (2005-10-01), pages 659-668, XP005078445, ISSN: 1525-0016, DOI: 10.1016/J.YMTHE.2005.04.022
- KUDLA GRZEGORZ ET AL: "High guanine and cytosine content increases mRNA levels in mammalian cells", PLOS BIOLOGY, PUBLIC LIBRARY OF SCIENCE, US, vol. 4, no. 6, 1 June 2006 (2006-06-01), pages e180/933-e180/942, XP002484439, ISSN: 1544-9173, DOI: 10.1371/JOURNAL.PBIO.0040180
- LAI, C. M. ET AL.: 'Long-term evaluation of AAV-mediated sFlt-1 gene therapy for ocular neovascularization in mice and monkeys' MOLECULAR THERAPY vol. 12, no. 4, 2005, pages 659 - 668, XP005078445
- AMBATI, B. K. ET AL.: 'Corneal avascularity is due to soluble VEGF receptor-1' NATURE vol. 443, no. 7114, 2006, pages 993 - 997, XP055004662
- SHEN, J. ET AL.: 'Suppression of ocular neovascularization with siRNA targeting VEGF receptor 1' GENE THERAPY vol. 13, no. 3, 2006, pages 225 - 234, XP002441146

## Description

### BACKGROUND OF THE INVENTION

### Field of the invention

The invention relates to modified soluble VEGF receptor-1 (also known as sFlt1) genes, nucleic acid vectors including the modified genes, optimized viral capsids including the modified genes, the modified genes for use in methods of treatment of diseases related to ocular neovascularization, such as macular degeneration.

### Discussion of Related Art

Leading causes of severe vision loss and blindness are ocular-related disorders wherein the vasculature of the eye is damaged or insufficiently regulated. Ocular-related diseases comprising a neovascularization aspect are many and include, for example, exudative age-related macular degeneration, diabetic retinopathy, corneal neovascularization, choroidal neovascularization, neovascular glaucoma, cyclitis, Hippel-Lindau Disease, retinopathy of prematurity, pterygium, histoplasmosis, iris neovascularization, macular edema, glaucoma-associated neovascularization, and the like.

Damage of the retina, i.e., retinal detachment, retinal tears, or retinal degeneration, is directly connected to vision loss. A common cause of retinal detachment, retinal tears, and retinal degeneration is abnormal, that being, uncontrolled vascularization of various ocular tissues.

It has been found that vascular endothelial growth factor (VEGF) is a major stimulatory factor for retinal neovascularisation. It is unlikely to be the only stimulatory factor but it is nevertheless the key factor involved. VEGF is upregulated by hypoxia and its levels are increased in the retina and vitreous of patients or laboratory animals with ischaemic retinopathies. Also, increased expression of VEGF in retinal photoreceptors stimulates neovascularisation in the retina and VEGF antagonists inhibit retinal or iris neovascularisation in animal models. For instance, Lai C.-M. et al., Molecular therapy, 2005; vol. 12 (4), 659:668, reported that the expression of a soluble form of the Flt1 VEGF receptor (sFlt1) is associated with the regression of neovascularization in the eyes of treated animals.

For many ocular-related disorders, no efficient therapeutic options currently are available. Laser photocoagulation involves administering laser burns to various areas of the eye and is used in the treatment of many neovascularization-linked disorders. Laser treatment does not guarantee that vision loss will be attenuated. In fact, many patients afflicted with age-related macular degeneration eventually experience severe vision loss in spite of treatment. Other treatment options for ocular-related disorders include thermotherapy, radiation therapy, surgery, e.g., macular translocation, removal of excess ocular tissue, drug therapy, and the like. However, in most cases, all available treatment options have limited therapeutic effect, require repeated, costly procedures, and/or are associated with dangerous side-effects.

Given the prevalence of ocular-related disorders, there remains a need for an effective prophylactic and therapeutic treatment of ocular-related disorders. Accordingly, the invention provides materials and methods for achieving a beneficial effect in the eye, such as inhibiting or reducing angiogenesis or preventing photoreceptor cell loss. This and other advantages of the invention will become apparent from the detailed description provided herein.

### SUMMARY OF THE INVENTION

The present invention provides a vector for use in a method for treating ocular neovascularization comprising delivering to target cells in the eye of a subject in need of treatment the vector, which comprises a promoter sequence in operable linkage with a polynucleotide sequence encoding an anti-angiogenesis gene product, wherein the anti-angiogenesis gene product is expressed in the target cells, thereby for use in treating ocular neovascularization in the subject.

The target cells are preferably retinal cells, more preferably retinal pigment epithelial cells, and the vector is preferably delivered to the target cells via direct subretinal injection

The present invention provides for modified soluble vascular endothelial growth factor (VEGF) receptor-1 genes (sFlt1), nucleic acid vectors including the modified genes, optimized viral capsids including the modified genes, and the modified genes for use in methods for the treatment of diseases caused ocular neovascularization, such as macular degeneration.

In a first aspect, the invention relates to an optimized sFlt1 gene for use in treating ocular disorder causing neovascularization in a human subject wherein the optimized gene has been modified to increase CG sequences and reduce cis motifs relative to a wild type gene, wherein the optimized gene is selected from the group consisting of SEQ ID NOs: 2 and 8.

In a second aspect, the invention relates to a virus vector comprising the optimized gene of the first aspect.

In a third aspect, the invention relates to a process of expressing an optimized sFlt1 peptide comprising:
transfecting a cell with polynucleotide that encodes the optimized sFlt1 peptide to produce a transformed host cell, wherein the polynucleotide encoding the optimized sFlt1 gene has been modified to increase CG sequences and reduce cis motifs relative to a wild type gene wherein the polynucleotide encoding the optimized sFlt1 gene has been selected from the group consisting of SEQ ID NOs: 2 and 8; and
maintaining the transformed host cell under biological conditions sufficient for expression of the peptide.

A fourth aspect of the invention relates to a pharmaceutical composition comprising an admixture of:
an isolated or purified nucleic acid comprising the sequence of:
   SEQ ID NOs: 2, 8 or a complement thereof; and
   a pharmaceutically acceptable carrier.

In an alternative aspect, the present invention provides an expression vector comprising a polynucleotide that encodes an optimized sFlt1 gene or fragment thereof. In one embodiment the expression vector is an AW virus vector including the sequence of AAV1, AAV2 (SEQ ID NO: 22), AAV3, AAV4, AAV5, AAV6, AAV7, AAV8, AAV 9, AAV10, AAV11, AAV12 or chimeric variants thereof such as variant AAV2 Capsid 2.5 (SEQ ID NO. 10). Other modified sequences nucleotide sequence of modified AAV 1.1 capsid wherein amino acid residue 265 is deleted (SEQ ID NO: 12), nucleotide sequence of modified AAV 6.1 capsid wherein amino acid residue 265 is deleted (SEQ ID NO: 14), nucleotide sequence of modified AAV 6.3.1 capsid wherein amino acid residue 265 is deleted and amino acid residue 531 is changed from a Lys to a Glu (SEQ ID NO: 15). The nucleotide sequence of wildtype AAV 1 capsid is shown in (SEQ ID NO: 11) and the nucleotide sequence of wildtype AAV 6 capsid is set forth in (SEQ ID NO: 13).

Also disclosed is a recombinant host cell transfected with a polynucleotide that encodes an optimized sFlt1 peptide of the present invention and selected from any one of SEQ ID NOs: 2 to 8.

Other features and advantages of the invention will be apparent from the following detailed description, drawings and claims.

### BRIEF DESCRIPTION OF THE FIGURES

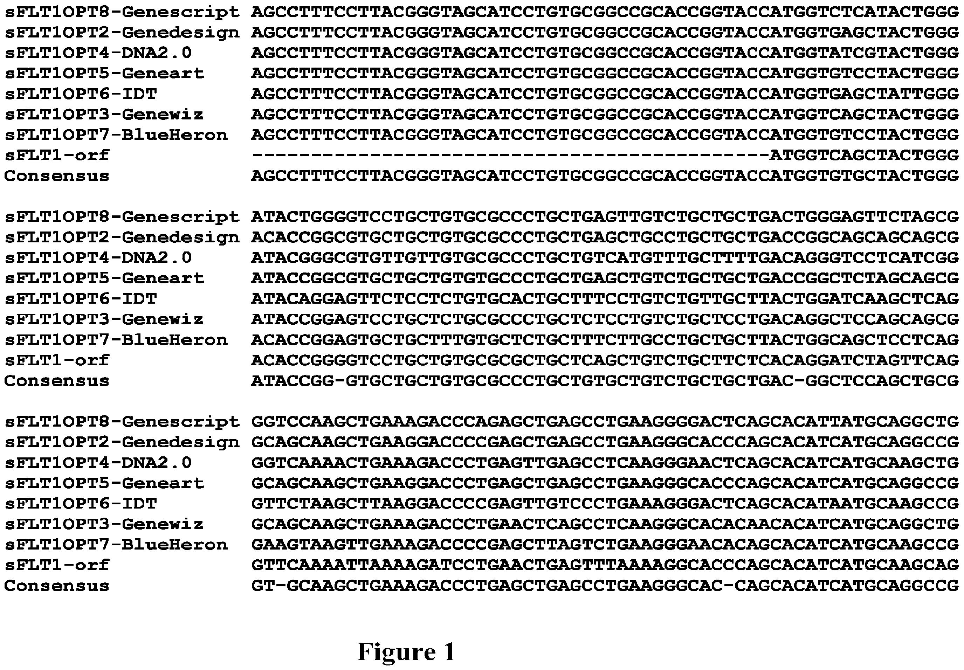

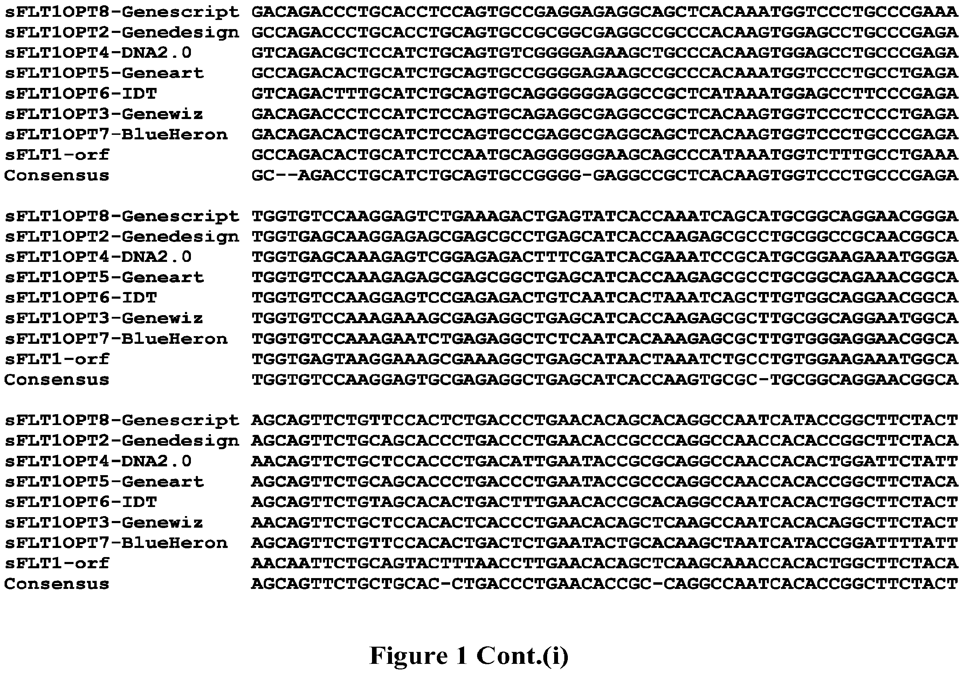

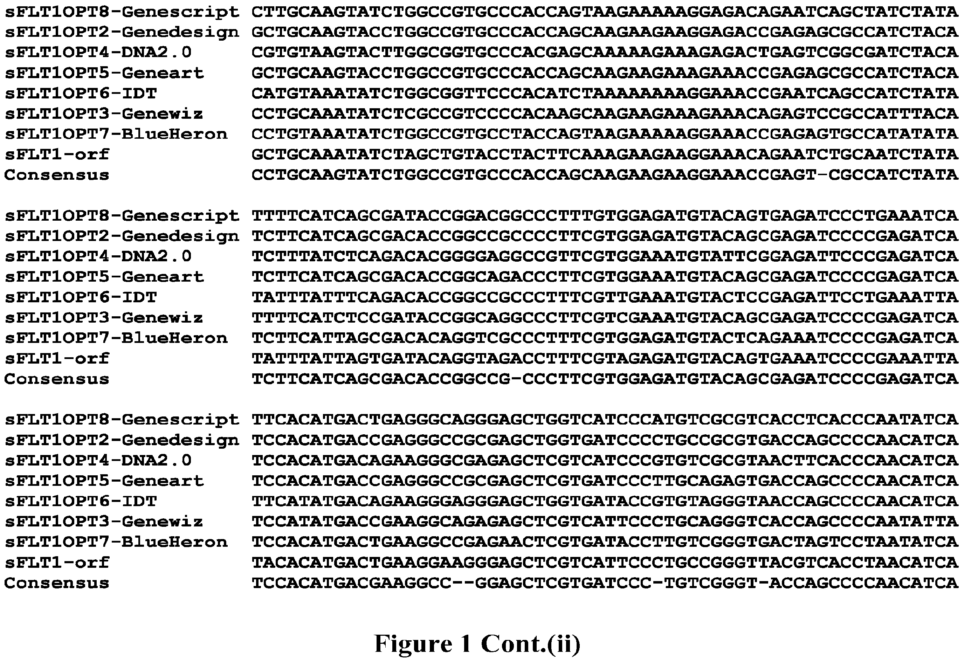

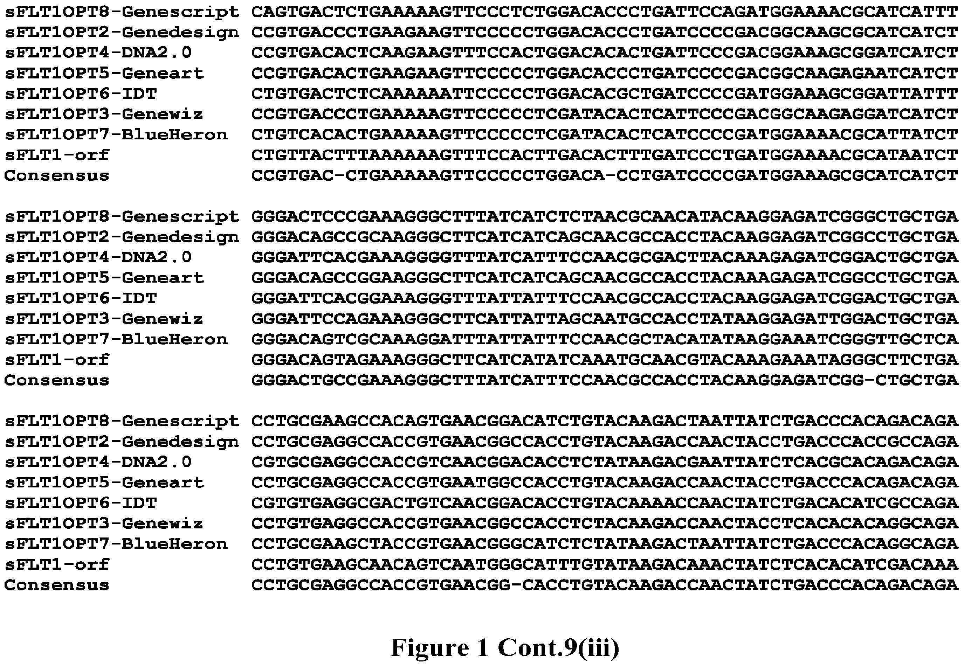

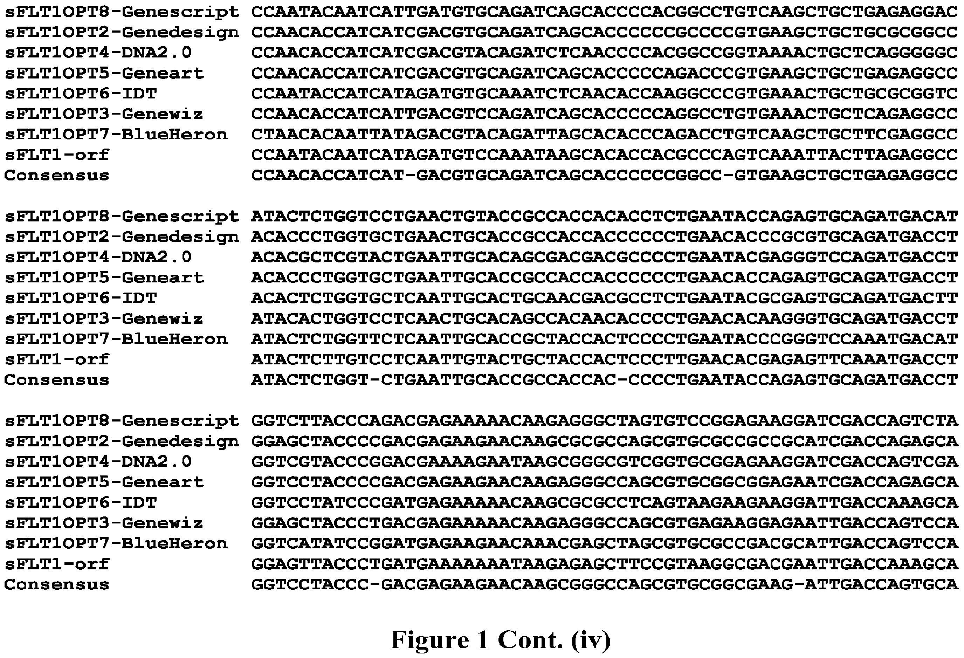

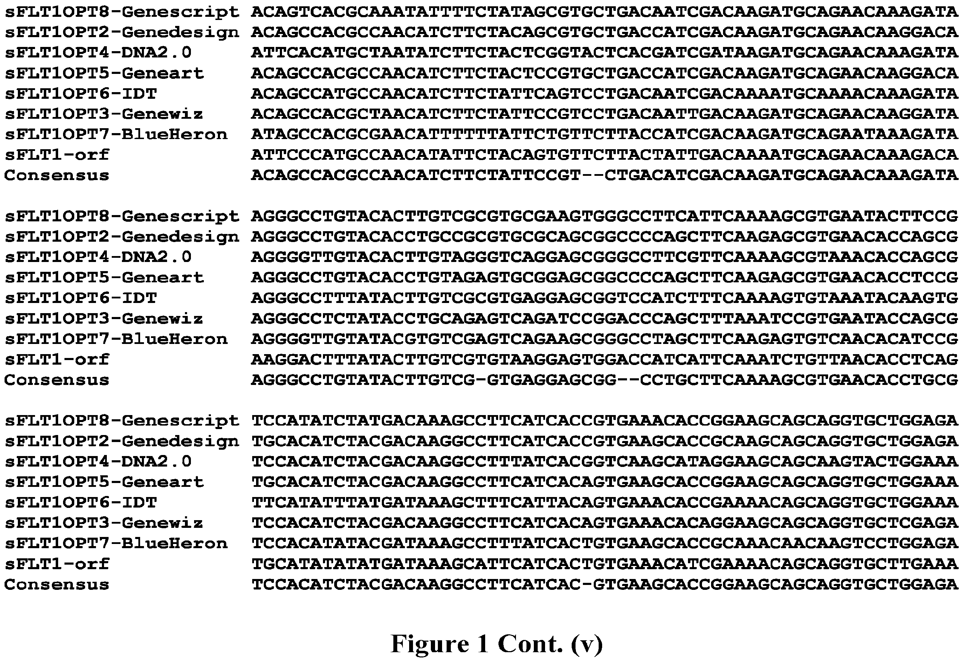

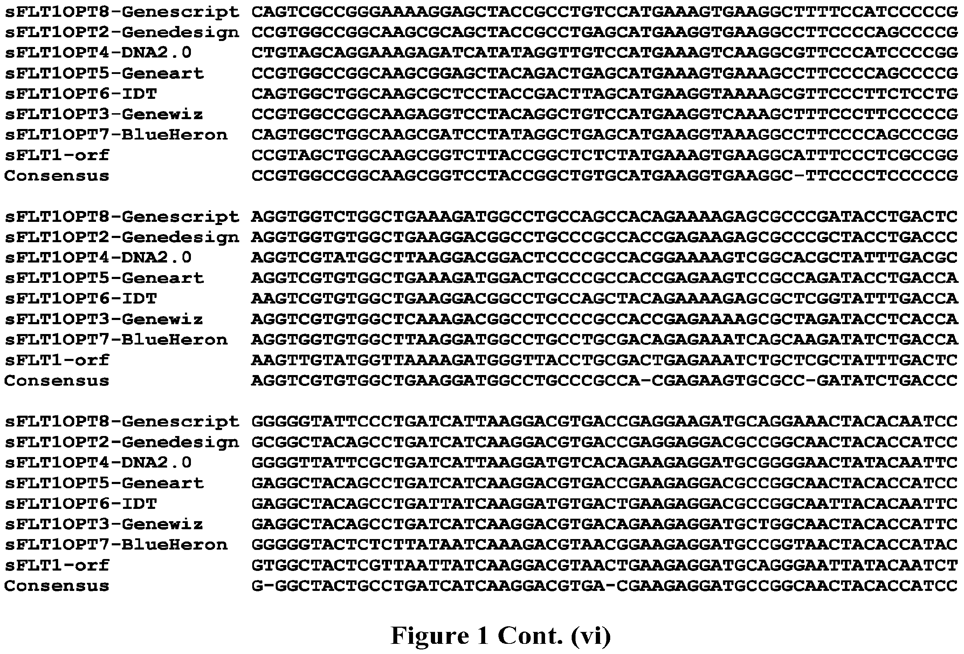

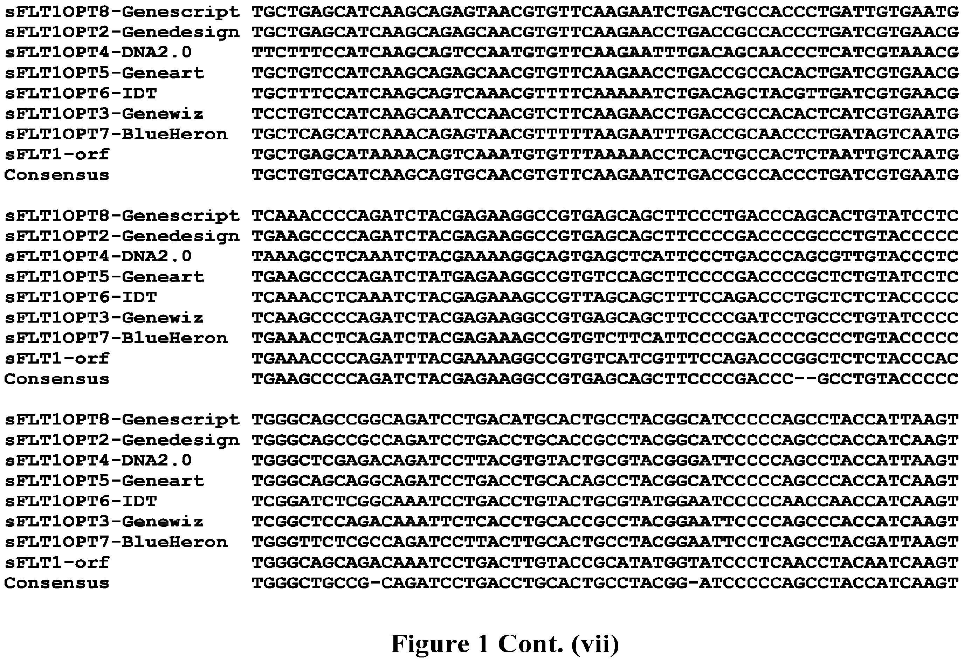

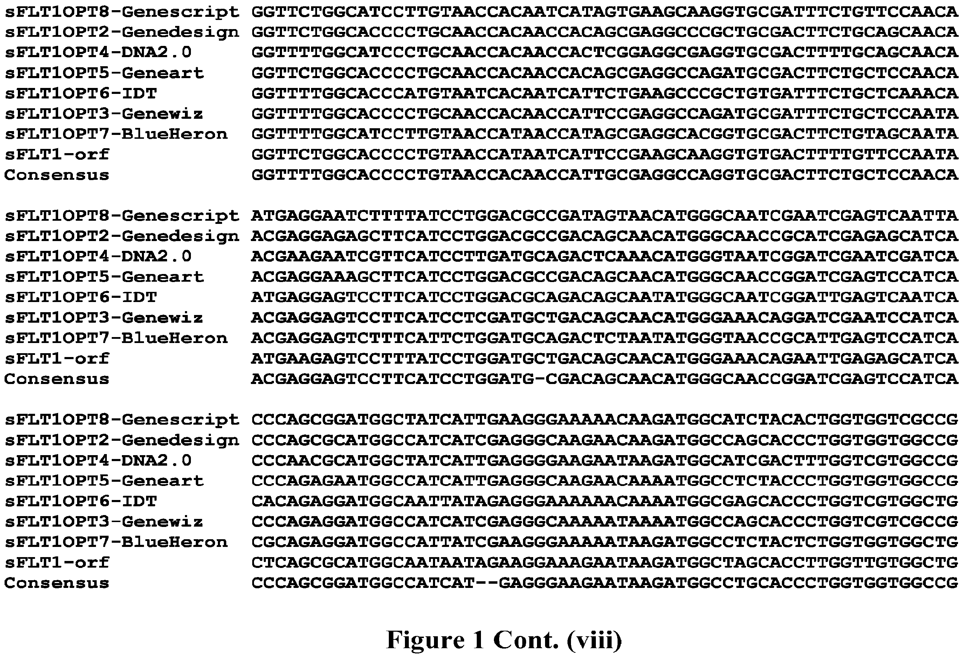

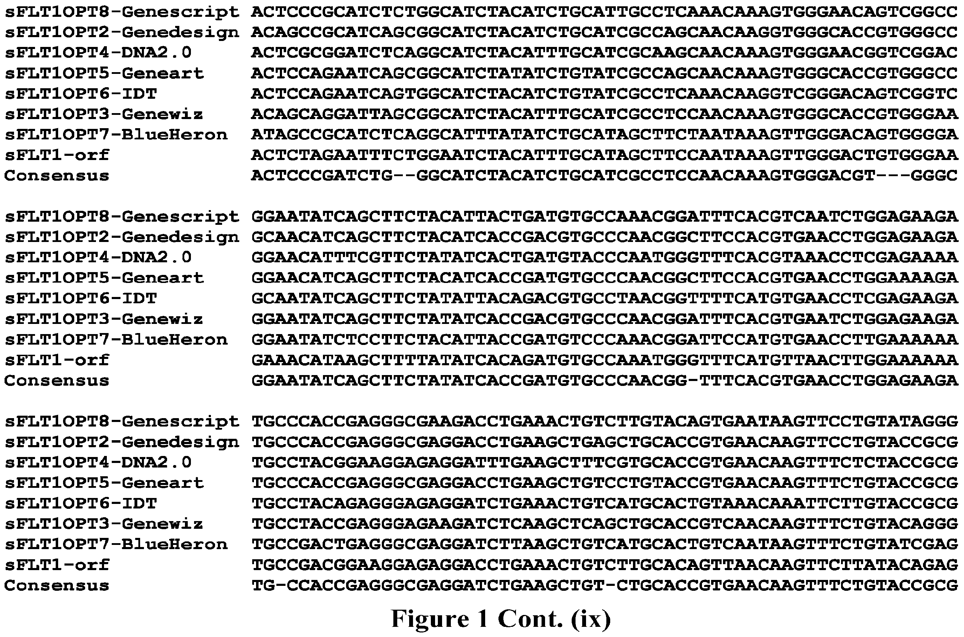

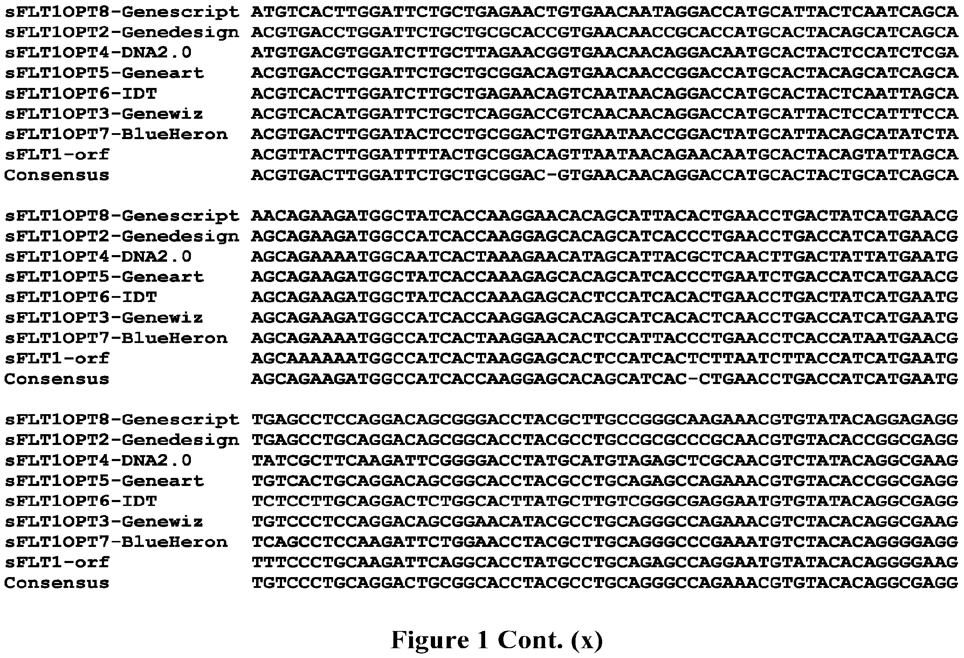

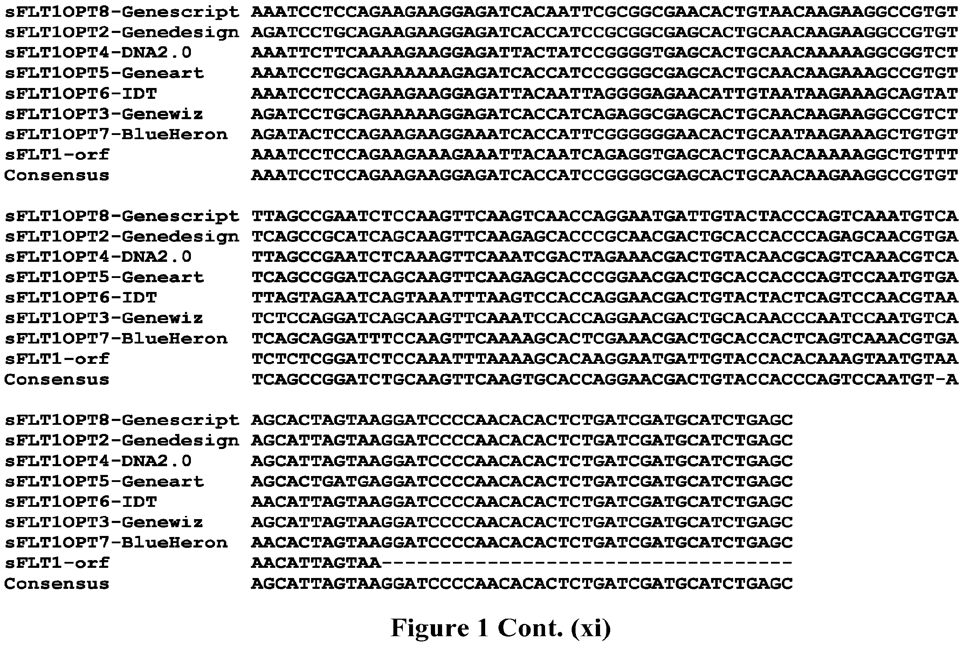

Figure 1 shows the sequences of eight optimized sFlt1 sequences, that being, SEQ ID NO: 1 (sFLT-orf unoptimized), SEQ ID NO: 2 (sFLT-OPT-2 GeneDesign); SEQ ID NO: 3 (sFLT-OPT-3 Genewiz); SEQ ID NO: 4 (sFLT-OPT-4 DNA2.0); SEQ ID NO: 5 (sFLT-OPT-5 Geneart); SEQ ID NO: 6 (sFLT-OPT-6 IDT); SEQ ID NO: 7 (sFLT-OPT-7 BlueHeron); SEQ ID NO: 8 (sFLT-OPT8 Genescript); SEQ ID NO: 9 (concensus)

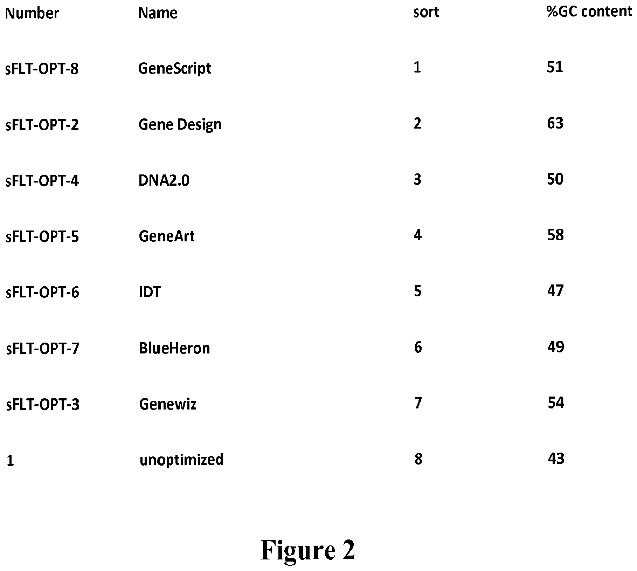

Figure 2 shows the increase of GC of the optimized sFlt1 sequences relative to the unoptimized.

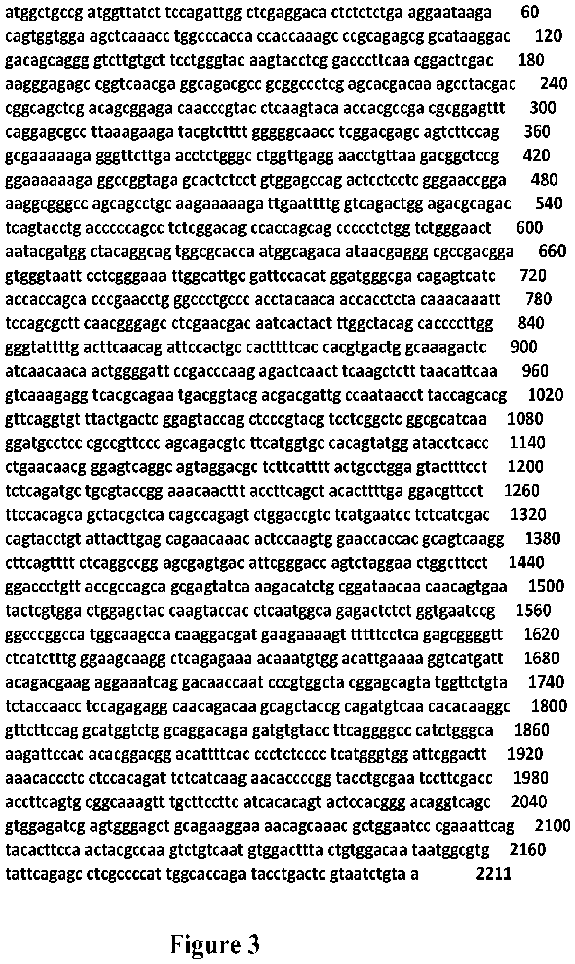

Figure 3 shows the nucleotide sequence of chimeric AAV 2.5 vector (SEQ ID NO: 10).

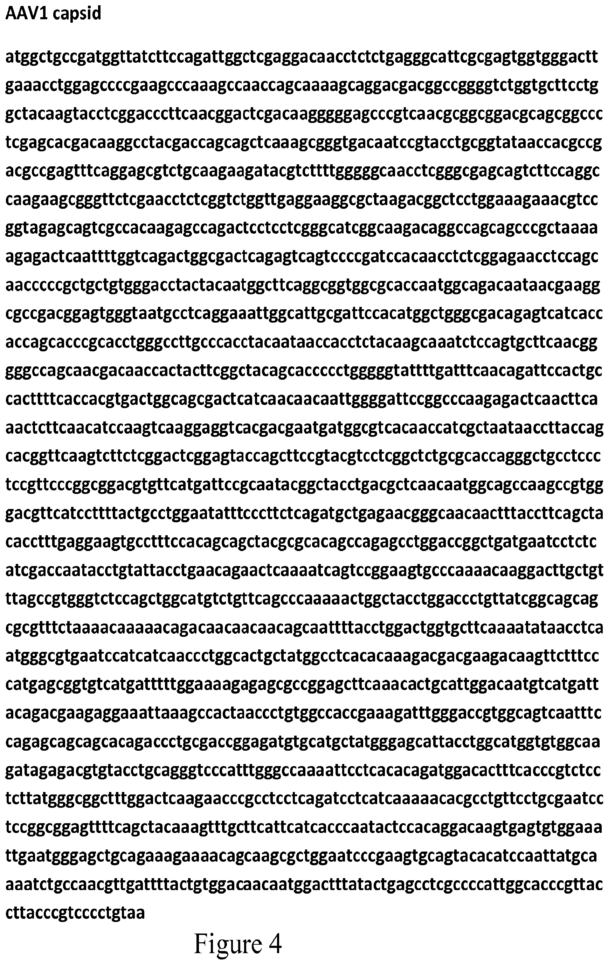

Figure 4 shows the nucleotide sequence of wildtype AAV 1 capsid (SEQ ID NO: 11).

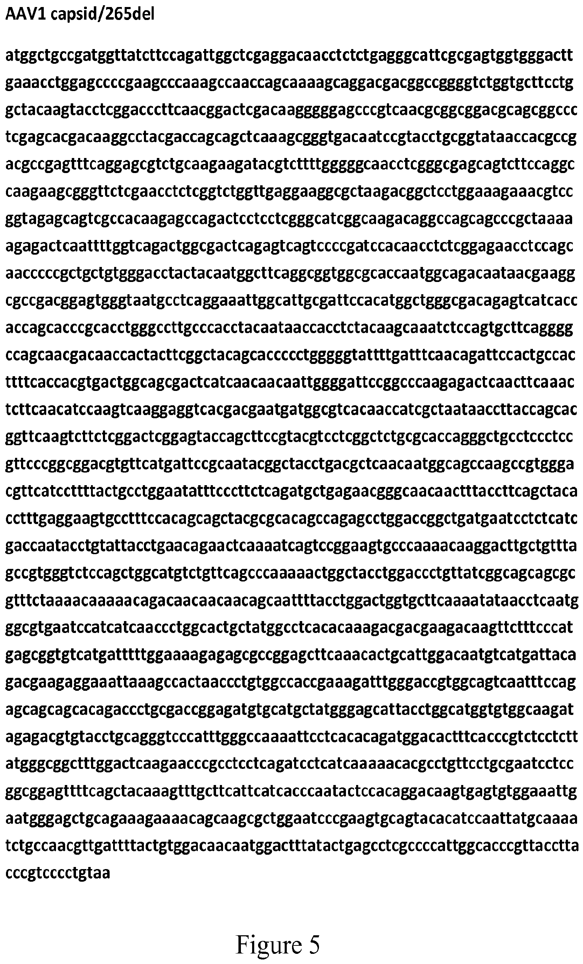

Figure 5 shows the nucleotide sequence of modified AAV 1.1 capsid wherein amino acid residue 265 is deleted (SEQ ID NO: 12).

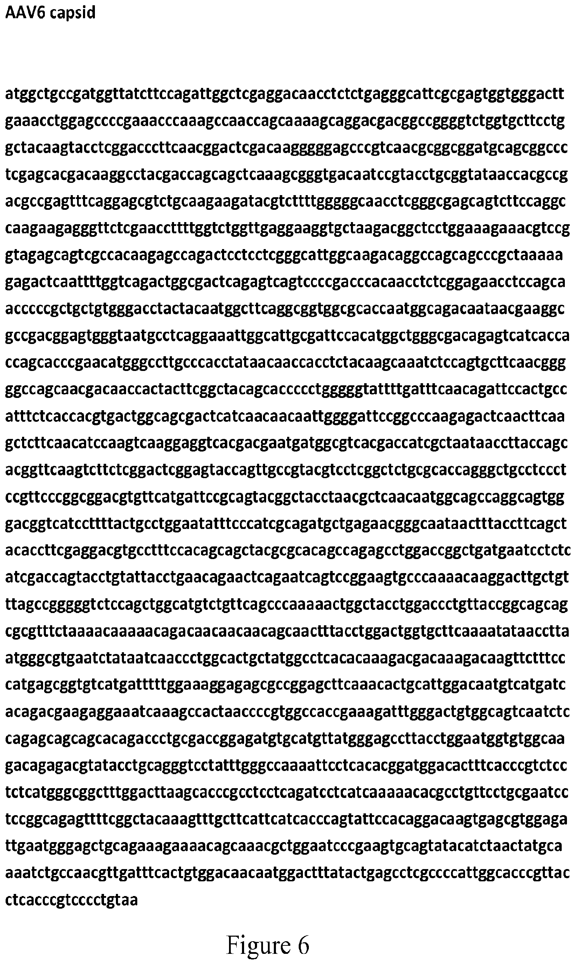

Figure 6 shows the nucleotide sequence of wildtype AAV 6 capsid (SEQ ID NO: 13).

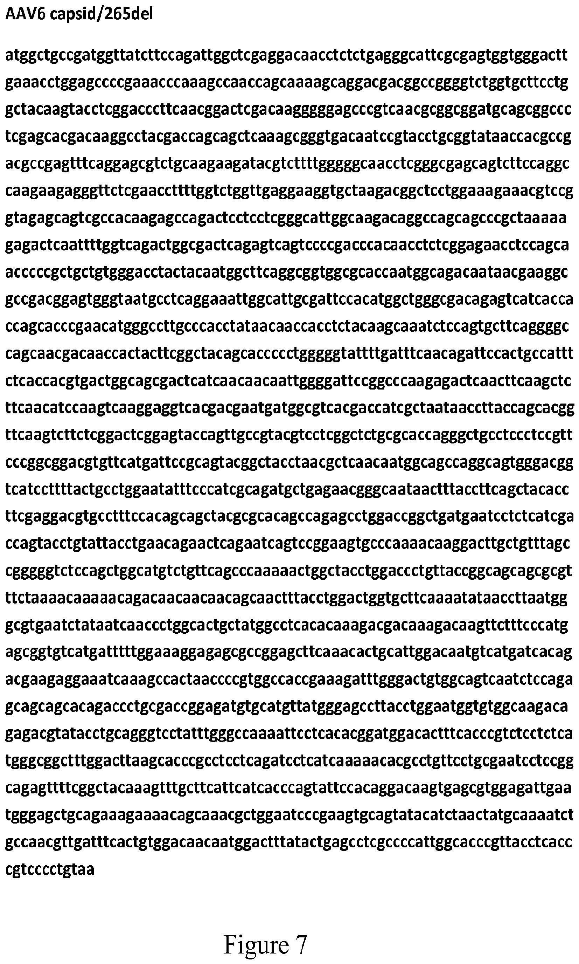

Figure 7 shows the nucleotide sequence of modified AAV 6.1 capsid wherein amino acid residue 265 is deleted (SEQ ID NO: 14).

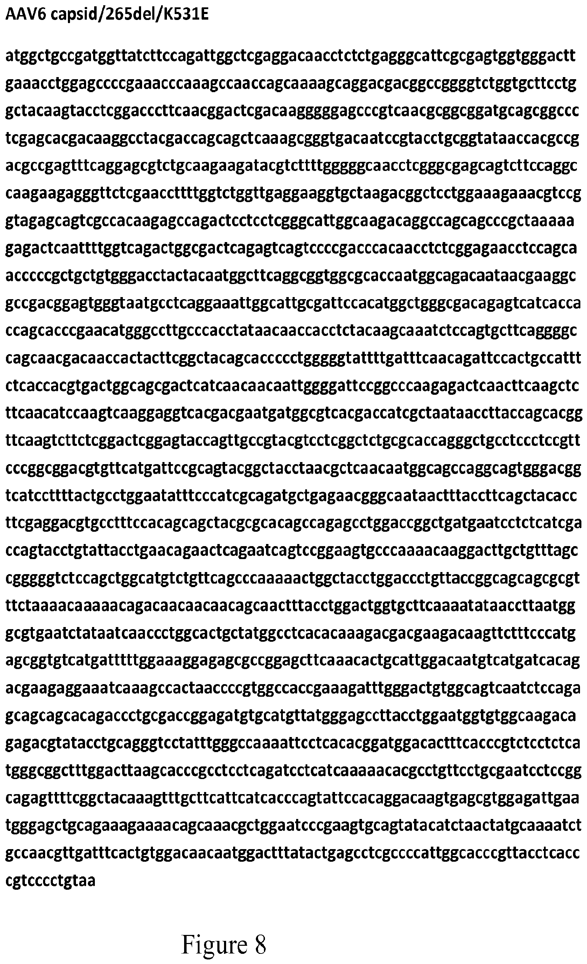

Figure 8 shows the nucleotide sequence of modified AAV 6.3.1 capsid wherein amino acid residue 265 is deleted and amino acid residue 531 is changed from a Lys to a Glu (SEQ ID NO: 15).

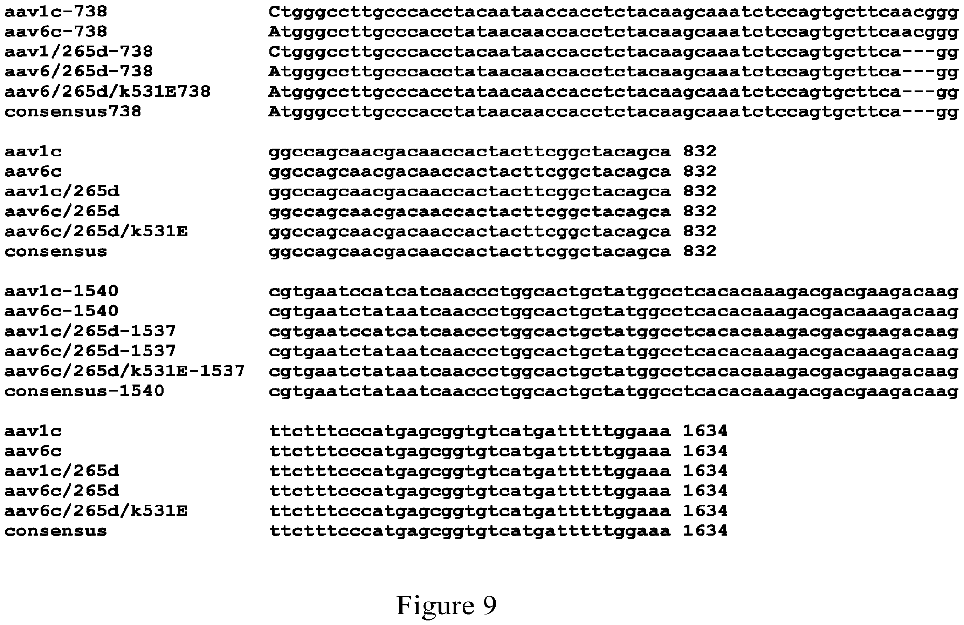

Figure 9 shows the sequences of vectors, that being, nucleotides 738 to 1634 of sequenced defined in Figures 4 to 8, respectively, SEQ ID NO: 16 (AAV1c wildtype), SEQ ID NO: 17 (AAV1.1/265 del); SEQ ID NO: 18 (AAV6c wildtype); SEQ ID NO: 19 (AAV6c.1/265del); and SEQ ID NO: 20 (AAV6c.1/265del/K531E) and consensus sequence (SEQ ID NO: 21).
Figure 10 shows the expression results for sFlt1 for one non-optimized and seven (7) optimized genes by different optimization algorithms and compared to an unoptimized gene sequence (SEQ ID NO: 1 to 8).
Figure 11 shows the expression results for sFlt1 in a serum medium for three of the optimized genes and a graph showing the concentration of the expressed proteins compared to an unoptimized gene sequence.
Figure 12 shows the expression results for sFlt1 for two of the optimized genes and a graph showing the concentration of the expressed proteins compared to an unoptimized gene sequence.
Figure 13 shows photographs of neovascularization of retina tissue when administering the non-optimized gene sequence (SEQ ID NO: 1) and two of the optimized sequences (SEQ ID NO: 2 and 8) included in AAV2 (SEQ ID NO: 22) The control included no sequences for sFlt1(SEQ ID NO: 23). The graph provides values for the mean area of neovascularization taken from the grouping of testing animals.
Figure 14 shows photographs and the leakage caused by laser photocoagulation and the increase in leakage in the control (SEQ ID NO: 23) and reduction when included non-optimized sFlt1 (SEQ ID NO: 1) over the testing period.
Figure 15 shows photographs and the leakage caused by laser photocoagulation and the increase in leakage in the control (SEQ ID NO: 23) and reduction when the vector included optimized sFlt1 (Opt-2) (SEQ ID NO: 24, TR: 20 bp - 164 bp, 3518 bp - 3662 bp; CBh promoter: 385 bp - 1199 bp; sFLT-opt2: 1208 bp - 2864 bp; SV40 PolyA: 3281 bp - 3431 bp)) over the testing period. Notably when the sFlt1 gene was optimized the reduction in damage or leakage is visible when compared to the results of Figure 14 using a non-optimized gene.

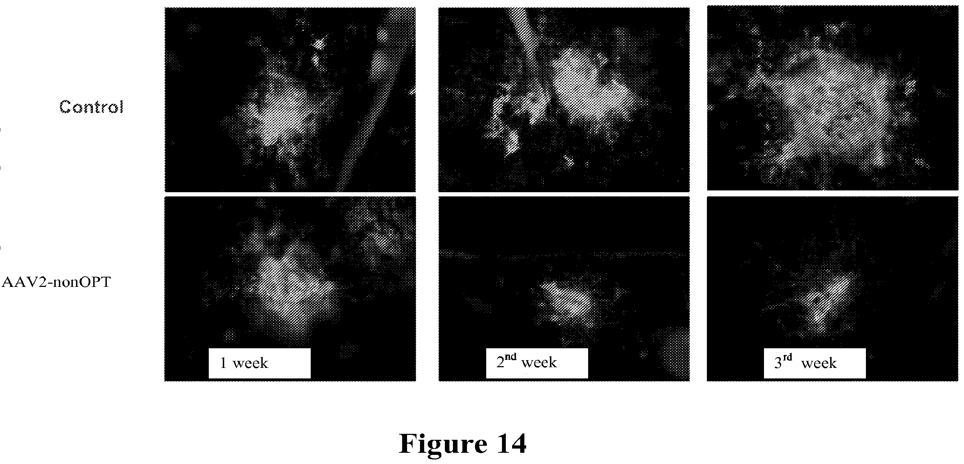

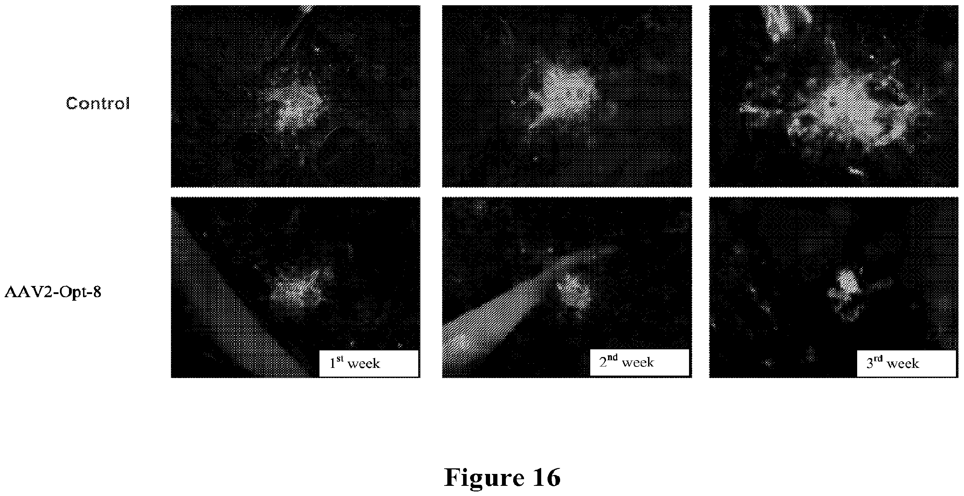

Figure 16 shows photographs and the leakage caused by laser photocoagulation and the increase in leakage in the control (SEQ ID NO: 23, (TR: 20 bp - 164 bp, 3118 bp - 3262 bp; CBh promoter: 385 bp - 1199 bp; Luciferase cDNA:1212 bp - 2864 bp; SV40 PolyA: 2881 bp - 3031 bp)) and reduction when the vector included optimized sFlt1 (Opt-8) (SEQ ID NO: 25, )TR: 20 bp - 164 bp,3518 bp - 3662 bp, CBh promoter: 385 bp - 1199 bp; sFLT-opt 8: 1208 bp - 2864 bp; SV40 PolyA: 3281 bp - 3431 bp )) over the testing period. Notably when the sFlt1 gene was optimized the reduction in damage or leakage is visible when compared to the results of Figure 14 using a non-optimized gene.
Figure 17 shows the results compiled from Figures 14-16 and measured the mean area of CNV when comparing a control (no sFlt1 gene SEQ ID NO: 23) when compared to the non-optimized gene (SEQ ID NO: 1) and Opt-2 (SEQ ID NO: 24) and Opt-8 (SEQ ID NO: 25) optimized genes.
Figure 18 shows the results when the Opt1(nonoptimized SEQ ID NO: 1), Opt-2 (SEQ ID NO: 24) and Opt-8 (SEQ ID NO; 25) were diluted 1:10 and administered to the testing animal. The mean area of CNV shows that Opt-2 maintained its effective in reducing CNV when compared to Opt-8 optimized genes and Opt-1 (nonoptimized).
Figure 19 shows the virus vector construct for the expression of the control Luciferase as shown in SEQ ID NO: 23.
Figure 20 shows the virus vector construct including the optimized sequence for OPT-2, as shown in SEQ ID NO: 24
Figure 21 shows the virus vector construct including the optimized sequence for OPT-8, as shown in SEQ ID NO: 25.

### DETAILED DESCRIPTION OF THE INVENTION

### Definitions

Unless otherwise defined, all technical and scientific terms used herein have the meaning commonly understood by one of ordinary skill in the art to which this invention belongs. The terminology used herein is for the purpose of describing particular embodiments and disclosures only and is not intended to be limiting of the invention. As used in the description of the invention and the appended claims, the singular forms "a", "an" and "the" are intended to include the plural forms as well, unless the context clearly indicates otherwise. The following terms have the meanings given:

"AAV Cap" means AAV Cap proteins, VP1, VP2 and VP3 and analogs thereof.

"AAV Rep" means AAV Rep proteins and analogs thereof.

"AAV TR" means a palindromic sequence, comprising mostly complementary, symmetrically arranged sequences, and includes analogs of native AAV TRs and analogs thereof.

"Biologically-effective" with respect to an amount of a viral vector is an amount that is sufficient to result in infection (or transduction) and expression of the transgene in a target cell.

"Cis-motifs" includes conserved sequences such as found at or close to the termini of the genomic sequence and recognized for initiation of replication; cryptic promoters or sequences at internal positions likely used for transcription initiation or termination.

"Chimeric" means, with respect to a viral capsid or particle, that the capsid or particle includes sequences from different parvoviruses, preferably different AAV serotypes, as described in Rabinowitz et al., U.S. Patent 6,491,907, entitled "Recombinant parvovirus vectors and method of making," granted on December 10, 2002. A particularly preferred chimeric viral capsid is the AAV2.5 capsid, which has the sequence of the AAV2 capsid with the following mutations: 263 Q→A; 265 insertion T; 705 N→A; 708 V→A; and 716 T→N. wherein the nucleotide sequence expressing such capsid is defined as SEQ ID NO: 8.

"Flanked," with respect to a sequence that is flanked by other elements, indicates the presence of one or more the flanking elements upstream and/or downstream, i.e., 5' and/or 3', relative to the sequence. The term "flanked" is not intended to indicate that the sequences are necessarily contiguous. For example, there may be intervening sequences between the nucleic acid encoding the transgene and a flanking element. A sequence (e.g., a transgene) that is "flanked" by two other elements (e.g., TRs), indicates that one element is located 5' to the sequence and the other is located 3' to the sequence; however, there may be intervening sequences therebetween.

Polynucleotide" means a sequence of nucleotides connected by phosphodiester linkages. Polynucleotides are presented herein in the direction from the 5' to the 3' direction. A polynucleotide of the present invention can be a deoxyribonucleic acid (DNA) molecule or ribonucleic acid (RNA) molecule. Where a polynucleotide is a DNA molecule, that molecule can be a gene or a cDNA molecule. Nucleotide bases are indicated herein by a single letter code: adenine (A), guanine (G), thymine (T), cytosine (C), inosine (I) and uracil (U). A polynucleotide of the present invention can be prepared using standard techniques well known to one of skill in the art.

"Transduction" of a cell by a virus means that there is transfer of DNA or RNA from the virus particle to the cell.

"Transfection" of a cell means that genetic material is introduced into a cell for the purpose of genetically modifying the cell. Transfection can be accomplished by a variety of means known in the art, such as transduction or electroporation.

"Polypeptide" encompasses both peptides and proteins, unless indicated otherwise.

"Transgene" is used in a broad sense to mean any heterologous nucleotide sequence incorporated in a viral vector for expression in a target cell and associated expression control sequences, such as promoters. It is appreciated by those of skill in the art that expression control sequences will be selected based on ability to promote expression of the transgene in the target cell. An example of a transgene is a nucleic acid encoding a therapeutic polypeptide.

"Vector," means a recombinant plasmid or virus that comprises a polynucleotide to be delivered into a host cell, either in vitro or in vivo.

"Recombinant" means a genetic entity distinct from that generally found in nature. As applied to a polynucleotide or gene, this means that the polynucleotide is the product of various combinations of cloning, restriction and/or ligation steps, and other procedures that result in the production of a construct that is distinct from a polynucleotide found in nature.

"Substantial homology" or "substantial similarity," means, when referring to a nucleic acid or fragment thereof, indicates that, when optimally aligned with appropriate nucleotide insertions or deletions with another nucleic acid (or its complementary strand), there is nucleotide sequence identity in at least about 95 to 99% of the sequence.

"Recombinant viral vector" means a recombinant polynucleotide vector comprising one or more heterologous sequences (i.e., polynucleotide sequence not of viral origin). In the case of recombinant parvovirus vectors, the recombinant polynucleotide is flanked by at least one, preferably two, inverted terminal repeat sequences (ITRs) that have been known to provide high-level persistent nucleic acid expression.

"Serotype" with respect to vector or virus capsid is defined by a distinct immunological profile based on the capsid protein sequences and capsid structure.

"Peptide", "polypeptide" and "protein" are used interchangeably to denote a sequence polymer of at least two amino acids covalently linked by an amide bond.

"Homologous" used in reference to peptides, refers to amino acid sequence similarity between two peptides. When an amino acid position in both of the peptides is occupied by identical amino acids, they are homologous at that position. Thus by "substantially homologous" means an amino acid sequence that is largely, but not entirely, homologous, and which retains most or all of the activity as the sequence to which it is homologous. As used herein, "substantially homologous" as used herein means that a sequence is at least 50% identical, and preferably at least 75% and more preferably 95% homology to the reference peptide. Additional peptide sequence modification are included, such as minor variations, deletions, substitutions or derivitizations of the amino acid sequence of the sequences disclosed herein, so long as the peptide has substantially the same activity or function as the unmodified peptides. Derivatives of an amino acid may include but not limited to trifluoroleucine, hexafluoroleucine, 5,5,5-trifluoroisoleucine, 4,4,4-trifluorovaline, p-fluorophenylaline, o-fluorotyrosine, m-fluorotyrosine, 2,3-difluorotyrosine, 4-fluorohistidine, 2-fluorohistidine, 2,4-difluorohistidine, fluoroproline, difluoroproline, 4-hydroxyproline, selenomethionine, telluromethionine, selenocysteine, selenatryptophans, 4-aminotryptophan, 5-aminotryptophan, 5-hydroxytryptophan, 7-azatryptophan, 4-fluorotryptophan, 5-fluorotryptophan, 6-fluorotryptophan, homoallylglycine, homopropargylglycine, 2-butynylglycine, cis-crotylglycine, allylglycine, dehydroleucine, dehydroproline, 2-amino-3-methyl-4-pentenoic acid, azidohomoalanine, asidoalanine, azidonorleucine, p-ethynylphenylalanine, p-azidophenylalanine, p-bromophenylalanine, p-acetylphenylalanine and benzofuranylalanine. Notably, a modified peptide will retain activity or function associated with the unmodified peptide, the modified peptide will generally have an amino acid sequence "substantially homologous" with the amino acid sequence of the unmodified sequence.

The invention provides modified nucleic acids encoding sFlt1 for use in treating ocular disorder causing neovascularization in a human subject. The invention also provides nucleic acid constructs for said use which include as part of their sequence the modified nucleic acid encoding sFlt1. For example, the invention includes plasmids and/or other vectors for the use indicated above that include the modified sFlt1 sequence along with other elements, such as regulatory elements. Further, the invention provides packaged gene delivery vehicle, such as a viral capsid, including the modified sFlt1 sequence for the use indicated above. The invention also includes methods of expressing sFlt1 by delivering the modified sequence into a cell along with elements required to promote expression in the cell. Also disclosed is gene therapy methods in which the modified sFlt1 sequence is administered to a subject, e.g., as a component of a vector and/or packaged as a component of a viral gene delivery vehicle. Treatment may, for example, be effected to reduce angiogenesis in retina tissue. Each of these aspects of the invention is discussed further in the ensuing sections.

### Modified Nucleic Acid for Expression of sFlt1

The invention provides a modified sequence encoding sFlt1 for use in treating ocular disorder causing neovascularization in a human subject. The modified sequence includes the native sFlt1 sequence including one or more optimizing modifications. Examples of optimizing modifications include elimination of one or more cis-acting motifs and introduction of one or more Kozak sequences. It is also disclosed that one or more cis-acting motifs are eliminated and one or more Kozak sequences are introduced.

Examples of cis acting motifs that may be eliminated include internal TATA-boxes; chi-sites; ribosomal entry sites; AT-rich and/or GC-rich sequence stretches; ARE, INS, and/or CRS sequence elements; repeat sequences and/or RNA secondary structures; (cryptic) splice donor and/or acceptor sites, branch points; and Sa1I. Preferably, GC content is enhanced relative to wild-type sFlt1 and one or more cis-acting motifs are removed. The GC content is preferably at least 20 to 90% greater than the wild type gene. Additionally, the codon adaptation index is preferably >75, >80, >85, >90, or >95.

The modified sFlt1 sequence may also include flanking restriction sites to facilitate subcloning into expression vector.

The invention includes a nucleic acid vector including the modified sFlt1 sequence and various regulatory elements. The precise nature of regulatory elements useful for gene expression will vary from organism to organism. In general, they include a promoter which directs the initiation of RNA transcription in the cell of interest. The promoter may be constitutive or regulated. Constitutive promoters are those which cause an operably linked gene to be expressed essentially at all times. Regulated promoters are those which can be activated or deactivated. Regulated promoters include inducible promoters, which are usually "off" but which may be induced to turn "on," and "repressible" promoters, which are usually "on" but may be turned "off." Many different regulators are known, including temperature, hormones, cytokines, heavy metals and regulatory proteins. The distinctions are not absolute; a constitutive promoter may often be regulated to some degree. In some cases an endogenous pathway may be utilized to provide regulation of the transgene expression, e.g., using a promoter that is naturally downregulated when the pathological condition improves.

Examples of suitable promoters include adenoviral promoters, such as the adenoviral major late promoter; heterologous promoters, such as the cytomegalovirus (CMV) promoter; the respiratory syncytial virus promoter; the Rous Sarcoma Virus (RSV) promoter; the albumin promoter; inducible promoters, such as the Mouse Mammary Tumor Virus (MMTV) promoter; the metallothionein promoter; heat shock promoters; the α-1-antitrypsin promoter; the hepatitis B surface antigen promoter; the transferrin promoter; the apolipoprotein A-1 promoter; and human sFlt1 promoters. The promoter may be a tissue-specific promoter, such as the mouse albumin promoter, which is active in liver cells as well as the transthyretin promoter (TTR).

### Packaged Modified sFlt1 Sequence

The modified sFlt1 sequence may also be provided as a component of a packaged viral vector. In general, packaged viral vectors include a viral vector packaged in a capsid. Viral vectors and viral capsids are discussed in the ensuing sections.

### Viral Vector

The viral vector component of the packaged viral vectors includes at least one modified sFlt1 sequence and associated expression control sequences for controlling expression of the modified sFlt1 sequence. The viral vector may include cis-acting functions sufficient to enable persistence as episomal forms or by mechanisms including integration of the modified sFlt1 sequence into the genome of a target cell.

In a preferred embodiment, the viral vector includes a portion of a parvovirus genome, such as an AAV genome with rep and cap deleted and replaced by the modified sFlt1 sequence and its associated expression control sequences. The modified sFlt1 sequence is typically inserted adjacent to one or two (i.e., flanked by) AAV TRs or TR elements adequate for viral replication; Xiao et al., J Virol 71; 2: 941-948 (1997), in place of the viral rep and cap ORFs. Other regulatory sequences suitable for use in facilitating tissue-specific expression of the modified sFlt1 sequence in the target cell may also be included.

The viral vector may be any suitable nucleic acid construct, such as a DNA or RNA construct and may be single stranded, double stranded, or duplexed.

### Viral Capsid

The viral capsid component of the packaged viral vectors may be a parvovirus capsid. AAV Cap and chimeric capsids are preferred. Examples of suitable parvovirus viral capsid components are capsid components from the family Parvoviridae, such as an autonomous parvovirus or a Dependovirus. For example, the viral capsid may be an AAV capsid (e.g., AAV1, AAV2, AAV3, AAV4, AAV5, AAV6, AAV7, AAV8 AAV 9, AAV10, AAV11 or AAV 12 capsid; one skilled in the art would know there are likely other variants not yet identified that perform the same or similar function), or may include components from two or more AAV capsids. A full complement of AAV Cap proteins includes VP1, VP2, and VP3. The ORF comprising nucleotide sequences encoding AAV VP capsid proteins may comprise less than a full complement AAV Cap proteins or the full complement of AAV Cap proteins may be provided.

One or more of the AAV Cap proteins may be a chimeric protein, including amino acid sequences AAV Caps from two or more viruses, preferably two or more AAVs, as described in Rabinowitz et al., U.S. Patent 6,491,907, entitled "Recombinant parvovirus vectors and method of making," granted on December 10, 2002. For example, the chimeric virus capsid can include an AAV1 Cap protein or subunit and at least one AAV2 Cap or subunit. The chimeric capsid can, for example, include an AAV capsid with one or more B19 Cap subunits of human parvovirus, e.g., an AAV Cap protein or subunit can be replaced by a B19 Cap protein or subunit. For example, in a preferred embodiment, the Vp3 subunit of the AAV capsid can be replaced by the Vp2 subunit of B 19.

### Production of Packaged Viral Vector

Also disclosed are packaging cells which may be cultured to produce packaged viral vectors of the invention. The packaging cells generally include cells with heterologous (1) viral vector function(s), (2) packaging function(s), and (3) helper function(s). Each of these component functions is discussed in the ensuing sections.

### Viral Vector Functions

The packaging cells include viral vector functions, along with packaging and vector functions. The viral vector functions typically include a portion of a parvovirus genome, such as an AAV genome, with rep and cap deleted and replaced by the modified sFlt1 sequence and its associated expression control sequences. The viral vector functions include sufficient expression control sequences to result in replication of the viral vector for packaging. Typically, the viral vector includes a portion of a parvovirus genome, such as an AAV genome with rep and cap deleted and replaced by the transgene and its associated expression control sequences. The transgene is typically flanked by two AAV TRs, in place of the deleted viral rep and cap ORFs. Appropriate expression control sequences are included, such as a tissue-specific promoter and other regulatory sequences suitable for use in facilitating tissue-specific expression of the transgene in the target cell. The transgene is typically a nucleic acid sequence that can be expressed to produce a therapeutic polypeptide or a marker polypeptide. The viral vector may be any suitable nucleic acid construct, such as a DNA or RNA construct and may be single stranded, double stranded, or duplexed.

The viral vector functions may suitably be provided as duplexed vector templates, as described in U.S. Patent Publication No. 2004/0029106 to Samulski et al.. Duplexed vectors are dimeric self-complementary (sc) polynucleotides (typically, DNA). For example, the DNA of the duplexed vectors can be selected so as to form a double-stranded hairpin structure due to intrastrand base pairing. Both strands of the duplexed DNA vectors may be packaged within a viral capsid. The duplexed vector provides a function comparable to double-stranded DNA virus vectors and can alleviate the need of the target cell to synthesize complementary DNA to the single-stranded genome normally encapsidated by the virus.

The TR(s) (resolvable and non-resolvable) selected for use in the viral vectors are preferably AAV sequences, with serotypes 1, 2, 3, 4, 5 and 6 being preferred. Resolvable AAV TRs need not have a wild-type TR sequence (e.g., a wild-type sequence may be altered by insertion, deletion, truncation or missense mutations), as long as the TR mediates the desired functions, e.g., virus packaging, integration, and/or provirus rescue, and the like. The TRs may be synthetic sequences that function as AAV inverted terminal repeats, such as the "double-D sequence" as described in U.S. Pat. No. 5,478,745 to Samulski et al.. Typically, but not necessarily, the TRs are from the same parvovirus, e.g., both TR sequences are from AAV2

The packaging functions include capsid components. The capsid components are preferably from a parvoviral capsid, such as an AAV capsid or a chimeric AAV capsid function. Examples of suitable parvovirus viral capsid components are capsid components from the family Parvoviridae, such as an autonomous parvovirus or a Dependovirus. For example, the capsid components may be selected from AAV capsids, e.g., AAV1-AAV12 and other novel capsids as yet unidentified or from non human primate sources. Capsid components may include components from two or more AAV capsids, providing a chimeric AAV.

It is also disclosed that one or more of the VP capsid proteins is a chimeric protein, comprising amino acid sequences from two or more viruses, preferably two or more AAVs, as described in Rabinowitz et al., U.S. Patent 6,491,907, entitled "Recombinant parvovirus vectors and method of making," granted on December 10, 2002.

For example, the chimeric virus capsid can include a capsid region from an adeno-associated virus (AAV) and at least one capsid region from a B19 virus. The chimeric capsid can, for example, include an AAV capsid with one or more B19 capsid subunits, e.g., an AAV capsid subunit can be replaced by a B19 capsid subunit. For example, it is disclosed that the VP1, VP2 or VP3 subunit of the AAV capsid can be replaced by the VP1, VP2 or VP3 subunit of B19. As another example, the chimeric capsid may include an AAV type 2 capsid in which the type 2 VP1 subunit has been replaced by the VP1 subunit from an AAV type 1, 3, 4, 5, or 6 capsid, preferably a type 3, 4, or 5 capsid. Alternatively, the chimeric parvovirus has an AAV type 2 capsid in which the type 2 VP2 subunit has been replaced by the VP2 subunit from an AAV type 1, 3, 4, 5, or 6 capsid, preferably a type 3, 4, or 5 capsid. Likewise, chimeric parvoviruses in which the VP3 subunit from an AAV type 1, 3, 4, 5 or 6 (more preferably, type 3, 4 or 5) is substituted for the VP3 subunit of an AAV type 2 capsid are preferred. As a further alternative, chimeric parvoviruses in which two of the AAV type 2 subunits are replaced by the subunits from an AAV of a different serotype (e.g., AAV type 1, 3, 4, 5 or 6) are preferred. In exemplary chimeric parvoviruses according to this disclosure, the VP1 and VP2, or VP1 and VP3, or VP2 and VP3 subunits of an AAV type 2 capsid are replaced by the corresponding subunits of an AAV of a different serotype (e.g., AAV type 1, 3, 4, 5 or 6). Likewise, it is also disclosed that the chimeric parvovirus has an AAV type 1, 3, 4, 5 or 6 capsid (preferably the type 2, 3 or 5 capsid) in which one or two subunits have been replaced with those from an AAV of a different serotype, as described above for AAV type 2.

The packaged viral vector generally includes the modified sFlt1 sequence and expression control sequences flanked by TR elements sufficient to result in packaging of the vector DNA and subsequent expression of the modified sFlt1 sequence in the transduced cell. The viral vector functions may, for example, be supplied to the cell as a component of a plasmid or an amplicon. The viral vector functions may exist extrachromosomally within the cell line and/or may be integrated into the cells' chromosomal DNA.

Any method of introducing the nucleotide sequence carrying the viral vector functions into a cellular host for replication and packaging may be employed, including but not limited to, electroporation, calcium phosphate precipitation, microinjection, cationic or anionic liposomes, and liposomes in combination with a nuclear localization signal. In embodiments wherein the viral vector functions are provided by transfection using a virus vector; standard methods for producing viral infection may be used.

### Packaging Functions

The packaging functions include genes for viral vector replication and packaging. Thus, for example, the packaging functions may include, as needed, functions necessary for viral gene expression, viral vector replication, rescue of the viral vector from the integrated state, viral gene expression, and packaging of the viral vector into a viral particle. The packaging functions may be supplied together or separately to the packaging cell using a genetic construct such as a plasmid or an amplicon. The packaging functions may exist extrachromosomally within the packaging cell, but are preferably integrated into the cell's chromosomal DNA. Examples include genes encoding AAV Rep and Cap proteins.

### Helper Functions

The helper functions include helper virus elements needed for establishing active infection of the packaging cell, which is required to initiate packaging of the viral vector. Examples include functions derived from adenovirus, baculovirus and/or herpes virus sufficient to result in packaging of the viral vector. For example, adenovirus helper functions will typically include adenovirus components E1a, E1b, E2a, E4, and VA RNA. The packaging functions may be supplied by infection of the packaging cell with the required virus. The packaging functions may be supplied together or separately to the packaging cell using a genetic construct such as a plasmid or an amplicon. The packaging functions may exist extrachromosomally within the packaging cell, but are preferably integrated into the cell's chromosomal DNA.

Any suitable helper virus functions may be employed. For example, where the packaging cells are insect cells, baculovirus may serve as a helper virus. Herpes virus may also be used as a helper virus in AAV packaging methods. Hybrid herpes viruses encoding the AAV Rep protein(s) may advantageously facilitate for more scalable AAV vector production schemes.

Any method of introducing the nucleotide sequence carrying the helper functions into a cellular host for replication and packaging may be employed, including but not limited to, electroporation, calcium phosphate precipitation, microinjection, cationic or anionic liposomes, and liposomes in combination with a nuclear localization signal. In embodiments wherein the helper functions are provided by transfection using a virus vector or infection using a helper virus; standard methods for producing viral infection may be used.

### Packaging Cell

Any suitable permissive or packaging cell known in the art may be employed in the production of the packaged viral vector. Mammalian cells or insect cells are preferred. Examples of cells useful for the production of packaging cells in the practice of the invention include, for example, human cell lines, such as VERO, WI38, MRC5, A549, 293 cells, B-50 or any other HeLa cells, HepG2, Saos-2, HuH7, and HT1080 cell lines.

Preferred cell lines for use as packaging cells are insect cell lines. Any insect cell which allows for replication of AAV and which can be maintained in culture can be used in accordance with the present invention. Examples include Spodoptera frugiperda, such as the Sf9 or Sf21 cell lines, Drosophila spp. cell lines, or mosquito cell lines, e.g., Aedes albopictus derived cell lines. A preferred cell line is the Spodoptera frugiperda Sf9 cell line. The following references concern the use of insect cells for expression of heterologous polypeptides, methods of introducing nucleic acids into such cells, and methods of maintaining such cells in culture: Methods in Molecular Biology, ed. Richard, Humana Press, NJ (1995); O'Reilly et al., Baculovirus Expression Vectors: A Laboratory Manual, Oxford Univ. Press (1994); Samulski et al., J. Vir. 63:3822-8 (1989); Kajigaya et al., Proc. Nat'l. Acad. Sci. USA 88: 4646-50 (1991); Ruffing et al., J. Vir. 66:6922-30 (1992); Kimbauer et al., Vir. 219:37-44 (1996); Zhao et al., Vir. 272:382-93 (2000); and Samulski et al., U.S. Pat. No. 6,204,059.

During production, the packaging cells generally include one or more viral vector functions along with helper functions and packaging functions sufficient to result in replication and packaging of the viral vector. These various functions may be supplied together or separately to the packaging cell using a genetic construct such as a plasmid or an amplicon, and they may exist extrachromosomally within the cell line or integrated into the cell's chromosomes.

The cells may be supplied with any one or more of the stated functions already incorporated, e.g., a cell line with one or more vector functions incorporated extrachromosomally or integrated into the cell's chromosomal DNA, a cell line with one or more packaging functions incorporated extrachromosomally or integrated into the cell's chromosomal DNA, or a cell line with helper functions incorporated extrachromosomally or integrated into the cell's chromosomal DNA.

### sFlt1 of the invention for use in treatment methods

The modified sFlt1 gene may be for use in gene therapy of ocular neovascularization diseases, such as macular degeneration. An individual may be in need of gene therapy for clinical management of angiogenesis in the retina. Further the present invention may include treatment for visual loss caused by macular edema and evident in subjects with diabetes, uveitis and after cataract surgery.

In particular embodiments, the present invention provides a pharmaceutical composition comprising a vector of the present invention including a modified gene of sFlt1 in a pharmaceutically-acceptable carrier and/or other medicinal agents, pharmaceutical agents, carriers, adjuvants, diluents, etc. For injection, the carrier will typically be a liquid. For other methods of administration, the carrier may be either solid or liquid. For inhalation administration, the carrier will be respirable, and will preferably be in solid or liquid particulate form. As an injection medium, it is preferred to use water that contains the additives usual for injection solutions, such as stabilizing agents, salts or saline, and/or buffers.

Exemplary pharmaceutically acceptable carriers include sterile, pyrogen-free water and sterile, pyrogen-free, phosphate buffered saline. Physiologically-acceptable carriers include pharmaceutically-acceptable carriers. Pharmaceutically acceptable carriers are those which are that is not biologically or otherwise undesirable, i.e., the material may be administered to a subject without causing undesirable biological effects which outweigh the advantageous biological effects of the material.

A pharmaceutical composition may be used, for example, in transfection of a cell ex vivo or in administering a viral vector or cell directly to a subject.

Recombinant virus vectors comprising the modified gene of sFlt1 are preferably administered to the cell in a biologically-effective amount. If the virus vector is administered to a cell *in vivo* (e.g., the virus is administered to a subject as described below), a biologically-effective amount of the virus vector is an amount that is sufficient to result in transduction and expression of the transgene in a target cell and in an amount to reduce the activity of VEGF.

Also disclosed is a method of treating subjects *in vivo* with the vector containing modified genes. Administration of the vector to a human subject or an animal in need thereof can be by any means known in the art for administering virus vectors.

Exemplary modes of administration include intravenous, subcutaneous, intradermal, intramuscular, and intraarticular administration, and the like, as well as direct tissue or organ injection, alternatively, intrathecal, direct intramuscular, intraventricular, intravenous, intraperitoneal, intranasal, or intraocular injections. Injectables can be prepared in conventional forms, either as liquid solutions or suspensions, solid forms suitable for solution or suspension in liquid prior to injection, or as emulsions. Alternatively, one may administer the virus in a local rather than systemic manner, for example, in a depot or sustained-release formulation.

Dosages of the inventive virus vector with the modified sFlt1 gene will depend upon the mode of administration, the disease or condition to be treated, the individual subject's condition, the particular viral vector, and the gene to be delivered, and can be determined in a routine manner. Exemplary doses for achieving therapeutic effects are virus titers of at least about 10⁵, 10⁶, 10⁷, 10⁸, 10⁹, 10¹⁰, 10¹¹, 10¹², 10¹³, 10¹⁴, 10¹⁵ transducing units or more, preferably about 10⁸-10¹³ transducing units, yet more preferably 10¹² transducing units.

The modified sFlt1 gene may be administered as components of a DNA molecule having regulatory elements appropriate for expression in the target cells. The modified sFlt1 gene may be administered as components of viral plasmids, such as rAAV vectors. Viral particles may be for use for administration as viral particles alone, whether as an *in vivo* direct delivery to the portal vasculature or for use as an *ex vivo* treatment comprising administering the vector viral particles *in vitro* to cells from the animal receiving treatment followed by introduction of the transduced cells back into the donor.

### Examples

### Expression of the Proteins

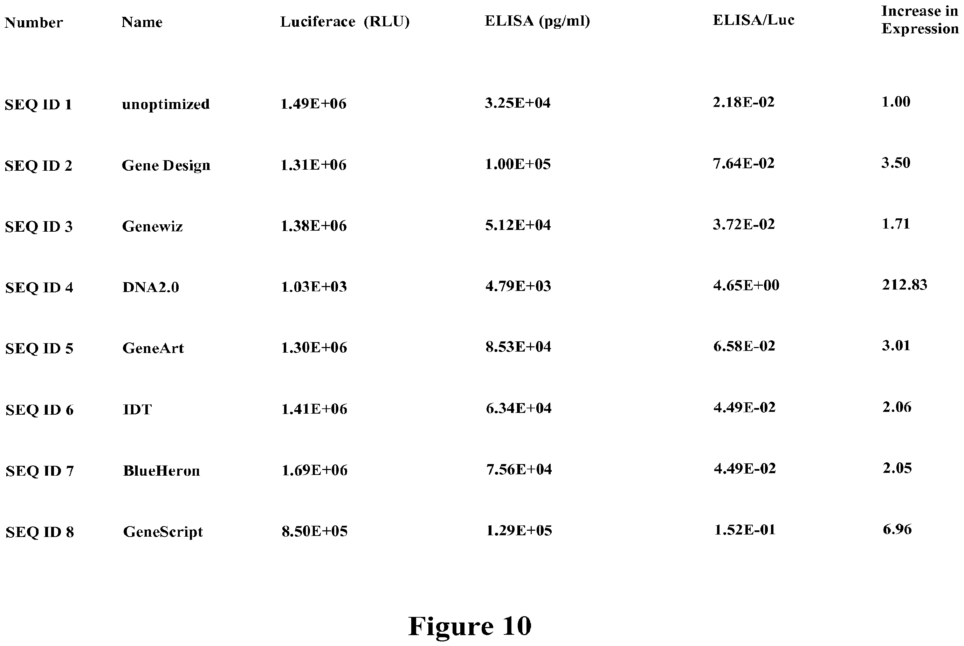

The novel and unique optimized gene sequences of SEQ ID NOs: 2 to 8 were created by seven different and distinct methods of optimization and were then subsequently expressed. As shown in Figure 10, the different sequences provided for very different results in three separate assays used to determine the level of protein expression. Using the unoptimized gene sequence as the control for the ELISA/Luc assay, then each optimized gene sequence expressed different amounts of the specific sFlt1 protein. Thus, the optimized sequences are each novel and the clearly it is not known as to which will provide for the optimal amount of the expressed sFlt1 protein.

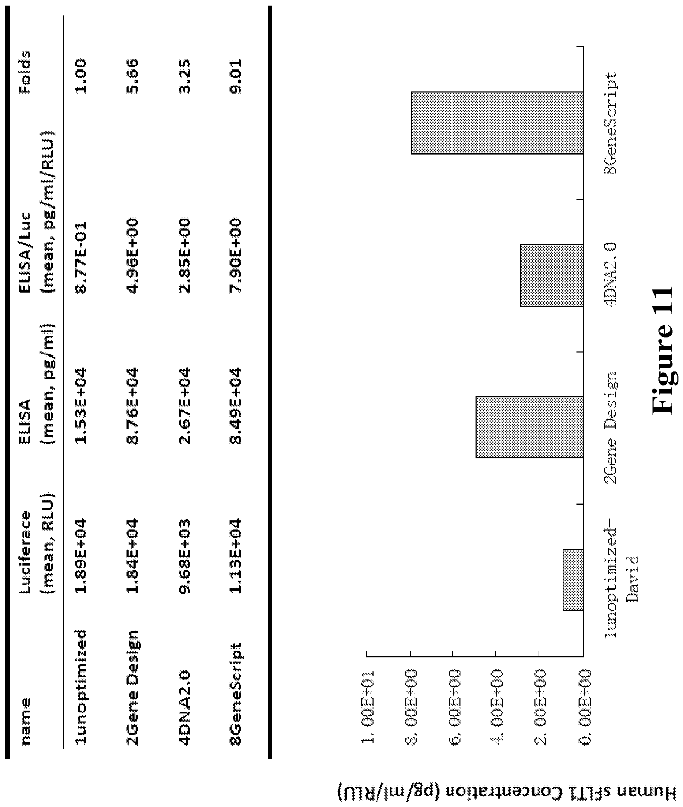

Figure 11 provides further indication of the diversity of different optimized gene sequences and the expression thereof wherein SEQ ID NO: 8 (GeneScript) provided the highest concentration of expression. Further, it should be noted that the medium used for expression of the proteins is different from that used in Figure 10.

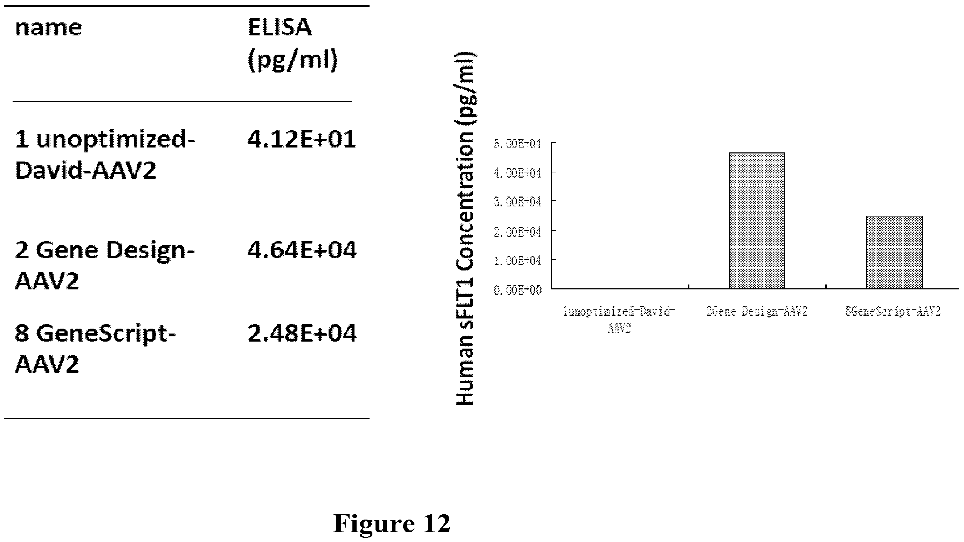

Using AAV2 carrying optimized genes vs. wild type sFlt1 coding sequence, side-by-side expression analysis was carried out *in vitro* (Figure 12) and ELISA was used to determine the total amount of sFlt1 protein. Based on these studies it is clear that the optimized sFlt1 genes, that being SEQ ID NO: 2 and SEQ ID NO: 8, expressed significantly more sFlt1 protein product when compared to the unoptimized coding sequence when delivered at the same dose.

### In Vivo Testing

Notably the mouse retina can be used as a tool to investigate conditions in which normal vascular development becomes unbalanced, leading to sight-threatening diseases, as occurs in human retinopathy of prematurity (ROP). Thus, successful modeling of human ROP in this animal model can replicate the phases of ROP including neovascularization (NV)-along with some of the more frequent complications like vascular leakage. Oxygen-induced retinopathy (OIR) was induced in the testing mice by exposure to increased levels of oxygen for about four days postnatal and then returned to normal oxygen content of about 20%. On P4, three specific vectors were injected into the eyes of the testing mice in an amount of about 0.5 microliters using a sharp needle. Also a control group was included wherein no injection was administered. The vectors included AAV2 - sFlt1 unoptimized (SEQ ID NO: 1), AAV2-OPT-2 (SEQ ID NO: 2, GeneDesign) and AAV2-OPT-8 (SEQ ID NO: 8, GeneScript) and a control wherein no sFlt1 was introduced. On P17 the different levels of concentration of expressed sFlt1 were measured by removing the eyes wherein the retina was dissected and cut radially into four quadrants and mounted for examination by fluorescence. The total neovascular area was determined relative to the total retina and then compared to the control.

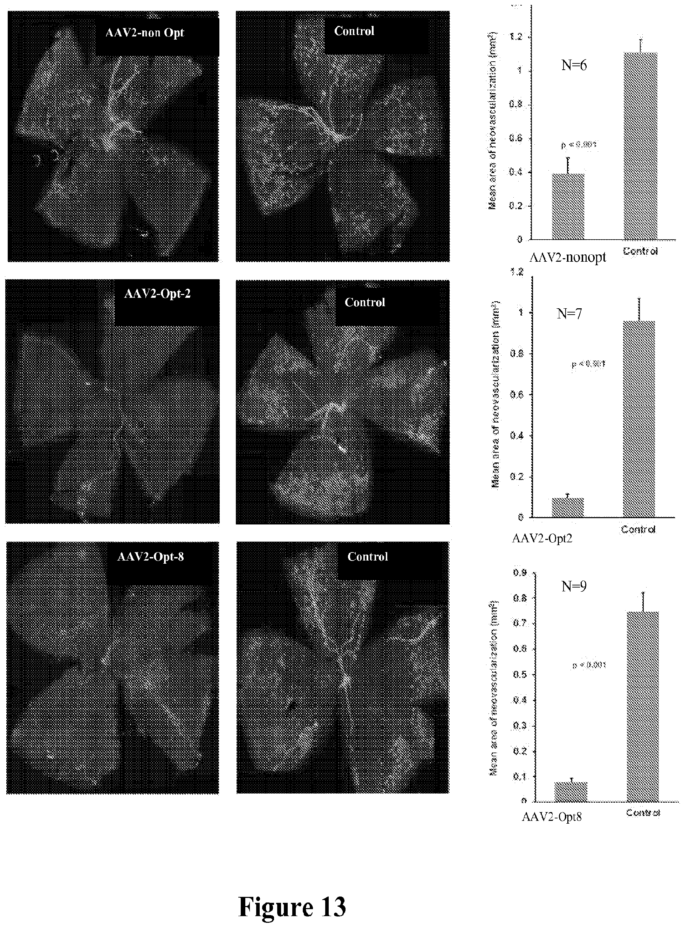

Figure 13 shows that using the AAV2-nonoptimized sFlt1 gene (Seq ID NO: 1) showed a mean area of neovascularization of about 0.4 mm² while the control (no sFLT1) was about 1.1 mm². Using the AAV2-Opt-2 vector (SEQ ID NO: 2) it was found that the optimized gene expressed more sFlt1 and this is evident because the mean area of neovascularization was reduced to out 0.1 mm² while the control showed a mean area of neovascularization of about 1.00 mm². Using the vector AAV2-Opt-8 (SEQ ID NO: 8) provided for increased expression of sFlt1 and a mean area of neovascularization of approximately 0.09mm² with a control of about 0.85 mm². Clearly the optimized genes expressing sFlt1 provided for increased levels of expression causing a surprising and unexpected reduction in the area of neovascularization of retina tissue and intensity of fluorescence.

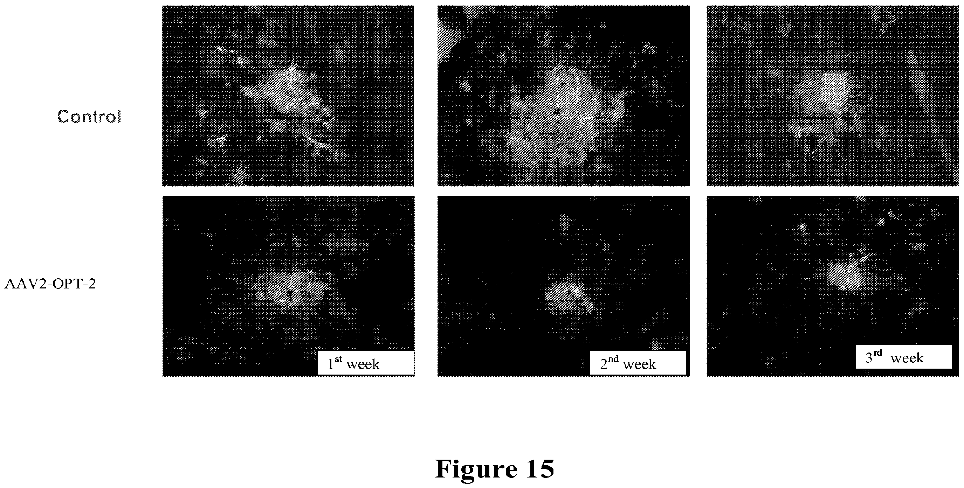

It was also found that the AAV mediated optimized sFlt1 gene therapy of the present invention reduced and/or prevented laser induced choroidal neovascularization (CNV) in mice. The above described AAV2 vectors, that being AAV2 - sFlt1 unoptimized (SEQ ID NO: 1), AVV2-OPT-2 (SEQ ID NO: 2, GeneDesign) and AAV2-OPT-8 (SEQ ID NO: 8, GeneScript) and a control wherein no sFlt1 were introduced by subretinal delivery in an amount of about 0.5 microliters. Two weeks later laser photocoagulation was performed on the mice eyes to create a model for CNV. Seven days after the laser treatment the mice were anesthetized and their pupils were removed. Photographs were taken after the intraperitoneal adminstration of FITC-GSA-Lectin as a stain. Figures 14, 15 and 16 showed that the laser photocoagulation caused leakage in both the controls and the AVV2 vector treated eyes, however leakages was reduced in the AAV2-optimized sFlt1 injected eyes. Importantly in the controls it is evident that the leakage increased while the two optimized treated eyes (Figures 15 and 16) showed a reduction of leakage over the period of viewing. AAV-2-Opt-8 showed the greatest reduction in leakage over the period of viewing (1^{st} week, 2^{nd} week and 3^{rd} week), as shown in Figure 16.

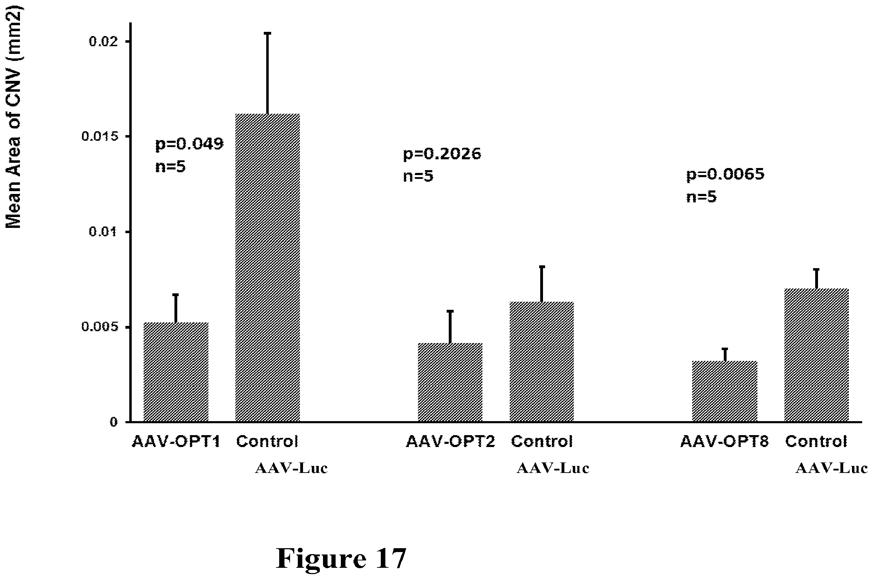

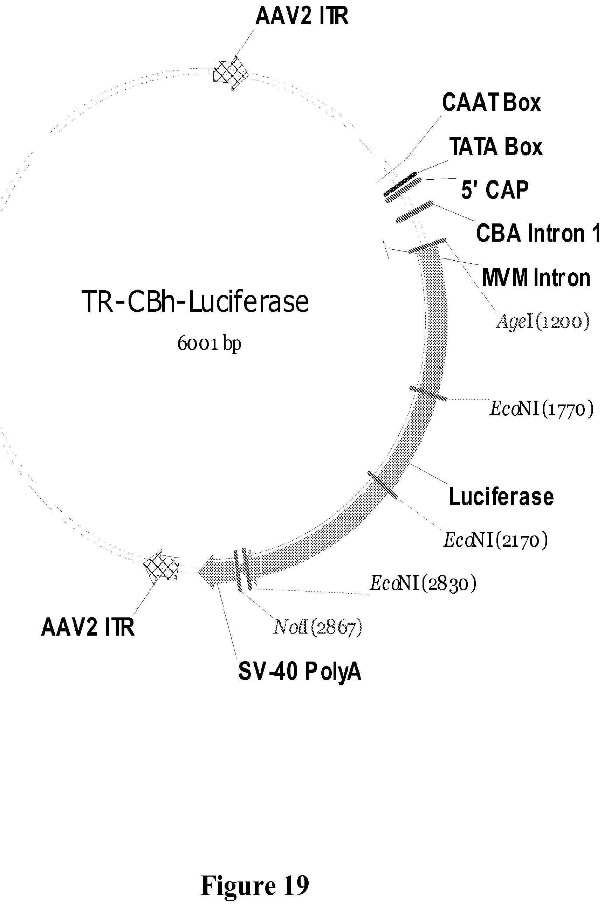

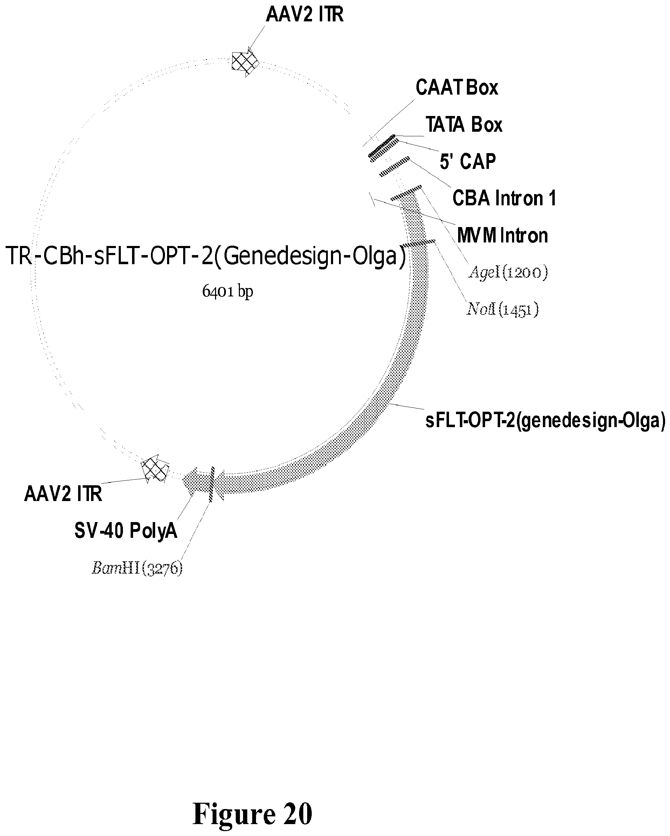

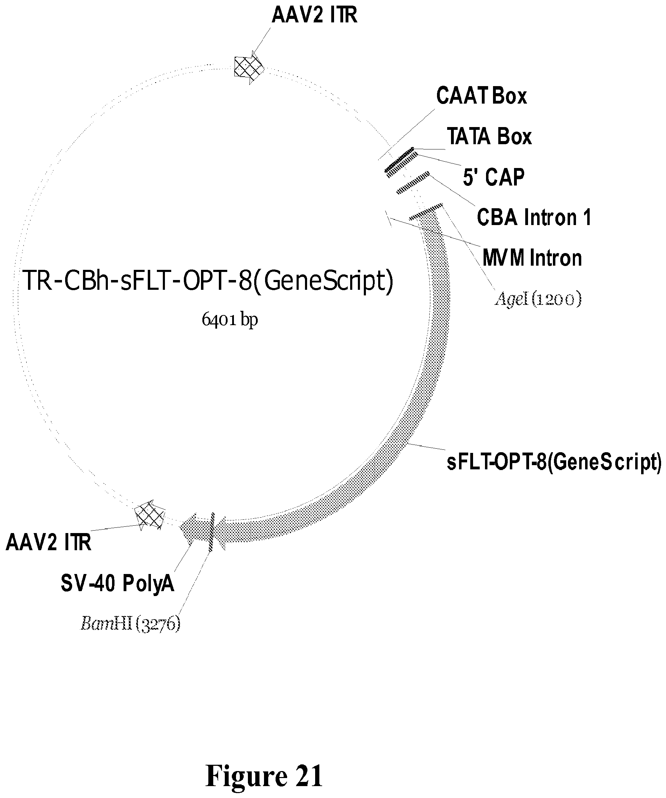

Figure 17 shows the mean area of CNV for the different groupings of AAV2 vectors (viral vector constructs of Figures 20 and 21) compared to the control (viral vector construct of Figure 19) wherein the testing group includes five mice for each result. Clearly, expression by the AAV2-Opt2 and AAV2-Opt8 genes showed the greatest reduction in area affected by CNV because of a reduced amount of leakage.

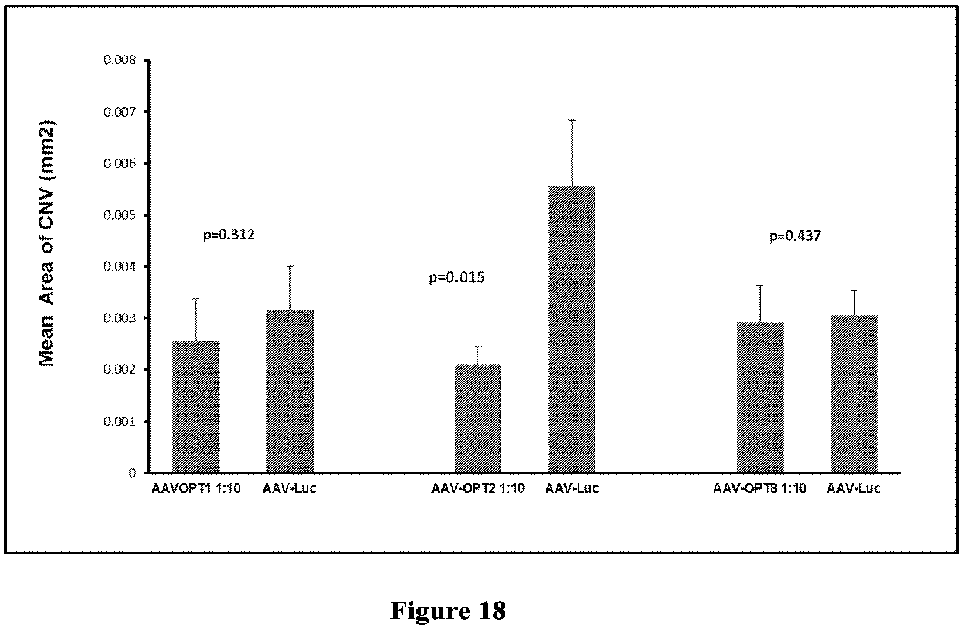

The above test was conducted again wherein the solutions, including the AAV2 vectors, were diluted 10 times and testing results regarding leakage and mean area of CNV is shown in Figure 18. All three vectors showed some inhibition of CNV, however, AAV2-Opt2 still exhibited substantial inhibition effects.

## Claims

1. An optimized sFlt1 gene for use in treating ocular disorder causing neovascularization in a human subject wherein the optimized gene has been modified to increase CG sequences and reduce cis motifs relative to a wild type gene, wherein the optimized gene is selected from the group consisting of SEQ ID NOs: 2 and 8.

2. A virus vector comprising the optimized gene of claim 1.

3. The virus vector according to claim 2 wherein the virus vector is selected from AAV1, AAV2, AAV3, AAV4, AAV5, AAV6, AAV7 AAV8, AAV9, AAV10, AAV11 or AAV12 capsids.

4. The optimized gene for use according to claim 1, wherein the optimized gene is SEQ ID NO: 8.

5. A process of expressing an optimized sFlt1 peptide comprising:
transfecting a cell with polynucleotide that encodes the optimized sFlt1 peptide to produce a transformed host cell, wherein the polynucleotide encoding the optimized sFlt1 gene has been modified to increase CG sequences and reduce cis motifs relative to a wild type gene, wherein the polynucleotide encoding the optimized sFlt1 gene has been selected from the group consisting of SEQ ID NOs: 2 and 8; and
maintaining the transformed host cell under biological conditions sufficient for expression of the peptide.

6. The process of claim 5, wherein the cell is a retina cell.

7. A pharmaceutical composition comprising an admixture of:
an isolated or purified nucleic acid comprising the sequence of:
SEQ ID NOs: 2, 8 or a complement thereof; and
a pharmaceutically acceptable carrier.

## Patentansprüche

1. Ein optimiertes sFlt1 Gen zur Verwendung in der Behandlung einer okularen Störung, welche Neovaskularisation in einem humanen Subjekt verursacht, worin das optimierte Gen modifiziert wurde um CG Sequenzen zu erhöhen und cis Motive zu reduzieren relativ zu einem Wildtyp Gen, worin das optimierte Gen ausgewählt ist aus der Gruppe bestehend aus SEQ ID NO: 2 und 8.

2. Ein Virus-Vektor, umfassend das optimierte Gen von Anspruch 1.

3. Der Virus-Vektor gemäß Anspruch 2, worin der Virus-Vektor ausgewählt ist aus AAV1, AAV2, AAV3, AAV4, AAV5, AAV6, AAV7 AAV8, AAV9, AAV10, AAV11 oder AAV12 Kapsiden.

4. Das optimierte Gen zur Verwendung gemäß Anspruch 1, worin das optimierte Gen SEQ ID NO: 8 ist.

5. Ein Verfahren um ein optimiertes sFlt1 Peptid zu exprimieren, umfassend:
Transfizieren einer Zelle mit einem Polynukleotid, welches das optimierte sFlt1 Peptid kodiert um eine transformierte Wirtszelle zu produzieren, worin das Polynukleotid, welches das optimierte sFlt1 Gen kodiert, modifiziert wurde um CG Sequenzen zu erhöhen und cis Motive zu reduzieren relativ zu einem Wildtyp Gen, worin das Polynukleotid, welches das optimierte sFlt1 Gen kodiert, ausgewählt wurde aus der Gruppe bestehend aus SEQ ID NO: 2 und 8; und
Erhalten der transformierten Wirtszelle unter biologischen Bedingungen, die ausreichend sind für die Expression des Peptids.

6. Das Verfahren von Anspruch 5, worin die Zelle eine Zelle der Retina ist.

7. Eine pharmazeutische Zusammensetzung umfassend eine Beimischung von:
einer isolierten oder gereinigten Nukleinsäure umfassend die Sequenz von:
SEQ ID NO: 2, 8 oder einem Komplement davon; und
einem pharmazeutisch akzeptablem Träger.

## Revendications

1. Gène sFlt1 optimisé pour une utilisation dans le traitement de trouble oculaire provoquant une néovascularisation chez un sujet humain, le gène optimisé ayant été modifié pour augmenter les séquences CG et réduire les motifs cis par rapport à un gène de type sauvage, le gène optimisé étant choisi dans le groupe constitué de SEQ ID NO: 2 et 8.

2. Vecteur viral comprenant le gène optimisé de la revendication 1.

3. Vecteur viral selon la revendication 2, le vecteur viral étant choisi parmi les capsides d'AAV1, AAV2, AAV3, AAV4, AAV5, AAV6, AAV7 AAV8, AAV9, AAV10 AAV11 ou AAV12.

4. Gène optimisé pour une utilisation selon la revendication 1, le gène optimisé étant la SEQ ID NO: 8.

5. Procédé d'expression d'un peptide sFlt1 optimisé comprenant :
la transfection d'une cellule avec un polynucléotide qui code pour le peptide sFlt1 optimisé pour produire une cellule hôte transformée, le polynucléotide codant pour le gène sFlt1 optimisé ayant été modifié pour augmenter les séquences CG et réduire les motifs cis par rapport à un gène de type sauvage, le polynucléotide codant pour le gène sFlt1 optimisé ayant été choisi dans le groupe constitué de SEQ ID NO: 2 et 8; et
le maintien de la cellule hôte transformée dans des conditions biologiques suffisantes pour l'expression du peptide.

6. Procédé de la revendication 5, dans lequel la cellule est une cellule de la rétine.

7. Composition pharmaceutique comprenant un mélange de :
un acide nucléique isolé ou purifié comprenant la séquence de :
SEQ ID NO: 2, 8 ou un complément de celles-ci ; et
un support pharmaceutiquement acceptable.


### SEQUENCE LISTING

<110> Asklepios Biopharmaceutical, Inc.
   Samulski, Richard J.
<120> MODIFIED SOLUBLE VEGF RECEPTOR-1 GENES AND VECTORS FOR GENE THERAPY
<130> 014860.022 PCT
<150> 61/782,450
   <151> 2013-03-14
<160> 26
<170> PatentIn version 3.5
<210> 1
   <211> 2067
   <212> DNA
   <213> Artificial Sequence
<220>
   <223> Synthetic Construct
<400> 1
   atggtcagct actgggacac cggggtcctg ctgtgcgcgc tgctcagctg tctgcttctc      60
   acaggatcta gttcaggttc aaaattaaaa gatcctgaac tgagtttaaa aggcacccag     120
   cacatcatgc aagcaggcca gacactgcat ctccaatgca ggggggaagc agcccataaa     180
   tggtctttgc ctgaaatggt gagtaaggaa agcgaaaggc tgagcataac taaatctgcc     240
   tgtggaagaa atggcaaaca attctgcagt actttaacct tgaacacagc tcaagcaaac     300
   cacactggct tctacagctg caaatatcta gctgtaccta cttcaaagaa gaaggaaaca     360
   gaatctgcaa tctatatatt tattagtgat acaggtagac ctttcgtaga gatgtacagt     420
   gaaatccccg aaattataca catgactgaa ggaagggagc tcgtcattcc ctgccgggtt     480
   acgtcaccta acatcactgt tactttaaaa aagtttccac ttgacacttt gatccctgat     540
   ggaaaacgca taatctggga cagtagaaag ggcttcatca tatcaaatgc aacgtacaaa     600
   gaaatagggc ttctgacctg tgaagcaaca gtcaatgggc atttgtataa gacaaactat     660
   ctcacacatc gacaaaccaa tacaatcata gatgtccaaa taagcacacc acgcccagtc     720
   aaattactta gaggccatac tcttgtcctc aattgtactg ctaccactcc cttgaacacg     780
   agagttcaaa tgacctggag ttaccctgat gaaaaaaata agagagcttc cgtaaggcga     840
   cgaattgacc aaagcaattc ccatgccaac atattctaca gtgttcttac tattgacaaa     900
   atgcagaaca aagacaaagg actttatact tgtcgtgtaa ggagtggacc atcattcaaa     960
   tctgttaaca cctcagtgca tatatatgat aaagcattca tcactgtgaa acatcgaaaa    1020
   cagcaggtgc ttgaaaccgt agctggcaag cggtcttacc ggctctctat gaaagtgaag    1080
   gcatttccct cgccggaagt tgtatggtta aaagatgggt tacctgcgac tgagaaatct    1140
   gctcgctatt tgactcgtgg ctactcgtta attatcaagg acgtaactga agaggatgca    1200
   gggaattata caatcttgct gagcataaaa cagtcaaatg tgtttaaaaa cctcactgcc    1260
   actctaattg tcaatgtgaa accccagatt tacgaaaagg ccgtgtcatc gtttccagac 1320
   ccggctctct acccactggg cagcagacaa atcctgactt gtaccgcata tggtatccct 1380
   caacctacaa tcaagtggtt ctggcacccc tgtaaccata atcattccga agcaaggtgt 1440
   gacttttgtt ccaataatga agagtccttt atcctggatg ctgacagcaa catgggaaac 1500
   agaattgaga gcatcactca gcgcatggca ataatagaag gaaagaataa gatggctagc 1560
   accttggttg tggctgactc tagaatttct ggaatctaca tttgcatagc ttccaataaa 1620
   gttgggactg tgggaagaaa cataagcttt tatatcacag atgtgccaaa tgggtttcat 1680
   gttaacttgg aaaaaatgcc gacggaagga gaggacctga aactgtcttg cacagttaac 1740
   aagttcttat acagagacgt tacttggatt ttactgcgga cagttaataa cagaacaatg 1800
   cactacagta ttagcaagca aaaaatggcc atcactaagg agcactccat cactcttaat 1860
   cttaccatca tgaatgtttc cctgcaagat tcaggcacct atgcctgcag agccaggaat 1920
   gtatacacag gggaagaaat cctccagaag aaagaaatta caatcagagg tgagcactgc 1980
   aacaaaaagg ctgttttctc tcggatctcc aaatttaaaa gcacaaggaa tgattgtacc 2040
   acacaaagta atgtaaaaca ttagtaa 2067
<210> 2
   <211> 2067
   <212> DNA
   <213> Artificial Sequence
<220>
   <223> Synthetic Construct
<400> 2
   atggtgagct actgggacac cggcgtgctg ctgtgcgccc tgctgagctg cctgctgctg      60
   accggcagca gcagcggcag caagctgaag gaccccgagc tgagcctgaa gggcacccag     120
   cacatcatgc aggccggcca gaccctgcac ctgcagtgcc gcggcgaggc cgcccacaag     180
   tggagcctgc ccgagatggt gagcaaggag agcgagcgcc tgagcatcac caagagcgcc     240
   tgcggccgca acggcaagca gttctgcagc accctgaccc tgaacaccgc ccaggccaac     300
   cacaccggct tctacagctg caagtacctg gccgtgccca ccagcaagaa gaaggagacc     360
   gagagcgcca tctacatctt catcagcgac accggccgcc ccttcgtgga gatgtacagc     420
   gagatccccg agatcatcca catgaccgag ggccgcgagc tggtgatccc ctgccgcgtg     480
   accagcccca acatcaccgt gaccctgaag aagttccccc tggacaccct gatccccgac     540
   ggcaagcgca tcatctggga cagccgcaag ggcttcatca tcagcaacgc cacctacaag     600
   gagatcggcc tgctgacctg cgaggccacc gtgaacggcc acctgtacaa gaccaactac     660
   ctgacccacc gccagaccaa caccatcatc gacgtgcaga tcagcacccc ccgccccgtg     720
   aagctgctgc gcggccacac cctggtgctg aactgcaccg ccaccacccc cctgaacacc     780
   cgcgtgcaga tgacctggag ctaccccgac gagaagaaca agcgcgccag cgtgcgccgc     840
   cgcatcgacc agagcaacag ccacgccaac atcttctaca gcgtgctgac catcgacaag      900
   atgcagaaca aggacaaggg cctgtacacc tgccgcgtgc gcagcggccc cagcttcaag      960
   agcgtgaaca ccagcgtgca catctacgac aaggccttca tcaccgtgaa gcaccgcaag     1020
   cagcaggtgc tggagaccgt ggccggcaag cgcagctacc gcctgagcat gaaggtgaag     1080
   gccttcccca gccccgaggt ggtgtggctg aaggacggcc tgcccgccac cgagaagagc     1140
   gcccgctacc tgacccgcgg ctacagcctg atcatcaagg acgtgaccga ggaggacgcc     1200
   ggcaactaca ccatcctgct gagcatcaag cagagcaacg tgttcaagaa cctgaccgcc     1260
   accctgatcg tgaacgtgaa gccccagatc tacgagaagg ccgtgagcag cttccccgac     1320
   cccgccctgt accccctggg cagccgccag atcctgacct gcaccgccta cggcatcccc     1380
   cagcccacca tcaagtggtt ctggcacccc tgcaaccaca accacagcga ggcccgctgc     1440
   gacttctgca gcaacaacga ggagagcttc atcctggacg ccgacagcaa catgggcaac     1500
   cgcatcgaga gcatcaccca gcgcatggcc atcatcgagg gcaagaacaa gatggccagc     1560
   accctggtgg tggccgacag ccgcatcagc ggcatctaca tctgcatcgc cagcaacaag     1620
   gtgggcaccg tgggccgcaa catcagcttc tacatcaccg acgtgcccaa cggcttccac     1680
   gtgaacctgg agaagatgcc caccgagggc gaggacctga agctgagctg caccgtgaac     1740
   aagttcctgt accgcgacgt gacctggatt ctgctgcgca ccgtgaacaa ccgcaccatg     1800
   cactacagca tcagcaagca gaagatggcc atcaccaagg agcacagcat caccctgaac     1860
   ctgaccatca tgaacgtgag cctgcaggac agcggcacct acgcctgccg cgcccgcaac     1920
   gtgtacaccg gcgaggagat cctgcagaag aaggagatca ccatccgcgg cgagcactgc     1980
   aacaagaagg ccgtgttcag ccgcatcagc aagttcaaga gcacccgcaa cgactgcacc     2040
   acccagagca acgtgaagca ttagtaa                                         2067
<210> 3
   <211> 2067
   <212> DNA
   <213> Artificial Sequence
<220>
   <223> Synthetic Construct
<400> 3
   atggtcagct actgggatac cggagtcctg ctctgcgccc tgctctcctg tctgctcctg      60
   acaggctcca gcagcggcag caagctgaaa gaccctgaac tcagcctcaa gggcacacaa     120
   cacatcatgc aggctggaca gaccctccat ctccagtgca gaggcgaggc cgctcacaag     180
   tggtccctcc ctgagatggt gtccaaagaa agcgagaggc tgagcatcac caagagcgct     240
   tgcggcagga atggcaaaca gttctgctcc acactcaccc tgaacacagc tcaagccaat     300
   cacacaggct tctactcctg caaatatctc gccgtcccca caagcaagaa gaaagaaaca     360
   gagtccgcca tttacatttt catctccgat accggcaggc ccttcgtcga aatgtacagc     420
   gagatccccg agatcatcca tatgaccgaa ggcagagagc tcgtcattcc ctgcagggtc     480
   accagcccca atattaccgt gaccctgaaa aagttccccc tcgatacact cattcccgac 540
   ggcaagagga tcatctggga ttccagaaag ggcttcatta ttagcaatgc cacctataag 600
   gagattggac tgctgacctg tgaggccacc gtgaacggcc acctctacaa gaccaactac 660
   ctcacacaca ggcagaccaa caccatcatt gacgtccaga tcagcacccc caggcctgtg 720
   aaactgctca gaggccatac actggtcctc aactgcacag ccacaacacc cctgaacaca 780
   agggtgcaga tgacctggag ctaccctgac gagaaaaaca agagggccag cgtgagaagg 840
   agaattgacc agtccaacag ccacgctaac atcttctatt ccgtcctgac aattgacaag 900
   atgcagaaca aggataaggg cctctatacc tgcagagtca gatccggacc cagctttaaa 960
   tccgtgaata ccagcgtcca catctacgac aaggccttca tcacagtgaa acacaggaag 1020
   cagcaggtgc tcgagaccgt ggccggcaag aggtcctaca ggctgtccat gaaggtcaaa 1080
   gctttccctt cccccgaggt cgtgtggctc aaagacggcc tccccgccac cgagaaaagc 1140
   gctagatacc tcaccagagg ctacagcctg atcatcaagg acgtgacaga agaggatgct 1200
   ggcaactaca ccattctcct gtccatcaag caatccaacg tcttcaagaa cctgaccgcc 1260
   acactcatcg tgaatgtcaa gccccagatc tacgagaagg ccgtgagcag cttccccgat 1320
   cctgccctgt atcccctcgg ctccagacaa attctcacct gcaccgccta cggaattccc 1380
   cagcccacca tcaagtggtt ttggcacccc tgcaaccaca accattccga ggccagatgc 1440
   gatttctgct ccaataacga ggagtccttc atcctcgatg ctgacagcaa catgggaaac 1500
   aggatcgaat ccatcaccca gaggatggcc atcatcgagg gcaaaaataa aatggccagc 1560
   accctggtcg tcgccgacag caggattagc ggcatctaca tttgcatcgc ctccaacaaa 1620
   gtgggcaccg tgggaaggaa tatcagcttc tatatcaccg acgtgcccaa cggatttcac 1680
   gtgaatctgg agaagatgcc taccgaggga gaagatctca agctcagctg caccgtcaac 1740
   aagtttctgt acagggacgt cacatggatt ctgctcagga ccgtcaacaa caggaccatg 1800
   cattactcca tttccaagca gaagatggcc atcaccaagg agcacagcat cacactcaac 1860
   ctgaccatca tgaatgtgtc cctccaggac agcggaacat acgcctgcag ggccagaaac 1920
   gtctacacag gcgaagagat cctgcagaaa aaggagatca ccatcagagg cgagcactgc 1980
   aacaagaagg ccgtcttctc caggatcagc aagttcaaat ccaccaggaa cgactgcaca 2040
   acccaatcca atgtcaagca ttagtaa 2067
<210> 4
   <211> 2067
   <212> DNA
   <213> Artificial Sequence
<220>
   <223> Synthetic Construct
<400> 4
   atggtatcgt actgggatac gggcgtgttg ttgtgcgccc tgctgtcatg tttgcttttg 60
   acagggtcct catcggggtc aaaactgaaa gaccctgagt tgagcctcaa gggaactcag     120
   cacatcatgc aagctggtca gacgctccat ctgcagtgtc ggggagaagc tgcccacaag     180
   tggagcctgc ccgagatggt gagcaaagag tcggagagac tttcgatcac gaaatccgca     240
   tgcggaagaa atgggaaaca gttctgctcc accctgacat tgaataccgc gcaggccaac     300
   cacactggat tctattcgtg taagtacttg gcggtgccca cgagcaaaaa gaaagagact     360
   gagtcggcga tctacatctt tatctcagac acggggaggc cgttcgtgga aatgtattcg     420
   gagattcccg agatcatcca catgacagaa gggcgagagc tcgtcatccc gtgtcgcgta     480
   acttcaccca acatcaccgt gacactcaag aagtttccac tggacacact gattcccgac     540
   ggaaagcgga tcatctggga ttcacgaaag gggtttatca tttccaacgc gacttacaaa     600
   gagatcggac tgctgacgtg cgaggccacc gtcaacggac acctctataa gacgaattat     660
   ctcacgcaca gacagaccaa caccatcatc gacgtacaga tctcaacccc acggccggta     720
   aaactgctca gggggcacac gctcgtactg aattgcacag cgacgacgcc cctgaatacg     780
   agggtccaga tgacctggtc gtacccggac gaaaagaata agcgggcgtc ggtgcggaga     840
   aggatcgacc agtcgaattc acatgctaat atcttctact cggtactcac gatcgataag     900
   atgcagaaca aagataaggg gttgtacact tgtagggtca ggagcgggcc ttcgttcaaa     960
   agcgtaaaca ccagcgtcca catctacgac aaggccttta tcacggtcaa gcataggaag    1020
   cagcaagtac tggaaactgt agcaggaaag agatcatata ggttgtccat gaaagtcaag    1080
   gcgttcccat ccccggaggt cgtatggctt aaggacggac tccccgccac ggaaaagtcg    1140
   gcacgctatt tgacgcgggg ttattcgctg atcattaagg atgtcacaga agaggatgcg    1200
   gggaactata caattcttct ttccatcaag cagtccaatg tgttcaagaa tttgacagca    1260
   accctcatcg taaacgtaaa gcctcaaatc tacgaaaagg cagtgagctc attccctgac    1320
   ccagcgttgt accctctggg ctcgagacag atccttacgt gtactgcgta cgggattccc    1380
   cagcctacca ttaagtggtt ttggcatccc tgcaaccaca accactcgga ggcgaggtgc    1440
   gacttttgca gcaacaacga agaatcgttc atccttgatg cagactcaaa catgggtaat    1500
   cggatcgaat cgatcaccca acgcatggct atcattgagg ggaagaataa gatggcatcg    1560
   actttggtcg tggccgactc gcggatctca ggcatctaca tttgcatcgc aagcaacaaa    1620
   gtgggaacgg tcggacggaa catttcgttc tatatcactg atgtacccaa tgggtttcac    1680
   gtaaacctcg agaaaatgcc tacggaagga gaggatttga agctttcgtg caccgtgaac    1740
   aagtttctct accgcgatgt gacgtggatc ttgcttagaa cggtgaacaa caggacaatg    1800
   cactactcca tctcgaagca gaaaatggca atcactaaag aacatagcat tacgctcaac    1860
   ttgactatta tgaatgtatc gcttcaagat tcggggacct atgcatgtag agctcgcaac    1920
   gtctatacag gcgaagaaat tcttcaaaag aaggagatta ctatccgggg tgagcactgc    1980
   aacaaaaagg cggtctttag ccgaatctca aagttcaaat cgactagaaa cgactgtaca    2040
   acgcagtcaa acgtcaagca ttagtaa                                       2067
<210> 5
   <211> 2067
   <212> DNA
   <213> Artificial Sequence
<220>
   <223> Synthetic Construct
<400> 5
   atggtgtcct actgggatac cggcgtgctg ctgtgtgccc tgctgagctg tctgctgctg      60
   accggctcta gcagcggcag caagctgaag gaccctgagc tgagcctgaa gggcacccag     120
   cacatcatgc aggccggcca gacactgcat ctgcagtgcc ggggagaagc cgcccacaaa     180
   tggtccctgc ctgagatggt gtccaaagag agcgagcggc tgagcatcac caagagcgcc     240
   tgcggcagaa acggcaagca gttctgcagc accctgaccc tgaataccgc ccaggccaac     300
   cacaccggct tctacagctg caagtacctg gccgtgccca ccagcaagaa gaaagaaacc     360
   gagagcgcca tctacatctt catcagcgac accggcagac ccttcgtgga aatgtacagc     420
   gagatccccg agatcatcca catgaccgag ggccgcgagc tcgtgatccc ttgcagagtg     480
   accagcccca acatcaccgt gacactgaag aagttccccc tggacaccct gatccccgac     540
   ggcaagagaa tcatctggga cagccggaag ggcttcatca tcagcaacgc cacctacaaa     600
   gagatcggcc tgctgacctg cgaggccacc gtgaatggcc acctgtacaa gaccaactac     660
   ctgacccaca gacagaccaa caccatcatc gacgtgcaga tcagcacccc cagacccgtg     720
   aagctgctga gaggccacac cctggtgctg aattgcaccg ccaccacccc cctgaacacc     780
   agagtgcaga tgacctggtc ctaccccgac gagaagaaca agagggccag cgtgcggcgg     840
   agaatcgacc agagcaacag ccacgccaac atcttctact ccgtgctgac catcgacaag     900
   atgcagaaca aggacaaggg cctgtacacc tgtagagtgc ggagcggccc cagcttcaag     960
   agcgtgaaca cctccgtgca catctacgac aaggccttca tcacagtgaa gcaccggaag    1020
   cagcaggtgc tggaaaccgt ggccggcaag cggagctaca gactgagcat gaaagtgaaa    1080
   gccttcccca gccccgaggt cgtgtggctg aaagatggac tgcccgccac cgagaagtcc    1140
   gccagatacc tgaccagagg ctacagcctg atcatcaagg acgtgaccga agaggacgcc    1200
   ggcaactaca ccatcctgct gtccatcaag cagagcaacg tgttcaagaa cctgaccgcc    1260
   acactgatcg tgaacgtgaa gccccagatc tatgagaagg ccgtgtccag cttccccgac    1320
   cccgctctgt atcctctggg cagcaggcag atcctgacct gcacagccta cggcatcccc    1380
   cagcccacca tcaagtggtt ctggcacccc tgcaaccaca accacagcga ggccagatgc    1440
   gacttctgct ccaacaacga ggaaagcttc atcctggacg ccgacagcaa catgggcaac    1500
   cggatcgagt ccatcaccca gagaatggcc atcattgagg gcaagaacaa aatggcctct    1560
   accctggtgg tggccgactc cagaatcagc ggcatctata tctgtatcgc cagcaacaaa    1620
   gtgggcaccg tgggccggaa catcagcttc tacatcaccg atgtgcccaa cggcttccac    1680
   gtgaacctgg aaaagatgcc caccgagggc gaggacctga agctgtcctg taccgtgaac 1740
   aagtttctgt accgcgacgt gacctggatt ctgctgcgga cagtgaacaa ccggaccatg 1800
   cactacagca tcagcaagca gaagatggct atcaccaaag agcacagcat caccctgaat 1860
   ctgaccatca tgaacgtgtc actgcaggac agcggcacct acgcctgcag agccagaaac 1920
   gtgtacaccg gcgaggaaat cctgcagaaa aaagagatca ccatccgggg cgagcactgc 1980
   aacaagaaag ccgtgttcag ccggatcagc aagttcaaga gcacccggaa cgactgcacc 2040
   acccagtcca atgtgaagca ctgatga 2067
<210> 6
   <211> 2067
   <212> DNA
   <213> Artificial Sequence
<220>
   <223> Synthetic Construct
<400> 6
   atggtgagct attgggatac aggagttctc ctctgtgcac tgctttcctg tctgttgctt     60
   actggatcaa gctcaggttc taagcttaag gaccccgagt tgtccctgaa agggactcag    120
   cacataatgc aagccggtca gactttgcat ctgcagtgca ggggggaggc cgctcataaa    180
   tggagccttc ccgagatggt gtccaaggag tccgagagac tgtcaatcac taaatcagct    240
   tgtggcagga acggcaagca gttctgtagc acactgactt tgaacaccgc acaggccaat    300
   cacactggct tctactcatg taaatatctg gcggttccca catctaaaaa aaaggaaacc    360
   gaatcagcca tctatatatt tatttcagac accggccgcc ctttcgttga aatgtactcc    420
   gagattcctg aaattattca tatgacagaa gggagggagc tggtgatacc gtgtagggta    480
   accagcccca acatcactgt gactctcaaa aaattccccc tggacacgct gatccccgat    540
   ggaaagcgga ttatttggga ttcacggaaa gggtttatta tttccaacgc cacctacaag    600
   gagatcggac tgctgacgtg tgaggcgact gtcaacggac acctgtacaa aaccaactat    660
   ctgacacatc gccagaccaa taccatcata gatgtgcaaa tctcaacacc aaggcccgtg    720
   aaactgctgc gcggtcacac tctggtgctc aattgcactg caacgacgcc tctgaatacg    780
   cgagtgcaga tgacttggtc ctatcccgat gagaaaaaca agcgcgcctc agtaagaaga    840
   aggattgacc aaagcaacag ccatgccaac atcttctatt cagtcctgac aatcgacaaa    900
   atgcaaaaca aagataaggg cctttatact tgtcgcgtga ggagcggtcc atctttcaaa    960
   agtgtaaata caagtgttca tatttatgat aaagctttca ttacagtgaa acaccgaaaa   1020
   cagcaggtgc tggaaacagt ggctggcaag cgctcctacc gacttagcat gaaggtaaaa   1080
   gcgttccctt ctcctgaagt cgtgtggctg aaggacggcc tgccagctac agaaaagagc   1140
   gctcggtatt tgaccagagg ctacagcctg attatcaagg atgtgactga agaggacgcc   1200
   ggcaattaca caattctgct ttccatcaag cagtcaaacg ttttcaaaaa tctgacagct   1260
   acgttgatcg tgaacgtcaa acctcaaatc tacgagaaag ccgttagcag ctttccagac   1320
   cctgctctct accccctcgg atctcggcaa atcctgacct gtactgcgta tggaatcccc 1380
   caaccaacca tcaagtggtt ttggcaccca tgtaatcaca atcattctga agcccgctgt 1440
   gatttctgct caaacaatga ggagtccttc atcctggacg cagacagcaa tatgggcaat 1500
   cggattgagt caatcacaca gaggatggca attatagagg gaaaaaacaa aatggcgagc 1560
   accctggtcg tggctgactc cagaatcagt ggcatctaca tctgtatcgc ctcaaacaag 1620
   gtcgggacag tcggtcgcaa tatcagcttc tatattacag acgtgcctaa cggttttcat 1680
   gtgaacctcg agaagatgcc tacagaggga gaggatctga aactgtcatg cactgtaaac 1740
   aaattcttgt accgcgacgt cacttggatc ttgctgagaa cagtcaataa caggaccatg 1800
   cactactcaa ttagcaagca gaagatggct atcaccaaag agcactccat cacactgaac 1860
   ctgactatca tgaatgtctc cttgcaggac tctggcactt atgcttgtcg ggcgaggaat 1920
   gtgtatacag gcgaggaaat cctccagaag aaggagatta caattagggg agaacattgt 1980
   aataagaaag cagtatttag tagaatcagt aaatttaagt ccaccaggaa cgactgtact 2040
   actcagtcca acgtaaaaca ttagtaa 2067
<210> 7
   <211> 2067
   <212> DNA
   <213> Artificial Sequence
<220>
   <223> Synthetic Construct
<400> 7
   atggtgtcct actgggacac cggagtgctg ctttgtgctc tgctttcttg cctgctgctt        60
   actggcagct cctcaggaag taagttgaaa gaccccgagc ttagtctgaa gggaacacag       120
   cacatcatgc aagccggaca gacactgcat ctccagtgcc gaggcgaggc agctcacaag 180
   tggtccctgc ccgagatggt gtccaaagaa tctgagaggc tctcaatcac aaagagcgct 240
   tgtgggagga acggcaagca gttctgttcc acactgactc tgaatactgc acaagctaat 300
   cataccggat tttattcctg taaatatctg gccgtgccta ccagtaagaa aaaggaaacc 360
   gagagtgcca tatatatctt cattagcgac acaggtcgcc ctttcgtgga gatgtactca 420
   gaaatccccg agatcatcca catgactgaa ggccgagaac tcgtgatacc ttgtcgggtg 480
   actagtccta atatcactgt cacactgaaa aagttccccc tcgatacact catccccgat 540
   ggaaaacgca ttatctggga cagtcgcaaa ggatttatta tttccaacgc tacatataag 600
   gaaatcgggt tgctcacctg cgaagctacc gtgaacgggc atctctataa gactaattat 660
   ctgacccaca ggcagactaa cacaattata gacgtacaga ttagcacacc cagacctgtc 720
   aagctgcttc gaggccatac tctggttctc aattgcaccg ctaccactcc cctgaatacc 780
   cgggtccaaa tgacatggtc atatccggat gagaagaaca aacgagctag cgtgcgccga 840
   cgcattgacc agtccaatag ccacgcgaac attttttatt ctgttcttac catcgacaag 900
   atgcagaata aagataaggg gttgtatacg tgtcgagtca gaagcgggcc tagcttcaag 960
   agtgtcaaca catccgtcca catatacgat aaagccttta tcactgtgaa gcaccgcaaa 1020
   caacaagtcc tggagacagt ggctggcaag cgatcctata ggctgagcat gaaggtaaag 1080
   gccttcccca gcccggaggt ggtgtggctt aaggatggcc tgcctgcgac agagaaatca 1140
   gcaagatatc tgaccagggg gtactctctt ataatcaaag acgtaacgga agaggatgcc 1200
   ggtaactaca ccatactgct cagcatcaaa cagagtaacg tttttaagaa tttgaccgca 1260
   accctgatag tcaatgtgaa acctcagatc tacgagaaag ccgtgtcttc attccccgac 1320
   cccgccctgt accccctggg ttctcgccag atccttactt gcactgccta cggaattcct 1380
   cagcctacga ttaagtggtt ttggcatcct tgtaaccata accatagcga ggcacggtgc 1440
   gacttctgta gcaataacga ggagtctttc attctggatg cagactctaa tatgggtaac 1500
   cgcattgagt ccatcacgca gaggatggcc attatcgaag ggaaaaataa gatggcctct 1560
   actctggtgg tggctgatag ccgcatctca ggcatttata tctgcatagc ttctaataaa 1620
   gttgggacag tggggaggaa tatctccttc tacattaccg atgtcccaaa cggattccat 1680
   gtgaaccttg aaaaaatgcc gactgagggc gaggatctta agctgtcatg cactgtcaat 1740
   aagtttctgt atcgagacgt gacttggata ctcctgcgga ctgtgaataa ccggactatg 1800
   cattacagca tatctaagca gaaaatggcc atcactaagg aacactccat taccctgaac 1860
   ctcaccataa tgaacgtcag cctccaagat tctggaacct acgcttgcag ggcccgaaat 1920
   gtctacacag gggaggagat actccagaag aaggaaatca ccattcgggg ggaacactgc 1980
   aataagaaag ctgtgttcag caggatttcc aagttcaaaa gcactcgaaa cgactgcacc 2040
   actcagtcaa acgtgaaaca ctagtaa 2067
<210> 8
   <211> 2067
   <212> DNA
   <213> Artificial Sequence
<220>
   <223> Synthetic Construct
<400> 8
   atggtctcat actgggatac tggggtcctg ctgtgcgccc tgctgagttg tctgctgctg       60
   actgggagtt ctagcgggtc caagctgaaa gacccagagc tgagcctgaa ggggactcag      120
   cacattatgc aggctggaca gaccctgcac ctccagtgcc gaggagaggc agctcacaaa      180
   tggtccctgc ccgaaatggt gtccaaggag tctgaaagac tgagtatcac caaatcagca      240
   tgcggcagga acgggaagca gttctgttcc actctgaccc tgaacacagc acaggccaat      300
   cataccggct tctactcttg caagtatctg gccgtgccca ccagtaagaa aaaggagaca      360
   gaatcagcta tctatatttt catcagcgat accggacggc cctttgtgga gatgtacagt      420
   gagatccctg aaatcattca catgactgag ggcagggagc tggtcatccc atgtcgcgtc      480
   acctcaccca atatcacagt gactctgaaa aagttccctc tggacaccct gattccagat      540
   ggaaaacgca tcatttggga ctcccgaaag ggctttatca tctctaacgc aacatacaag 600
   gagatcgggc tgctgacctg cgaagccaca gtgaacggac atctgtacaa gactaattat 660
   ctgacccaca gacagaccaa tacaatcatt gatgtgcaga tcagcacccc acggcctgtc 720
   aagctgctga gaggacatac tctggtcctg aactgtaccg ccaccacacc tctgaatacc 780
   agagtgcaga tgacatggtc ttacccagac gagaaaaaca agagggctag tgtccggaga 840
   aggatcgacc agtctaacag tcacgcaaat attttctata gcgtgctgac aatcgacaag 900
   atgcagaaca aagataaggg cctgtacact tgtcgcgtgc gaagtgggcc ttcattcaaa 960
   agcgtgaata cttccgtcca tatctatgac aaagccttca tcaccgtgaa acaccggaag 1020
   cagcaggtgc tggagacagt cgccgggaaa aggagctacc gcctgtccat gaaagtgaag 1080
   gcttttccat cccccgaggt ggtctggctg aaagatggcc tgccagccac agaaaagagc 1140
   gcccgatacc tgactcgggg gtattccctg atcattaagg acgtgaccga ggaagatgca 1200
   ggaaactaca caatcctgct gagcatcaag cagagtaacg tgttcaagaa tctgactgcc 1260
   accctgattg tgaatgtcaa accccagatc tacgagaagg ccgtgagcag cttccctgac 1320
   ccagcactgt atcctctggg cagccggcag atcctgacat gcactgccta cggcatcccc 1380
   cagcctacca ttaagtggtt ctggcatcct tgtaaccaca atcatagtga agcaaggtgc 1440
   gatttctgtt ccaacaatga ggaatctttt atcctggacg ccgatagtaa catgggcaat 1500
   cgaatcgagt caattaccca gcggatggct atcattgaag ggaaaaacaa gatggcatct 1560
   acactggtgg tcgccgactc ccgcatctct ggcatctaca tctgcattgc ctcaaacaaa 1620
   gtgggaacag tcggccggaa tatcagcttc tacattactg atgtgccaaa cggatttcac 1680
   gtcaatctgg agaagatgcc caccgagggc gaagacctga aactgtcttg tacagtgaat 1740
   aagttcctgt atagggatgt cacttggatt ctgctgagaa ctgtgaacaa taggaccatg 1800
   cattactcaa tcagcaaaca gaagatggct atcaccaagg aacacagcat tacactgaac 1860
   ctgactatca tgaacgtgag cctccaggac agcgggacct acgcttgccg ggcaagaaac 1920
   gtgtatacag gagaggaaat cctccagaag aaggagatca caattcgcgg cgaacactgt 1980
   aacaagaagg ccgtgtttag ccgaatctcc aagttcaagt caaccaggaa tgattgtact 2040
   acccagtcaa atgtcaagca ctagtaa 2067
<210> 9
   <211> 2012
   <212> DNA
   <213> Artificial Sequence
<220>
   <223> Synthetic Construct
<400> 9
   atggtgtgct actgggatac cgggtgctgc tgtgcgccct gctgtgctgt ctgctgctga 60
   cggctccagc tgcggtgcaa gctgaaagac cctgagctga gcctgaaggg caccagcaca 120
   tcatgcaggc cggcagacct gcatctgcag tgccggggga ggccgctcac aagtggtccc      180
   tgcccgagat ggtgtccaag gagtgcgaga ggctgagcat caccaagtgc gctgcggcag      240
   gaacggcaag cagttctgct gcacctgacc ctgaacaccg ccaggccaat cacaccggct      300
   tctactcctg caagtatctg gccgtgccca ccagcaagaa gaaggaaacc gagtcgccat      360
   ctatatcttc atcagcgaca ccggccgccc ttcgtggaga tgtacagcga gatccccgag      420
   atcatccaca tgacgaaggc cggagctcgt gatccctgtc gggtaccagc cccaacatca      480
   ccgtgacctg aaaaagttcc ccctggacac ctgatccccg atggaaagcg catcatctgg      540
   gactgccgaa agggctttat catttccaac gccacctaca aggagatcgg ctgctgacct      600
   gcgaggccac cgtgaacggc acctgtacaa gaccaactat ctgacccaca gacagaccaa      660
   caccatcatg acgtgcagat cagcaccccc cggccgtgaa gctgctgaga ggccatactc      720
   tggtctgaat tgcaccgcca ccaccccctg aataccagag tgcagatgac ctggtcctac      780
   ccgacgagaa gaacaagcgg gccagcgtgc ggcgaagatt gaccagtgca acagccacgc      840
   caacatcttc tattccgtct gacatcgaca agatgcagaa caaagataag ggcctgtata      900
   cttgtcggtg aggagcggcc tgcttcaaaa gcgtgaacac ctgcgtccac atctacgaca      960
   aggccttcat cacgtgaagc accggaagca gcaggtgctg gagaccgtgg ccggcaagcg     1020
   gtcctaccgg ctgtgcatga aggtgaaggc ttcccctccc ccgaggtcgt gtggctgaag     1080
   gatggcctgc ccgccacgag aagtgcgccg atatctgacc cgggctactg cctgatcatc     1140
   aaggacgtga cgaagaggat gccggcaact acaccatcct gctgtgcatc aagcagtgca     1200
   acgtgttcaa gaatctgacc gccaccctga tcgtgaatgt gaagccccag atctacgaga     1260
   aggccgtgag cagcttcccc gacccgcctg taccccctgg gctgccgcag atcctgacct     1320
   gcactgccta cggatccccc agcctaccat caagtggttt tggcacccct gtaaccacaa     1380
   ccattgcgag gccaggtgcg acttctgctc caacaacgag gagtccttca tcctggatgc     1440
   gacagcaaca tgggcaaccg gatcgagtcc atcacccagc ggatggccat catgagggaa     1500
   gaataagatg gcctgcaccc tggtggtggc cgactcccga tctgggcatc tacatctgca     1560
   tcgcctccaa caaagtggga cgtgggcgga atatcagctt ctatatcacc gatgtgccca     1620
   acggtttcac gtgaacctgg agaagatgcc accgagggcg aggatctgaa gctgtctgca     1680
   ccgtgaacaa gtttctgtac cgcgacgtga cttggattct gctgcggacg tgaacaacag     1740
   gaccatgcac tactgcatca gcaagcagaa gatggccatc accaaggagc acagcatcac     1800
   ctgaacctga ccatcatgaa tgtgtccctg caggactgcg gcacctacgc ctgcagggcc     1860
   agaaacgtgt acacaggcga ggaaatcctc cagaagaagg agatcaccat ccggggcgag     1920
   cactgcaaca agaaggccgt gttcagccgg atctgcaagt tcaagtgcac caggaacgac     1980
   tgtaccaccc agtccaatgt aagcattagt aa                                   2012
<210> 10
   <211> 2211
   <212> DNA
   <213> Artificial Sequence
<220>
   <223> Synthetic Construct
<400> 10
   atggctgccg atggttatct tccagattgg ctcgaggaca ctctctctga aggaataaga      60
   cagtggtgga agctcaaacc tggcccacca ccaccaaagc ccgcagagcg gcataaggac     120
   gacagcaggg gtcttgtgct tcctgggtac aagtacctcg gacccttcaa cggactcgac     180
   aagggagagc cggtcaacga ggcagacgcc gcggccctcg agcacgacaa agcctacgac     240
   cggcagctcg acagcggaga caacccgtac ctcaagtaca accacgccga cgcggagttt     300
   caggagcgcc ttaaagaaga tacgtctttt gggggcaacc tcggacgagc agtcttccag     360
   gcgaaaaaga gggttcttga acctctgggc ctggttgagg aacctgttaa gacggctccg     420
   ggaaaaaaga ggccggtaga gcactctcct gtggagccag actcctcctc gggaaccgga     480
   aaggcgggcc agcagcctgc aagaaaaaga ttgaattttg gtcagactgg agacgcagac     540
   tcagtacctg acccccagcc tctcggacag ccaccagcag ccccctctgg tctgggaact     600
   aatacgatgg ctacaggcag tggcgcacca atggcagaca ataacgaggg cgccgacgga     660
   gtgggtaatt cctcgggaaa ttggcattgc gattccacat ggatgggcga cagagtcatc     720
   accaccagca cccgaacctg ggccctgccc acctacaaca accacctcta caaacaaatt     780
   tccagcgctt caacgggagc ctcgaacgac aatcactact ttggctacag caccccttgg     840
   gggtattttg acttcaacag attccactgc cacttttcac cacgtgactg gcaaagactc     900
   atcaacaaca actggggatt ccgacccaag agactcaact tcaagctctt taacattcaa     960
   gtcaaagagg tcacgcagaa tgacggtacg acgacgattg ccaataacct taccagcacg    1020
   gttcaggtgt ttactgactc ggagtaccag ctcccgtacg tcctcggctc ggcgcatcaa    1080
   ggatgcctcc cgccgttccc agcagacgtc ttcatggtgc cacagtatgg atacctcacc    1140
   ctgaacaacg ggagtcaggc agtaggacgc tcttcatttt actgcctgga gtactttcct    1200
   tctcagatgc tgcgtaccgg aaacaacttt accttcagct acacttttga ggacgttcct    1260
   ttccacagca gctacgctca cagccagagt ctggaccgtc tcatgaatcc tctcatcgac    1320
   cagtacctgt attacttgag cagaacaaac actccaagtg gaaccaccac gcagtcaagg    1380
   cttcagtttt ctcaggccgg agcgagtgac attcgggacc agtctaggaa ctggcttcct    1440
   ggaccctgtt accgccagca gcgagtatca aagacatctg cggataacaa caacagtgaa    1500
   tactcgtgga ctggagctac caagtaccac ctcaatggca gagactctct ggtgaatccg    1560
   ggcccggcca tggcaagcca caaggacgat gaagaaaagt tttttcctca gagcggggtt    1620
   ctcatctttg ggaagcaagg ctcagagaaa acaaatgtgg acattgaaaa ggtcatgatt    1680
   acagacgaag aggaaatcag gacaaccaat cccgtggcta cggagcagta tggttctgta    1740
   tctaccaacc tccagagagg caacagacaa gcagctaccg cagatgtcaa cacacaaggc    1800
   gttcttccag gcatggtctg gcaggacaga gatgtgtacc ttcaggggcc catctgggca      1860
   aagattccac acacggacgg acattttcac ccctctcccc tcatgggtgg attcggactt      1920
   aaacaccctc ctccacagat tctcatcaag aacaccccgg tacctgcgaa tccttcgacc      1980
   accttcagtg cggcaaagtt tgcttccttc atcacacagt actccacggg acaggtcagc      2040
   gtggagatcg agtgggagct gcagaaggaa aacagcaaac gctggaatcc cgaaattcag      2100
   tacacttcca actacgccaa gtctgtcaat gtggacttta ctgtggacaa taatggcgtg      2160
   tattcagagc ctcgccccat tggcaccaga tacctgactc gtaatctgta a               2211
<210> 11
   <211> 2211
   <212> DNA
   <213> Artificial Sequence
<220>
   <223> Synthetic Construct
<400> 11
   atggctgccg atggttatct tccagattgg ctcgaggaca acctctctga gggcattcgc      60
   gagtggtggg acttgaaacc tggagccccg aagcccaaag ccaaccagca aaagcaggac     120
   gacggccggg gtctggtgct tcctggctac aagtacctcg gacccttcaa cggactcgac     180
   aagggggagc ccgtcaacgc ggcggacgca gcggccctcg agcacgacaa ggcctacgac     240
   cagcagctca aagcgggtga caatccgtac ctgcggtata accacgccga cgccgagttt     300
   caggagcgtc tgcaagaaga tacgtctttt gggggcaacc tcgggcgagc agtcttccag     360
   gccaagaagc gggttctcga acctctcggt ctggttgagg aaggcgctaa gacggctcct     420
   ggaaagaaac gtccggtaga gcagtcgcca caagagccag actcctcctc gggcatcggc     480
   aagacaggcc agcagcccgc taaaaagaga ctcaattttg gtcagactgg cgactcagag     540
   tcagtccccg atccacaacc tctcggagaa cctccagcaa cccccgctgc tgtgggacct     600
   actacaatgg cttcaggcgg tggcgcacca atggcagaca ataacgaagg cgccgacgga     660
   gtgggtaatg cctcaggaaa ttggcattgc gattccacat ggctgggcga cagagtcatc     720
   accaccagca cccgcacctg ggccttgccc acctacaata accacctcta caagcaaatc     780
   tccagtgctt caacgggggc cagcaacgac aaccactact tcggctacag caccccctgg    840
   gggtattttg atttcaacag attccactgc cacttttcac cacgtgactg gcagcgactc     900
   atcaacaaca attggggatt ccggcccaag agactcaact tcaaactctt caacatccaa     960
   gtcaaggagg tcacgacgaa tgatggcgtc acaaccatcg ctaataacct taccagcacg    1020
   gttcaagtct tctcggactc ggagtaccag cttccgtacg tcctcggctc tgcgcaccag    1080
   ggctgcctcc ctccgttccc ggcggacgtg ttcatgattc cgcaatacgg ctacctgacg    1140
   ctcaacaatg gcagccaagc cgtgggacgt tcatcctttt actgcctgga atatttccct    1200
   tctcagatgc tgagaacggg caacaacttt accttcagct acacctttga ggaagtgcct    1260
   ttccacagca gctacgcgca cagccagagc ctggaccggc tgatgaatcc tctcatcgac    1320
   caatacctgt attacctgaa cagaactcaa aatcagtccg gaagtgccca aaacaaggac      1380
   ttgctgttta gccgtgggtc tccagctggc atgtctgttc agcccaaaaa ctggctacct      1440
   ggaccctgtt atcggcagca gcgcgtttct aaaacaaaaa cagacaacaa caacagcaat      1500
   tttacctgga ctggtgcttc aaaatataac ctcaatgggc gtgaatccat catcaaccct      1560
   ggcactgcta tggcctcaca caaagacgac gaagacaagt tctttcccat gagcggtgtc      1620
   atgatttttg gaaaagagag cgccggagct tcaaacactg cattggacaa tgtcatgatt      1680
   acagacgaag aggaaattaa agccactaac cctgtggcca ccgaaagatt tgggaccgtg      1740
   gcagtcaatt tccagagcag cagcacagac cctgcgaccg gagatgtgca tgctatggga      1800
   gcattacctg gcatggtgtg gcaagataga gacgtgtacc tgcagggtcc catttgggcc      1860
   aaaattcctc acacagatgg acactttcac ccgtctcctc ttatgggcgg ctttggactc      1920
   aagaacccgc ctcctcagat cctcatcaaa aacacgcctg ttcctgcgaa tcctccggcg      1980
   gagttttcag ctacaaagtt tgcttcattc atcacccaat actccacagg acaagtgagt      2040
   gtggaaattg aatgggagct gcagaaagaa aacagcaagc gctggaatcc cgaagtgcag      2100
   tacacatcca attatgcaaa atctgccaac gttgatttta ctgtggacaa caatggactt      2160
   tatactgagc ctcgccccat tggcacccgt taccttaccc gtcccctgta a               2211
<210> 12
   <211> 2208
   <212> DNA
   <213> Artificial Sequence
<220>
   <223> Synthetic Construct
<400> 12
   atggctgccg atggttatct tccagattgg ctcgaggaca acctctctga gggcattcgc        60
   gagtggtggg acttgaaacc tggagccccg aagcccaaag ccaaccagca aaagcaggac       120
   gacggccggg gtctggtgct tcctggctac aagtacctcg gacccttcaa cggactcgac       180
   aagggggagc ccgtcaacgc ggcggacgca gcggccctcg agcacgacaa ggcctacgac       240
   cagcagctca aagcgggtga caatccgtac ctgcggtata accacgccga cgccgagttt       300
   caggagcgtc tgcaagaaga tacgtctttt gggggcaacc tcgggcgagc agtcttccag       360
   gccaagaagc gggttctcga acctctcggt ctggttgagg aaggcgctaa gacggctcct       420
   ggaaagaaac gtccggtaga gcagtcgcca caagagccag actcctcctc gggcatcggc       480
   aagacaggcc agcagcccgc taaaaagaga ctcaattttg gtcagactgg cgactcagag       540
   tcagtccccg atccacaacc tctcggagaa cctccagcaa cccccgctgc tgtgggacct       600
   actacaatgg cttcaggcgg tggcgcacca atggcagaca ataacgaagg cgccgacgga       660
   gtgggtaatg cctcaggaaa ttggcattgc gattccacat ggctgggcga cagagtcatc       720
   accaccagca cccgcacctg ggccttgccc acctacaata accacctcta caagcaaatc       780
   tccagtgctt caggggccag caacgacaac cactacttcg gctacagcac cccctggggg 840
   tattttgatt tcaacagatt ccactgccac ttttcaccac gtgactggca gcgactcatc 900
   aacaacaatt ggggattccg gcccaagaga ctcaacttca aactcttcaa catccaagtc 960
   aaggaggtca cgacgaatga tggcgtcaca accatcgcta ataaccttac cagcacggtt 1020
   caagtcttct cggactcgga gtaccagctt ccgtacgtcc tcggctctgc gcaccagggc 1080
   tgcctccctc cgttcccggc ggacgtgttc atgattccgc aatacggcta cctgacgctc 1140
   aacaatggca gccaagccgt gggacgttca tccttttact gcctggaata tttcccttct 1200
   cagatgctga gaacgggcaa caactttacc ttcagctaca cctttgagga agtgcctttc 1260
   cacagcagct acgcgcacag ccagagcctg gaccggctga tgaatcctct catcgaccaa 1320
   tacctgtatt acctgaacag aactcaaaat cagtccggaa gtgcccaaaa caaggacttg 1380
   ctgtttagcc gtgggtctcc agctggcatg tctgttcagc ccaaaaactg gctacctgga 1440
   ccctgttatc ggcagcagcg cgtttctaaa acaaaaacag acaacaacaa cagcaatttt 1500
   acctggactg gtgcttcaaa atataacctc aatgggcgtg aatccatcat caaccctggc 1560
   actgctatgg cctcacacaa agacgacgaa gacaagttct ttcccatgag cggtgtcatg 1620
   atttttggaa aagagagcgc cggagcttca aacactgcat tggacaatgt catgattaca 1680
   gacgaagagg aaattaaagc cactaaccct gtggccaccg aaagatttgg gaccgtggca 1740
   gtcaatttcc agagcagcag cacagaccct gcgaccggag atgtgcatgc tatgggagca 1800
   ttacctggca tggtgtggca agatagagac gtgtacctgc agggtcccat ttgggccaaa 1860
   attcctcaca cagatggaca ctttcacccg tctcctctta tgggcggctt tggactcaag 1920
   aacccgcctc ctcagatcct catcaaaaac acgcctgttc ctgcgaatcc tccggcggag 1980
   ttttcagcta caaagtttgc ttcattcatc acccaatact ccacaggaca agtgagtgtg 2040
   gaaattgaat gggagctgca gaaagaaaac agcaagcgct ggaatcccga agtgcagtac 2100
   acatccaatt atgcaaaatc tgccaacgtt gattttactg tggacaacaa tggactttat 2160
   actgagcctc gccccattgg cacccgttac cttacccgtc ccctgtaa 2208
<210> 13
   <211> 2211
   <212> DNA
   <213> Artificial Sequence
<220>
   <223> Synthetic Construct
<400> 13
   atggctgccg atggttatct tccagattgg ctcgaggaca acctctctga gggcattcgc 60
   gagtggtggg acttgaaacc tggagccccg aaacccaaag ccaaccagca aaagcaggac 120
   gacggccggg gtctggtgct tcctggctac aagtacctcg gacccttcaa cggactcgac 180
   aagggggagc ccgtcaacgc ggcggatgca gcggccctcg agcacgacaa ggcctacgac 240
   cagcagctca aagcgggtga caatccgtac ctgcggtata accacgccga cgccgagttt 300
   caggagcgtc tgcaagaaga tacgtctttt gggggcaacc tcgggcgagc agtcttccag      360
   gccaagaaga gggttctcga accttttggt ctggttgagg aaggtgctaa gacggctcct      420
   ggaaagaaac gtccggtaga gcagtcgcca caagagccag actcctcctc gggcattggc      480
   aagacaggcc agcagcccgc taaaaagaga ctcaattttg gtcagactgg cgactcagag      540
   tcagtccccg acccacaacc tctcggagaa cctccagcaa cccccgctgc tgtgggacct      600
   actacaatgg cttcaggcgg tggcgcacca atggcagaca ataacgaagg cgccgacgga      660
   gtgggtaatg cctcaggaaa ttggcattgc gattccacat ggctgggcga cagagtcatc      720
   accaccagca cccgaacatg ggccttgccc acctataaca accacctcta caagcaaatc      780
   tccagtgctt caacgggggc cagcaacgac aaccactact tcggctacag caccccctgg      840
   gggtattttg atttcaacag attccactgc catttctcac cacgtgactg gcagcgactc      900
   atcaacaaca attggggatt ccggcccaag agactcaact tcaagctctt caacatccaa      960
   gtcaaggagg tcacgacgaa tgatggcgtc acgaccatcg ctaataacct taccagcacg     1020
   gttcaagtct tctcggactc ggagtaccag ttgccgtacg tcctcggctc tgcgcaccag     1080
   ggctgcctcc ctccgttccc ggcggacgtg ttcatgattc cgcagtacgg ctacctaacg     1140
   ctcaacaatg gcagccaggc agtgggacgg tcatcctttt actgcctgga atatttccca     1200
   tcgcagatgc tgagaacggg caataacttt accttcagct acaccttcga ggacgtgcct     1260
   ttccacagca gctacgcgca cagccagagc ctggaccggc tgatgaatcc tctcatcgac     1320
   cagtacctgt attacctgaa cagaactcag aatcagtccg gaagtgccca aaacaaggac     1380
   ttgctgttta gccgggggtc tccagctggc atgtctgttc agcccaaaaa ctggctacct     1440
   ggaccctgtt accggcagca gcgcgtttct aaaacaaaaa cagacaacaa caacagcaac     1500
   tttacctgga ctggtgcttc aaaatataac cttaatgggc gtgaatctat aatcaaccct     1560
   ggcactgcta tggcctcaca caaagacgac aaagacaagt tctttcccat gagcggtgtc     1620
   atgatttttg gaaaggagag cgccggagct tcaaacactg cattggacaa tgtcatgatc     1680
   acagacgaag aggaaatcaa agccactaac cccgtggcca ccgaaagatt tgggactgtg     1740
   gcagtcaatc tccagagcag cagcacagac cctgcgaccg gagatgtgca tgttatggga     1800
   gccttacctg gaatggtgtg gcaagacaga gacgtatacc tgcagggtcc tatttggccc     1860
   aaaattcctc acacggatgg acactttcac ccgtctcctc tcatgggcgg ctttggactt     1920
   aagcacccgc ctcctcagat cctcatcaaa aacacgcctg ttcctgcgaa tcctccggca     1980
   gagttttcgg ctacaaagtt tgcttcattc atcacccagt attccacagg acaagtgagc     2040
   gtggagattg aatgggagct gcagaaagaa aacagcaaac gctggaatcc cgaagtgcag     2100
   tatacatcta actatgcaaa atctgccaac gttgatttca ctgtggacaa caatggactt     2160
   tatactgagc ctcgccccat tggcacccgt tacctcaccc gtcccctgta a              2211
<210> 14
   <211> 2208
   <212> DNA
   <213> Artificial Sequence
<220>
   <223> Synthetic Construct
<400> 14
   atggctgccg atggttatct tccagattgg ctcgaggaca acctctctga gggcattcgc     60
   gagtggtggg acttgaaacc tggagccccg aaacccaaag ccaaccagca aaagcaggac    120
   gacggccggg gtctggtgct tcctggctac aagtacctcg gacccttcaa cggactcgac    180
   aagggggagc ccgtcaacgc ggcggatgca gcggccctcg agcacgacaa ggcctacgac    240
   cagcagctca aagcgggtga caatccgtac ctgcggtata accacgccga cgccgagttt    300
   caggagcgtc tgcaagaaga tacgtctttt gggggcaacc tcgggcgagc agtcttccag    360
   gccaagaaga gggttctcga accttttggt ctggttgagg aaggtgctaa gacggctcct    420
   ggaaagaaac gtccggtaga gcagtcgcca caagagccag actcctcctc gggcattggc    480
   aagacaggcc agcagcccgc taaaaagaga ctcaattttg gtcagactgg cgactcagag    540
   tcagtccccg acccacaacc tctcggagaa cctccagcaa cccccgctgc tgtgggacct    600
   actacaatgg cttcaggcgg tggcgcacca atggcagaca ataacgaagg cgccgacgga    660
   gtgggtaatg cctcaggaaa ttggcattgc gattccacat ggctgggcga cagagtcatc    720
   accaccagca cccgaacatg ggccttgccc acctataaca accacctcta caagcaaatc    780
   tccagtgctt caggggccag caacgacaac cactacttcg gctacagcac cccctggggg    840
   tattttgatt tcaacagatt ccactgccat ttctcaccac gtgactggca gcgactcatc    900
   aacaacaatt ggggattccg gcccaagaga ctcaacttca agctcttcaa catccaagtc    960
   aaggaggtca cgacgaatga tggcgtcacg accatcgcta ataaccttac cagcacggtt   1020
   caagtcttct cggactcgga gtaccagttg ccgtacgtcc tcggctctgc gcaccagggc   1080
   tgcctccctc cgttcccggc ggacgtgttc atgattccgc agtacggcta cctaacgctc   1140
   aacaatggca gccaggcagt gggacggtca tccttttact gcctggaata tttcccatcg   1200
   cagatgctga gaacgggcaa taactttacc ttcagctaca ccttcgagga cgtgcctttc   1260
   cacagcagct acgcgcacag ccagagcctg gaccggctga tgaatcctct catcgaccag   1320
   tacctgtatt acctgaacag aactcagaat cagtccggaa gtgcccaaaa caaggacttg   1380
   ctgtttagcc gggggtctcc agctggcatg tctgttcagc ccaaaaactg gctacctgga   1440
   ccctgttacc ggcagcagcg cgtttctaaa acaaaaacag acaacaacaa cagcaacttt   1500
   acctggactg gtgcttcaaa atataacctt aatgggcgtg aatctataat caaccctggc   1560
   actgctatgg cctcacacaa agacgacaaa gacaagttct ttcccatgag cggtgtcatg   1620
   atttttggaa aggagagcgc cggagcttca aacactgcat tggacaatgt catgatcaca   1680
   gacgaagagg aaatcaaagc cactaacccc gtggccaccg aaagatttgg gactgtggca   1740
   gtcaatctcc agagcagcag cacagaccct gcgaccggag atgtgcatgt tatgggagcc   1800
   ttacctggaa tggtgtggca agacagagac gtatacctgc agggtcctat ttgggccaaa 1860
   attcctcaca cggatggaca ctttcacccg tctcctctca tgggcggctt tggacttaag 1920
   cacccgcctc ctcagatcct catcaaaaac acgcctgttc ctgcgaatcc tccggcagag 1980
   ttttcggcta caaagtttgc ttcattcatc acccagtatt ccacaggaca agtgagcgtg 2040
   gagattgaat gggagctgca gaaagaaaac agcaaacgct ggaatcccga agtgcagtat 2100
   acatctaact atgcaaaatc tgccaacgtt gatttcactg tggacaacaa tggactttat 2160
   actgagcctc gccccattgg cacccgttac ctcacccgtc ccctgtaa 2208
<210> 15
   <211> 2208
   <212> DNA
   <213> Artificial Sequence
<220>
   <223> Syntheitc Constuct
<400> 15
   atggctgccg atggttatct tccagattgg ctcgaggaca acctctctga gggcattcgc 60
   gagtggtggg acttgaaacc tggagccccg aaacccaaag ccaaccagca aaagcaggac 120
   gacggccggg gtctggtgct tcctggctac aagtacctcg gacccttcaa cggactcgac 180
   aagggggagc ccgtcaacgc ggcggatgca gcggccctcg agcacgacaa ggcctacgac 240
   cagcagctca aagcgggtga caatccgtac ctgcggtata accacgccga cgccgagttt 300
   caggagcgtc tgcaagaaga tacgtctttt gggggcaacc tcgggcgagc agtcttccag 360
   gccaagaaga gggttctcga accttttggt ctggttgagg aaggtgctaa gacggctcct 420
   ggaaagaaac gtccggtaga gcagtcgcca caagagccag actcctcctc gggcattggc 480
   aagacaggcc agcagcccgc taaaaagaga ctcaattttg gtcagactgg cgactcagag 540
   tcagtccccg acccacaacc tctcggagaa cctccagcaa cccccgctgc tgtgggacct 600
   actacaatgg cttcaggcgg tggcgcacca atggcagaca ataacgaagg cgccgacgga 660
   gtgggtaatg cctcaggaaa ttggcattgc gattccacat ggctgggcga cagagtcatc 720
   accaccagca cccgaacatg ggccttgccc acctataaca accacctcta caagcaaatc 780
   tccagtgctt caggggccag caacgacaac cactacttcg gctacagcac cccctggggg 840
   tattttgatt tcaacagatt ccactgccat ttctcaccac gtgactggca gcgactcatc 900
   aacaacaatt ggggattccg gcccaagaga ctcaacttca agctcttcaa catccaagtc 960
   aaggaggtca cgacgaatga tggcgtcacg accatcgcta ataaccttac cagcacggtt 1020
   caagtcttct cggactcgga gtaccagttg ccgtacgtcc tcggctctgc gcaccagggc 1080
   tgcctccctc cgttcccggc ggacgtgttc atgattccgc agtacggcta cctaacgctc 1140
   aacaatggca gccaggcagt gggacggtca tccttttact gcctggaata tttcccatcg 1200
   cagatgctga gaacgggcaa taactttacc ttcagctaca ccttcgagga cgtgcctttc 1260
   cacagcagct acgcgcacag ccagagcctg gaccggctga tgaatcctct catcgaccag 1320
   tacctgtatt acctgaacag aactcagaat cagtccggaa gtgcccaaaa caaggacttg 1380
   ctgtttagcc gggggtctcc agctggcatg tctgttcagc ccaaaaactg gctacctgga 1440
   ccctgttacc ggcagcagcg cgtttctaaa acaaaaacag acaacaacaa cagcaacttt 1500
   acctggactg gtgcttcaaa atataacctt aatgggcgtg aatctataat caaccctggc 1560
   actgctatgg cctcacacaa agacgacgaa gacaagttct ttcccatgag cggtgtcatg 1620
   atttttggaa aggagagcgc cggagcttca aacactgcat tggacaatgt catgatcaca 1680
   gacgaagagg aaatcaaagc cactaacccc gtggccaccg aaagatttgg gactgtggca 1740
   gtcaatctcc agagcagcag cacagaccct gcgaccggag atgtgcatgt tatgggagcc 1800
   ttacctggaa tggtgtggca agacagagac gtatacctgc agggtcctat ttgggccaaa 1860
   attcctcaca cggatggaca ctttcacccg tctcctctca tgggcggctt tggacttaag 1920
   cacccgcctc ctcagatcct catcaaaaac acgcctgttc ctgcgaatcc tccggcagag 1980
   ttttcggcta caaagtttgc ttcattcatc acccagtatt ccacaggaca agtgagcgtg 2040
   gagattgaat gggagctgca gaaagaaaac agcaaacgct ggaatcccga agtgcagtat 2100
   acatctaact atgcaaaatc tgccaacgtt gatttcactg tggacaacaa tggactttat 2160
   actgagcctc gccccattgg cacccgttac ctcacccgtc ccctgtaa 2208
<210> 16
   <211> 897
   <212> DNA
   <213> Artificial Sequence
<220>
   <223> Synthetic Construct
<400> 16
   ctgggccttg cccacctaca ataaccacct ctacaagcaa atctccagtg cttcaacggg        60
   ggccagcaac gacaaccact acttcggcta cagcaccccc tgggggtatt ttgatttcaa       120
   cagattccac tgccactttt caccacgtga ctggcagcga ctcatcaaca acaattgggg       180
   attccggccc aagagactca acttcaaact cttcaacatc caagtcaagg aggtcacgac       240
   gaatgatggc gtcacaacca tcgctaataa ccttaccagc acggttcaag tcttctcgga       300
   ctcggagtac cagcttccgt acgtcctcgg ctctgcgcac cagggctgcc tccctccgtt       360
   cccggcggac gtgttcatga ttccgcaata cggctacctg acgctcaaca atggcagcca       420
   agccgtggga cgttcatcct tttactgcct ggaatatttc ccttctcaga tgctgagaac       480
   gggcaacaac tttaccttca gctacacctt tgaggaagtg cctttccaca gcagctacgc       540
   gcacagccag agcctggacc ggctgatgaa tcctctcatc gaccaatacc tgtattacct       600
   gaacagaact caaaatcagt ccggaagtgc ccaaaacaag gacttgctgt ttagccgtgg       660
   gtctccagct ggcatgtctg ttcagcccaa aaactggcta cctggaccct gttatcggca       720
   gcagcgcgtt tctaaaacaa aaacagacaa caacaacagc aattttacct ggactggtgc       780
   ttcaaaatat aacctcaatg ggcgtgaatc catcatcaac cctggcactg ctatggcctc       840
   acacaaagac gacgaagaca agttctttcc catgagcggt gtcatgattt ttggaaa          897
<210> 17
   <211> 894
   <212> DNA
   <213> Artificial Sequence
<220>
   <223> Synthetic Construct
<400> 17
   ctgggccttg cccacctaca ataaccacct ctacaagcaa atctccagtg cttcaggggc 60
   cagcaacgac aaccactact tcggctacag caccccctgg gggtattttg atttcaacag 120
   attccactgc cacttttcac cacgtgactg gcagcgactc atcaacaaca attggggatt 180
   ccggcccaag agactcaact tcaaactctt caacatccaa gtcaaggagg tcacgacgaa 240
   tgatggcgtc acaaccatcg ctaataacct taccagcacg gttcaagtct tctcggactc 300
   ggagtaccag cttccgtacg tcctcggctc tgcgcaccag ggctgcctcc ctccgttccc 360
   ggcggacgtg ttcatgattc cgcaatacgg ctacctgacg ctcaacaatg gcagccaagc 420
   cgtgggacgt tcatcctttt actgcctgga atatttccct tctcagatgc tgagaacggg 480
   caacaacttt accttcagct acacctttga ggaagtgcct ttccacagca gctacgcgca 540
   cagccagagc ctggaccggc tgatgaatcc tctcatcgac caatacctgt attacctgaa 600
   cagaactcaa aatcagtccg gaagtgccca aaacaaggac ttgctgttta gccgtgggtc 660
   tccagctggc atgtctgttc agcccaaaaa ctggctacct ggaccctgtt atcggcagca 720
   gcgcgtttct aaaacaaaaa cagacaacaa caacagcaat tttacctgga ctggtgcttc 780
   aaaatataac ctcaatgggc gtgaatccat catcaaccct ggcactgcta tggcctcaca 840
   caaagacgac gaagacaagt tctttcccat gagcggtgtc atgatttttg gaaa 894
<210> 18
   <211> 897
   <212> DNA
   <213> Artificial Sequence
<220>
   <223> Synthetic construct
<400> 18
   atgggccttg cccacctata acaaccacct ctacaagcaa atctccagtg cttcaacggg 60
   ggccagcaac gacaaccact acttcggcta cagcaccccc tgggggtatt ttgatttcaa 120
   cagattccac tgccatttct caccacgtga ctggcagcga ctcatcaaca acaattgggg 180
   attccggccc aagagactca acttcaagct cttcaacatc caagtcaagg aggtcacgac 240
   gaatgatggc gtcacgacca tcgctaataa ccttaccagc acggttcaag tcttctcgga 300
   ctcggagtac cagttgccgt acgtcctcgg ctctgcgcac cagggctgcc tccctccgtt 360
   cccggcggac gtgttcatga ttccgcagta cggctaccta acgctcaaca atggcagcca      420
   ggcagtggga cggtcatcct tttactgcct ggaatatttc ccatcgcaga tgctgagaac      480
   gggcaataac tttaccttca gctacacctt cgaggacgtg cctttccaca gcagctacgc      540
   gcacagccag agcctggacc ggctgatgaa tcctctcatc gaccagtacc tgtattacct      600
   gaacagaact cagaatcagt ccggaagtgc ccaaaacaag gacttgctgt ttagccgggg      660
   gtctccagct ggcatgtctg ttcagcccaa aaactggcta cctggaccct gttaccggca      720
   gcagcgcgtt tctaaaacaa aaacagacaa caacaacagc aactttacct ggactggtgc      780
   ttcaaaatat aaccttaatg ggcgtgaatc tataatcaac cctggcactg ctatggcctc      840
   acacaaagac gacaaagaca agttctttcc catgagcggt gtcatgattt ttggaaa         897
<210> 19
   <211> 894
   <212> DNA
   <213> Artificial Sequence
<220>
   <223> Synthetic Construct
<400> 19
   atgggccttg cccacctata acaaccacct ctacaagcaa atctccagtg cttcaggggc       60
   cagcaacgac aaccactact tcggctacag caccccctgg gggtattttg atttcaacag     120
   attccactgc catttctcac cacgtgactg gcagcgactc atcaacaaca attggggatt      180
   ccggcccaag agactcaact tcaagctctt caacatccaa gtcaaggagg tcacgacgaa      240
   tgatggcgtc acgaccatcg ctaataacct taccagcacg gttcaagtct tctcggactc      300
   ggagtaccag ttgccgtacg tcctcggctc tgcgcaccag ggctgcctcc ctccgttccc      360
   ggcggacgtg ttcatgattc cgcagtacgg ctacctaacg ctcaacaatg gcagccaggc      420
   agtgggacgg tcatcctttt actgcctgga atatttccca tcgcagatgc tgagaacggg      480
   caataacttt accttcagct acaccttcga ggacgtgcct ttccacagca gctacgcgca      540
   cagccagagc ctggaccggc tgatgaatcc tctcatcgac cagtacctgt attacctgaa      600
   cagaactcag aatcagtccg gaagtgccca aaacaaggac ttgctgttta gccgggggtc      660
   tccagctggc atgtctgttc agcccaaaaa ctggctacct ggaccctgtt accggcagca      720
   gcgcgtttct aaaacaaaaa cagacaacaa caacagcaac tttacctgga ctggtgcttc      780
   aaaatataac cttaatgggc gtgaatctat aatcaaccct ggcactgcta tggcctcaca      840
   caaagacgac aaagacaagt tctttcccat gagcggtgtc atgatttttg gaaa            894
<210> 20
   <211> 894
   <212> DNA
   <213> Artificial Sequence
<220>
   <223> Synthetic Construct
<400> 20
   atgggccttg cccacctata acaaccacct ctacaagcaa atctccagtg cttcaggggc     60
   cagcaacgac aaccactact tcggctacag caccccctgg gggtattttg atttcaacag   120
   attccactgc catttctcac cacgtgactg gcagcgactc atcaacaaca attggggatt    180
   ccggcccaag agactcaact tcaagctctt caacatccaa gtcaaggagg tcacgacgaa    240
   tgatggcgtc acgaccatcg ctaataacct taccagcacg gttcaagtct tctcggactc    300
   ggagtaccag ttgccgtacg tcctcggctc tgcgcaccag ggctgcctcc ctccgttccc    360
   ggcggacgtg ttcatgattc cgcagtacgg ctacctaacg ctcaacaatg gcagccaggc    420
   agtgggacgg tcatcctttt actgcctgga atatttccca tcgcagatgc tgagaacggg    480
   caataacttt accttcagct acaccttcga ggacgtgcct ttccacagca gctacgcgca    540
   cagccagagc ctggaccggc tgatgaatcc tctcatcgac cagtacctgt attacctgaa    600
   cagaactcag aatcagtccg gaagtgccca aaacaaggac ttgctgttta gccgggggtc    660
   tccagctggc atgtctgttc agcccaaaaa ctggctacct ggaccctgtt accggcagca    720
   gcgcgtttct aaaacaaaaa cagacaacaa caacagcaac tttacctgga ctggtgcttc    780
   aaaatataac cttaatgggc gtgaatctat aatcaaccct ggcactgcta tggcctcaca    840
   caaagacgac gaagacaagt tctttcccat gagcggtgtc atgatttttg gaaa           894
<210> 21
   <211> 187
   <212> DNA
   <213> Artificial Sequence
<220>
   <223> Synthetic Construct
<400> 21
   atgggccttg cccacctata acaaccacct ctacaagcaa atctccagtg cttcaggggc     60
   cagcaacgac aaccactact tcggctacag cacgtgaatc tataatcaac cctggcactg    120
   ctatggcctc acacaaagac gacgaagaca agttctttcc catgagcggt gtcatgattt    180
   ttggaaa                                                              187
<210> 22
   <211> 2208
   <212> DNA
   <213> Artificial Sequence
<220>
   <223> Synthetic Construct
<400> 22
   atggctgccg atggttatct tccagattgg ctcgaggaca ctctctctga aggaataaga      60
   cagtggtgga agctcaaacc tggcccacca ccaccaaagc ccgcagagcg gcataaggac     120
   gacagcaggg gtcttgtgct tcctgggtac aagtacctcg gacccttcaa cggactcgac     180
   aagggagagc cggtcaacga ggcagacgcc gcggccctcg agcacgacaa agcctacgac     240
   cggcagctcg acagcggaga caacccgtac ctcaagtaca accacgccga cgcggagttt 300
   caggagcgcc ttaaagaaga tacgtctttt gggggcaacc tcggacgagc agtcttccag 360
   gcgaaaaaga gggttcttga acctctgggc ctggttgagg aacctgttaa gacggctccg 420
   ggaaaaaaga ggccggtaga gcactctcct gtggagccag actcctcctc gggaaccgga 480
   aaggcgggcc agcagcctgc aagaaaaaga ttgaattttg gtcagactgg agacgcagac 540
   tcagtacctg acccccagcc tctcggacag ccaccagcag ccccctctgg tctgggaact 600
   aatacgatgg ctacaggcag tggcgcacca atggcagaca ataacgaggg cgccgacgga 660
   gtgggtaatt cctcgggaaa ttggcattgc gattccacat ggatgggcga cagagtcatc 720
   accaccagca cccgaacctg ggccctgccc acctacaaca accacctcta caaacaaatt 780
   tccagccaat caggagcctc gaacgacaat cactactttg gctacagcac cccttggggg 840
   tattttgact tcaacagatt ccactgccac ttttcaccac gtgactggca aagactcatc 900
   aacaacaact ggggattccg acccaagaga ctcaacttca agctctttaa cattcaagtc 960
   aaagaggtca cgcagaatga cggtacgacg acgattgcca ataaccttac cagcacggtt 1020
   caggtgttta ctgactcgga gtaccagctc ccgtacgtcc tcggctcggc gcatcaagga 1080
   tgcctcccgc cgttcccagc agacgtcttc atggtgccac agtatggata cctcaccctg 1140
   aacaacggga gtcaggcagt aggacgctct tcattttact gcctggagta ctttccttct 1200
   cagatgctgc gtaccggaaa caactttacc ttcagctaca cttttgagga cgttcctttc 1260
   cacagcagct acgctcacag ccagagtctg gaccgtctca tgaatcctct catcgaccag 1320
   tacctgtatt acttgagcag aacaaacact ccaagtggaa ccaccacgca gtcaaggctt 1380
   cagttttctc aggccggagc gagtgacatt cgggaccagt ctaggaactg gcttcctgga 1440
   ccctgttacc gccagcagcg agtatcaaag acatctgcgg ataacaacaa cagtgaatac 1500
   tcgtggactg gagctaccaa gtaccacctc aatggcagag actctctggt gaatccgggc 1560
   ccggccatgg caagccacaa ggacgatgaa gaaaagtttt ttcctcagag cggggttctc 1620
   atctttggga agcaaggctc agagaaaaca aatgtggaca ttgaaaaggt catgattaca 1680
   gacgaagagg aaatcaggac aaccaatccc gtggctacgg agcagtatgg ttctgtatct 1740
   accaacctcc agagaggcaa cagacaagca gctaccgcag atgtcaacac acaaggcgtt 1800
   cttccaggca tggtctggca ggacagagat gtgtaccttc aggggcccat ctgggcaaag 1860
   attccacaca cggacggaca ttttcacccc tctcccctca tgggtggatt cggacttaaa 1920
   caccctcctc cacagattct catcaagaac accccggtac ctgcgaatcc ttcgaccacc 1980
   ttcagtgcgg caaagtttgc ttccttcatc acacagtact ccacgggaca ggtcagcgtg 2040
   gagatcgagt gggagctgca gaaggaaaac agcaaacgct ggaatcccga aattcagtac 2100
   acttccaact acaacaagtc tgttaatgtg gactttactg tggacactaa tggcgtgtat 2160
   tcagagcctc gccccattgg caccagatac ctgactcgta atctgtaa 2208
<210> 23
   <211> 6001
   <212> DNA
   <213> Artificial Sequence
<220>
   <223> Synthetic construct
<400> 23
   gggggggggg gggggggttg gccactccct ctctgcgcgc tcgctcgctc actgaggccg      60
   ggcgaccaaa ggtcgcccga cgcccgggct ttgcccgggc ggcctcagtg agcgagcgag     120
   cgcgcagaga gggagtggcc aactccatca ctaggggttc ctagatcttc aatattggcc     180
   attagccata ttattcattg gttatatagc ataaatcaat attggatatt ggccattgca     240
   tacgttgtat ctatatcata atatgtacat ttatattggc tcatgtccaa tatgaccgcc     300
   atgttggcat tgattattga ctagttatta atagtaatca attacggggt cattagttca     360
   tagcccatat atggagttcc gcgttacata acttacggta aatggcccgc ctggctgacc     420
   gcccaacgac ccccgcccat tgacgtcaat aatgacgtat gttcccatag taacgccaat     480
   agggactttc cattgacgtc aatgggtgga gtatttacgg taaactgccc acttggcagt     540
   acatcaagtg tatcatatgc caagtccgcc ccctattgac gtcaatgacg gtaaatggcc     600
   cgcctggcat tatgcccagt acatgacctt acgggacttt cctacttggc agtacatcta     660
   cgtattagtc atcgctatta ccatggtcga ggtgagcccc acgttctgct tcactctccc     720
   catctccccc ccctccccac ccccaatttt gtatttattt atttttaatt tattttgtgc      780
   agcgatgggg gcgggggggg ggggggggcg cgcgccaggc ggggcggggc ggggcgaggg      840
   gcggggcggg gcgaggcgga gaggtgcggc ggcagccaat cagagcggcg cgctccgaaa     900
   gtttcctttt atggcgaggc ggcggcggcg gcggccctat aaaaagcgaa gcgcgcggcg     960
   ggcgggagtc gctgcgacgc tgccttcgcc ccgtgccccg ctccgccgcc gcctcgcgcc    1020
   gcccgccccg gctctgactg accgcgttac tcccacaggt gagcgggcgg gacggccctt    1080
   ctcctccggg ctgtaattag ctgagcaaga ggtaagggtt taagggatgg ttggttggtg    1140
   gggtattaat gtttaattac ctggagcacc tgcctgaaat cacttttttt caggttggac    1200
   cggtcgccac catggaagac gccaaaaaca taaagaaagg cccggcgcca ttctatccgc    1260
   tggaagatgg aaccgctgga gagcaactgc ataaggctat gaagagatac gccctggttc    1320
   ctggaacaat tgcttttaca gatgcacata tcgaggtgga catcacttac gctgagtact    1380
   tcgaaatgtc cgttcggttg gcagaagcta tgaaacgata tgggctgaat acaaatcaca    1440
   gaatcgtcgt atgcagtgaa aactctcttc aattctttat gccggtgttg ggcgcgttat    1500
   ttatcggagt tgcagttgcg cccgcgaacg acatttataa tgaacgtgaa ttgctcaaca    1560
   gtatgggcat ttcgcagcct accgtggtgt tcgtttccaa aaaggggttg caaaaaattt    1620
   tgaacgtgca aaaaaagctc ccaatcatcc aaaaaattat tatcatggat tctaaaacgg    1680
   attaccaggg atttcagtcg atgtacacgt tcgtcacatc tcatctacct cccggtttta    1740
   atgaatacga ttttgtgcca gagtccttcg atagggacaa gacaattgca ctgatcatga 1800
   actcctctgg atctactggt ctgcctaaag gtgtcgctct gcctcataga actgcctgcg 1860
   tgagattctc gcatgccaga gatcctattt ttggcaatca aatcattccg gatactgcga 1920
   ttttaagtgt tgttccattc catcacggtt ttggaatgtt tactacactc ggatatttga 1980
   tatgtggatt tcgagtcgtc ttaatgtata gatttgaaga agagctgttt ctgaggagcc 2040
   ttcaggatta caagattcaa agtgcgctgc tggtgccaac cctattctcc ttcttcgcca 2100
   aaagcactct gattgacaaa tacgatttat ctaatttaca cgaaattgct tctggtggcg 2160
   ctcccctctc taaggaagtc ggggaagcgg ttgccaagag gttccatctg ccaggtatca 2220
   ggcaaggata tgggctcact gagactacat cagctattct gattacaccc gagggggatg 2280
   ataaaccggg cgcggtcggt aaagttgttc cattttttga agcgaaggtt gtggatctgg 2340
   ataccgggaa aacgctgggc gttaatcaaa gaggcgaact gtgtgtgaga ggtcctatga 2400
   ttatgtccgg ttatgtaaac aatccggaag cgaccaacgc cttgattgac aaggatggat 2460
   ggctacattc tggagacata gcttactggg acgaagacga acacttcttc atcgttgacc 2520
   gcctgaagtc tctgattaag tacaaaggct atcaggtggc tcccgctgaa ttggaatcca 2580
   tcttgctcca acaccccaac atcttcgacg caggtgtcgc aggtcttccc gacgatgacg 2640
   ccggtgaact tcccgccgcc gttgttgttt tggagcacgg aaagacgatg acggaaaaag 2700
   agatcgtgga ttacgtcgcc agtcaagtaa caaccgcgaa aaagttgcgc ggaggagttg 2760
   tgtttgtgga cgaagtaccg aaaggtctta ccggaaaact cgacgcaaga aaaatcagag 2820
   agatcctcat aaaggccaag aagggcggaa agatcgccgt gtaagcggcc gcggggatcc 2880
   agacatgata agatacattg atgagtttgg acaaaccaca actagaatgc agtgaaaaaa 2940
   atgctttatt tgtgaaattt gtgatgctat tgctttattt gtaaccatta taagctgcaa 3000
   taaacaagtt aacaacaaca attgcattca ttttatgttt caggttcagg gggagatgtg 3060
   ggaggttttt taaagcaagt aaaacctcta caaatgtggt aaaatcgata aggatctagg 3120
   aacccctagt gatggagttg gccactccct ctctgcgcgc tcgctcgctc actgaggccg 3180
   cccgggcaaa gcccgggcgt cgggcgacct ttggtcgccc ggcctcagtg agcgagcgag 3240
   cgcgcagaga gggagtggcc aacccccccc cccccccccc tgcagcctgg cgtaatagcg 3300
   aagaggcccg caccgatcgc ccttcccaac agttgcgtag cctgaatggc gaatggcgcg 3360
   acgcgccctg tagcggcgca ttaagcgcgg cgggtgtggt ggttacgcgc agcgtgaccg 3420
   ctacacttgc cagcgcccta gcgcccgctc ctttcgcttt cttcccttcc tttctcgcca 3480
   cgttcgccgg ctttccccgt caagctctaa atcgggggct ccctttaggg ttccgattta 3540
   gtgctttacg gcacctcgac cccaaaaaac ttgattaggg tgatggttca cgtagtgggc 3600
   catcgccctg atagacggtt tttcgccctt tgacgttgga gtccacgttc tttaatagtg 3660
   gactcttgtt ccaaactgga acaacactca accctatctc ggtctattct tttgatttat 3720
   aagggatttt gccgatttcg gcctattggt taaaaaatga gctgatttaa caaaaattta 3780
   acgcgaattt taacaaaata ttaacgttta caatttcctg atgcgctatt ttctccttac 3840
   gcatctgtgc ggtatttcac accgcatatg gtgcactctc agtacaatct gctctgatgc 3900
   cgcatagtta agccagcccc gacacccgcc aacacccgct gacgcgccct gacgggcttg 3960
   tctgctcccg gcatccgctt acagacaagc tgtgaccgtc tccgggagct gcatgtgtca 4020
   gaggttttca ccgtcatcac cgaaacgcgc gagacgaaag ggcctcgtga tacgcctatt 4080
   tttataggtt aatgtcatga taataatggt ttcttagacg tcaggtggca cttttcgggg 4140
   aaatgtgcgc ggaaccccta tttgtttatt tttctaaata cattcaaata tgtatccgct 4200
   catgagacaa taaccctgat aaatgcttca ataatattga aaaaggaaga gtatgagtat 4260
   tcaacatttc cgtgtcgccc ttattccctt ttttgcggca ttttgccttc ctgtttttgc 4320
   tcacccagaa acgctggtga aagtaaaaga tgctgaagat cagttgggtg cacgagtggg 4380
   ttacatcgaa ctggatctca acagcggtaa gatccttgag agttttcgcc ccgaagaacg 4440
   ttttccaatg atgagcactt ttaaagttct gctatgtggc gcggtattat cccgtattga 4500
   cgccgggcaa gagcaactcg gtcgccgcat acactattct cagaatgact tggttgagta 4560
   ctcaccagtc acagaaaagc atcttacgga tggcatgaca gtaagagaat tatgcagtgc 4620
   tgccataacc atgagtgata acactgcggc caacttactt ctgacaacga tcggaggacc 4680
   gaaggagcta accgcttttt tgcacaacat gggggatcat gtaactcgcc ttgatcgttg 4740
   ggaaccggag ctgaatgaag ccataccaaa cgacgagcgt gacaccacga tgcctgtagc 4800
   aatggcaaca acgttgcgca aactattaac tggcgaacta cttactctag cttcccggca 4860
   acaattaata gactggatgg aggcggataa agttgcagga ccacttctgc gctcggccct 4920
   tccggctggc tggtttattg cggataaatc tggagccggt gagcgtgggt ctcgcggtat 4980
   cattgcagca ctggggccag atggtaagcc ctcccgtatc gtagttatct acacgacggg 5040
   gagtcaggca actatggatg aacgaaatag acagatcgct gagataggtg cctcactgat 5100
   taagcattgg taactgtcag accaagttta ctcatatata ctttagattg atttaaaact 5160
   tcatttttaa tttaaaagga tctaggtgaa gatccttttt gataatctca tgaccaaaat 5220
   cccttaacgt gagttttcgt tccactgagc gtcagacccc gtagaaaaga tcaaaggatc 5280
   ttcttgagat cctttttttc tgcgcgtaat ctgctgcttg caaacaaaaa aaccaccgct 5340
   accagcggtg gtttgtttgc cggatcaaga gctaccaact ctttttccga aggtaactgg 5400
   cttcagcaga gcgcagatac caaatactgt ccttctagtg tagccgtagt taggccacca 5460
   cttcaagaac tctgtagcac cgcctacata cctcgctctg ctaatcctgt taccagtggc 5520
   tgctgccagt ggcgataagt cgtgtcttac cgggttggac tcaagacgat agttaccgga 5580
   taaggcgcag cggtcgggct gaacgggggg ttcgtgcaca cagcccagct tggagcgaac 5640
   gacctacacc gaactgagat acctacagcg tgagcattga gaaagcgcca cgcttcccga 5700
   agggagaaag gcggacaggt atccggtaag cggcagggtc ggaacaggag agcgcacgag 5760
   ggagcttcca gggggaaacg cctggtatct ttatagtcct gtcgggtttc gccacctctg 5820
   acttgagcgt cgatttttgt gatgctcgtc aggggggcgg agcctatgga aaaacgccag 5880
   caacgcggcc tttttacggt tcctggcctt ttgctggcct tttgctcaca tgttctttcc 5940
   tgcgttatcc cctgattctg tggataaccg tattaccgcc tttgagtgag ctgataccgc 6000
   t 6001
<210> 24
   <211> 6401
   <212> DNA
   <213> Artificial Sequence
<220>
   <223> Synthetic construct
<400> 24
   gggggggggg gggggggttg gccactccct ctctgcgcgc tcgctcgctc actgaggccg      60
   ggcgaccaaa ggtcgcccga cgcccgggct ttgcccgggc ggcctcagtg agcgagcgag      120
   cgcgcagaga gggagtggcc aactccatca ctaggggttc ctagatcttc aatattggcc      180
   attagccata ttattcattg gttatatagc ataaatcaat attggatatt ggccattgca      240
   tacgttgtat ctatatcata atatgtacat ttatattggc tcatgtccaa tatgaccgcc      300
   atgttggcat tgattattga ctagttatta atagtaatca attacggggt cattagttca      360
   tagcccatat atggagttcc gcgttacata acttacggta aatggcccgc ctggctgacc      420
   gcccaacgac ccccgcccat tgacgtcaat aatgacgtat gttcccatag taacgccaat      480
   agggactttc cattgacgtc aatgggtgga gtatttacgg taaactgccc acttggcagt      540
   acatcaagtg tatcatatgc caagtccgcc ccctattgac gtcaatgacg gtaaatggcc      600
   cgcctggcat tatgcccagt acatgacctt acgggacttt cctacttggc agtacatcta      660
   cgtattagtc atcgctatta ccatggtcga ggtgagcccc acgttctgct tcactctccc      720
   catctccccc ccctccccac ccccaatttt gtatttattt attttttaat tattttgtgc      780
   agcgatgggg gcgggggggg ggggggggcg cgcgccaggc ggggcggggc ggggcgaggg      840
   gcggggcggg gcgaggcgga gaggtgcggc ggcagccaat cagagcggcg cgctccgaaa      900
   gtttcctttt atggcgaggc ggcggcggcg gcggccctat aaaaagcgaa gcgcgcggcg      960
   ggcgggagtc gctgcgacgc tgccttcgcc ccgtgccccg ctccgccgcc gcctcgcgcc     1020
   gcccgccccg gctctgactg accgcgttac tcccacaggt gagcgggcgg gacggccctt     1080
   ctcctccggg ctgtaattag ctgagcaaga ggtaagggtt taagggatgg ttggttggtg     1140
   gggtattaat gtttaattac ctggagcacc tgcctgaaat cacttttttt caggttggac     1200
   cggtaccatg gtgagctact gggacaccgg cgtgctgctg tgcgccctgc tgagctgcct     1260
   gctgctgacc ggcagcagca gcggcagcaa gctgaaggac cccgagctga gcctgaaggg     1320
   cacccagcac atcatgcagg ccggccagac cctgcacctg cagtgccgcg gcgaggccgc     1380
   ccacaagtgg agcctgcccg agatggtgag caaggagagc gagcgcctga gcatcaccaa     1440
   gagcgcctgc ggccgcaacg gcaagcagtt ctgcagcacc ctgaccctga acaccgccca    1500
   ggccaaccac accggcttct acagctgcaa gtacctggcc gtgcccacca gcaagaagaa    1560
   ggagaccgag agcgccatct acatcttcat cagcgacacc ggccgcccct tcgtggagat    1620
   gtacagcgag atccccgaga tcatccacat gaccgagggc cgcgagctgg tgatcccctg    1680
   ccgcgtgacc agccccaaca tcaccgtgac cctgaagaag ttccccctgg acaccctgat   1740
   ccccgacggc aagcgcatca tctgggacag ccgcaagggc ttcatcatca gcaacgccac    1800
   ctacaaggag atcggcctgc tgacctgcga ggccaccgtg aacggccacc tgtacaagac    1860
   caactacctg acccaccgcc agaccaacac catcatcgac gtgcagatca gcaccccccg    1920
   ccccgtgaag ctgctgcgcg gccacaccct ggtgctgaac tgcaccgcca ccacccccct    1980
   gaacacccgc gtgcagatga cctggagcta ccccgacgag aagaacaagc gcgccagcgt    2040
   gcgccgccgc atcgaccaga gcaacagcca cgccaacatc ttctacagcg tgctgaccat    2100
   cgacaagatg cagaacaagg acaagggcct gtacacctgc cgcgtgcgca gcggccccag    2160
   cttcaagagc gtgaacacca gcgtgcacat ctacgacaag gccttcatca ccgtgaagca    2220
   ccgcaagcag caggtgctgg agaccgtggc cggcaagcgc agctaccgcc tgagcatgaa    2280
   ggtgaaggcc ttccccagcc ccgaggtggt gtggctgaag gacggcctgc ccgccaccga    2340
   gaagagcgcc cgctacctga cccgcggcta cagcctgatc atcaaggacg tgaccgagga    2400
   ggacgccggc aactacacca tcctgctgag catcaagcag agcaacgtgt tcaagaacct    2460
   gaccgccacc ctgatcgtga acgtgaagcc ccagatctac gagaaggccg tgagcagctt    2520
   ccccgacccc gccctgtacc ccctgggcag ccgccagatc ctgacctgca ccgcctacgg    2580
   catcccccag cccaccatca agtggttctg gcacccctgc aaccacaacc acagcgaggc    2640
   ccgctgcgac ttctgcagca acaacgagga gagcttcatc ctggacgccg acagcaacat    2700
   gggcaaccgc atcgagagca tcacccagcg catggccatc atcgagggca agaacaagat    2760
   ggccagcacc ctggtggtgg ccgacagccg catcagcggc atctacatct gcatcgccag    2820
   caacaaggtg ggcaccgtgg gccgcaacat cagcttctac atcaccgacg tgcccaacgg    2880
   cttccacgtg aacctggaga agatgcccac cgagggcgag gacctgaagc tgagctgcac    2940
   cgtgaacaag ttcctgtacc gcgacgtgac ctggattctg ctgcgcaccg tgaacaaccg    3000
   caccatgcac tacagcatca gcaagcagaa gatggccatc accaaggagc acagcatcac    3060
   cctgaacctg accatcatga acgtgagcct gcaggacagc ggcacctacg cctgccgcgc    3120
   ccgcaacgtg tacaccggcg aggagatcct gcagaagaag gagatcacca tccgcggcga    3180
   gcactgcaac aagaaggccg tgttcagccg catcagcaag ttcaagagca cccgcaacga    3240
   ctgcaccacc cagagcaacg tgaagcatta gtaaggatcc agacatgata agatacattg    3300
   atgagtttgg acaaaccaca actagaatgc agtgaaaaaa atgctttatt tgtgaaattt    3360
   gtgatgctat tgctttattt gtaaccatta taagctgcaa taaacaagtt aacaacaaca    3420
   attgcattca ttttatgttt caggttcagg gggagatgtg ggaggttttt taaagcaagt 3480
   aaaacctcta caaatgtggt aaaatcgata aggatctagg aacccctagt gatggagttg 3540
   gccactccct ctctgcgcgc tcgctcgctc actgaggccg cccgggcaaa gcccgggcgt 3600
   cgggcgacct ttggtcgccc ggcctcagtg agcgagcgag cgcgcagaga gggagtggcc 3660
   aacccccccc cccccccccc tgcagcctgg cgtaatagcg aagaggcccg caccgatcgc 3720
   ccttcccaac agttgcgtag cctgaatggc gaatggcgcg acgcgccctg tagcggcgca 3780
   ttaagcgcgg cgggtgtggt ggttacgcgc agcgtgaccg ctacacttgc cagcgcccta 3840
   gcgcccgctc ctttcgcttt cttcccttcc tttctcgcca cgttcgccgg ctttccccgt 3900
   caagctctaa atcgggggct ccctttaggg ttccgattta gtgctttacg gcacctcgac 3960
   cccaaaaaac ttgattaggg tgatggttca cgtagtgggc catcgccctg atagacggtt 4020
   tttcgccctt tgacgttgga gtccacgttc tttaatagtg gactcttgtt ccaaactgga 4080
   acaacactca accctatctc ggtctattct tttgatttat aagggatttt gccgatttcg 4140
   gcctattggt taaaaaatga gctgatttaa caaaaattta acgcgaattt taacaaaata 4200
   ttaacgttta caatttcctg atgcgctatt ttctccttac gcatctgtgc ggtatttcac 4260
   accgcatatg gtgcactctc agtacaatct gctctgatgc cgcatagtta agccagcccc 4320
   gacacccgcc aacacccgct gacgcgccct gacgggcttg tctgctcccg gcatccgctt 4380
   acagacaagc tgtgaccgtc tccgggagct gcatgtgtca gaggttttca ccgtcatcac 4440
   cgaaacgcgc gagacgaaag ggcctcgtga tacgcctatt tttataggtt aatgtcatga 4500
   taataatggt ttcttagacg tcaggtggca cttttcgggg aaatgtgcgc ggaaccccta 4560
   tttgtttatt tttctaaata cattcaaata tgtatccgct catgagacaa taaccctgat 4620
   aaatgcttca ataatattga aaaaggaaga gtatgagtat tcaacatttc cgtgtcgccc 4680
   ttattccctt ttttgcggca ttttgccttc ctgtttttgc tcacccagaa acgctggtga 4740
   aagtaaaaga tgctgaagat cagttgggtg cacgagtggg ttacatcgaa ctggatctca 4800
   acagcggtaa gatccttgag agttttcgcc ccgaagaacg ttttccaatg atgagcactt 4860
   ttaaagttct gctatgtggc gcggtattat cccgtattga cgccgggcaa gagcaactcg 4920
   gtcgccgcat acactattct cagaatgact tggttgagta ctcaccagtc acagaaaagc 4980
   atcttacgga tggcatgaca gtaagagaat tatgcagtgc tgccataacc atgagtgata 5040
   acactgcggc caacttactt ctgacaacga tcggaggacc gaaggagcta accgcttttt 5100
   tgcacaacat gggggatcat gtaactcgcc ttgatcgttg ggaaccggag ctgaatgaag 5160
   ccataccaaa cgacgagcgt gacaccacga tgcctgtagc aatggcaaca acgttgcgca 5220
   aactattaac tggcgaacta cttactctag cttcccggca acaattaata gactggatgg 5280
   aggcggataa agttgcagga ccacttctgc gctcggccct tccggctggc tggtttattg 5340
   cggataaatc tggagccggt gagcgtgggt ctcgcggtat cattgcagca ctggggccag 5400
   atggtaagcc ctcccgtatc gtagttatct acacgacggg gagtcaggca actatggatg 5460
   aacgaaatag acagatcgct gagataggtg cctcactgat taagcattgg taactgtcag      5520
   accaagttta ctcatatata ctttagattg atttaaaact tcatttttaa tttaaaagga      5580
   tctaggtgaa gatccttttt gataatctca tgaccaaaat cccttaacgt gagttttcgt      5640
   tccactgagc gtcagacccc gtagaaaaga tcaaaggatc ttcttgagat cctttttttc      5700
   tgcgcgtaat ctgctgcttg caaacaaaaa aaccaccgct accagcggtg gtttgtttgc      5760
   cggatcaaga gctaccaact ctttttccga aggtaactgg cttcagcaga gcgcagatac      5820
   caaatactgt ccttctagtg tagccgtagt taggccacca cttcaagaac tctgtagcac      5880
   cgcctacata cctcgctctg ctaatcctgt taccagtggc tgctgccagt ggcgataagt      5940
   cgtgtcttac cgggttggac tcaagacgat agttaccgga taaggcgcag cggtcgggct      6000
   gaacgggggg ttcgtgcaca cagcccagct tggagcgaac gacctacacc gaactgagat      6060
   acctacagcg tgagcattga gaaagcgcca cgcttcccga agggagaaag gcggacaggt      6120
   atccggtaag cggcagggtc ggaacaggag agcgcacgag ggagcttcca gggggaaacg      6180
   cctggtatct ttatagtcct gtcgggtttc gccacctctg acttgagcgt cgatttttgt      6240
   gatgctcgtc aggggggcgg agcctatgga aaaacgccag caacgcggcc tttttacggt      6300
   tcctggcctt ttgctggcct tttgctcaca tgttctttcc tgcgttatcc cctgattctg      6360
   tggataaccg tattaccgcc tttgagtgag ctgataccgc t                          6401
<210> 25
   <211> 6401
   <212> DNA
   <213> Artificial Sequence
<220>
   <223> Synthetic Construct
<400> 25
   gggggggggg gggggggttg gccactccct ctctgcgcgc tcgctcgctc actgaggccg         60
   ggcgaccaaa ggtcgcccga cgcccgggct ttgcccgggc ggcctcagtg agcgagcgag        120
   cgcgcagaga gggagtggcc aactccatca ctaggggttc ctagatcttc aatattggcc        180
   attagccata ttattcattg gttatatagc ataaatcaat attggatatt ggccattgca        240
   tacgttgtat ctatatcata atatgtacat ttatattggc tcatgtccaa tatgaccgcc        300
   atgttggcat tgattattga ctagttatta atagtaatca attacggggt cattagttca        360
   tagcccatat atggagttcc gcgttacata acttacggta aatggcccgc ctggctgacc        420
   gcccaacgac ccccgcccat tgacgtcaat aatgacgtat gttcccatag taacgccaat        480
   agggactttc cattgacgtc aatgggtgga gtatttacgg taaactgccc acttggcagt        540
   acatcaagtg tatcatatgc caagtccgcc ccctattgac gtcaatgacg gtaaatggcc        600
   cgcctggcat tatgcccagt acatgacctt acgggacttt cctacttggc agtacatcta        660
   cgtattagtc atcgctatta ccatggtcga ggtgagcccc acgttctgct tcactctccc        720
   catctccccc ccctccccac ccccaatttt gtatttattt attttttaat tattttgtgc    780
   agcgatgggg gcgggggggg ggggggggcg cgcgccaggc ggggcggggc ggggcgaggg    840
   gcggggcggg gcgaggcgga gaggtgcggc ggcagccaat cagagcggcg cgctccgaaa    900
   gtttcctttt atggcgaggc ggcggcggcg gcggccctat aaaaagcgaa gcgcgcggcg    960
   ggcgggagtc gctgcgacgc tgccttcgcc ccgtgccccg ctccgccgcc gcctcgcgcc   1020
   gcccgccccg gctctgactg accgcgttac tcccacaggt gagcgggcgg gacggccctt   1080
   ctcctccggg ctgtaattag ctgagcaaga ggtaagggtt taagggatgg ttggttggtg   1140
   gggtattaat gtttaattac ctggagcacc tgcctgaaat cacttttttt caggttggac   1200
   cggtaccatg gtctcatact gggatactgg ggtcctgctg tgcgccctgc tgagttgtct   1260
   gctgctgact gggagttcta gcgggtccaa gctgaaagac ccagagctga gcctgaaggg   1320
   gactcagcac attatgcagg ctggacagac cctgcacctc cagtgccgag gagaggcagc   1380
   tcacaaatgg tccctgcccg aaatggtgtc caaggagtct gaaagactga gtatcaccaa   1440
   atcagcatgc ggcaggaacg ggaagcagtt ctgttccact ctgaccctga acacagcaca   1500
   ggccaatcat accggcttct actcttgcaa gtatctggcc gtgcccacca gtaagaaaaa   1560
   ggagacagaa tcagctatct atattttcat cagcgatacc ggacggccct ttgtggagat   1620
   gtacagtgag atccctgaaa tcattcacat gactgagggc agggagctgg tcatcccatg   1680
   tcgcgtcacc tcacccaata tcacagtgac tctgaaaaag ttccctctgg acaccctgat   1740
   tccagatgga aaacgcatca tttgggactc ccgaaagggc tttatcatct ctaacgcaac   1800
   atacaaggag atcgggctgc tgacctgcga agccacagtg aacggacatc tgtacaagac   1860
   taattatctg acccacagac agaccaatac aatcattgat gtgcagatca gcaccccacg   1920
   gcctgtcaag ctgctgagag gacatactct ggtcctgaac tgtaccgcca ccacacctct   1980
   gaataccaga gtgcagatga catggtctta cccagacgag aaaaacaaga gggctagtgt   2040
   ccggagaagg atcgaccagt ctaacagtca cgcaaatatt ttctatagcg tgctgacaat   2100
   cgacaagatg cagaacaaag ataagggcct gtacacttgt cgcgtgcgaa gtgggccttc   2160
   attcaaaagc gtgaatactt ccgtccatat ctatgacaaa gccttcatca ccgtgaaaca   2220
   ccggaagcag caggtgctgg agacagtcgc cgggaaaagg agctaccgcc tgtccatgaa   2280
   agtgaaggct tttccatccc ccgaggtggt ctggctgaaa gatggcctgc cagccacaga   2340
   aaagagcgcc cgatacctga ctcgggggta ttccctgatc attaaggacg tgaccgagga   2400
   agatgcagga aactacacaa tcctgctgag catcaagcag agtaacgtgt tcaagaatct   2460
   gactgccacc ctgattgtga atgtcaaacc ccagatctac gagaaggccg tgagcagctt   2520
   ccctgaccca gcactgtatc ctctgggcag ccggcagatc ctgacatgca ctgcctacgg   2580
   catcccccag cctaccatta agtggttctg gcatccttgt aaccacaatc atagtgaagc   2640
   aaggtgcgat ttctgttcca acaatgagga atcttttatc ctggacgccg atagtaacat   2700
   gggcaatcga atcgagtcaa ttacccagcg gatggctatc attgaaggga aaaacaagat   2760
   ggcatctaca ctggtggtcg ccgactcccg catctctggc atctacatct gcattgcctc 2820
   aaacaaagtg ggaacagtcg gccggaatat cagcttctac attactgatg tgccaaacgg 2880
   atttcacgtc aatctggaga agatgcccac cgagggcgaa gacctgaaac tgtcttgtac 2940
   agtgaataag ttcctgtata gggatgtcac ttggattctg ctgagaactg tgaacaatag 3000
   gaccatgcat tactcaatca gcaaacagaa gatggctatc accaaggaac acagcattac 3060
   actgaacctg actatcatga acgtgagcct ccaggacagc gggacctacg cttgccgggc 3120
   aagaaacgtg tatacaggag aggaaatcct ccagaagaag gagatcacaa ttcgcggcga 3180
   acactgtaac aagaaggccg tgtttagccg aatctccaag ttcaagtcaa ccaggaatga 3240
   ttgtactacc cagtcaaatg tcaagcacta gtaaggatcc agacatgata agatacattg 3300
   atgagtttgg acaaaccaca actagaatgc agtgaaaaaa atgctttatt tgtgaaattt 3360
   gtgatgctat tgctttattt gtaaccatta taagctgcaa taaacaagtt aacaacaaca 3420
   attgcattca ttttatgttt caggttcagg gggagatgtg ggaggttttt taaagcaagt 3480
   aaaacctcta caaatgtggt aaaatcgata aggatctagg aacccctagt gatggagttg 3540
   gccactccct ctctgcgcgc tcgctcgctc actgaggccg cccgggcaaa gcccgggcgt 3600
   cgggcgacct ttggtcgccc ggcctcagtg agcgagcgag cgcgcagaga gggagtggcc 3660
   aacccccccc cccccccccc tgcagcctgg cgtaatagcg aagaggcccg caccgatcgc 3720
   ccttcccaac agttgcgtag cctgaatggc gaatggcgcg acgcgccctg tagcggcgca 3780
   ttaagcgcgg cgggtgtggt ggttacgcgc agcgtgaccg ctacacttgc cagcgcccta 3840
   gcgcccgctc ctttcgcttt cttcccttcc tttctcgcca cgttcgccgg ctttccccgt 3900
   caagctctaa atcgggggct ccctttaggg ttccgattta gtgctttacg gcacctcgac 3960
   cccaaaaaac ttgattaggg tgatggttca cgtagtgggc catcgccctg atagacggtt 4020
   tttcgccctt tgacgttgga gtccacgttc tttaatagtg gactcttgtt ccaaactgga 4080
   acaacactca accctatctc ggtctattct tttgatttat aagggatttt gccgatttcg 4140
   gcctattggt taaaaaatga gctgatttaa caaaaattta acgcgaattt taacaaaata 4200
   ttaacgttta caatttcctg atgcgctatt ttctccttac gcatctgtgc ggtatttcac 4260
   accgcatatg gtgcactctc agtacaatct gctctgatgc cgcatagtta agccagcccc 4320
   gacacccgcc aacacccgct gacgcgccct gacgggcttg tctgctcccg gcatccgctt 4380
   acagacaagc tgtgaccgtc tccgggagct gcatgtgtca gaggttttca ccgtcatcac 4440
   cgaaacgcgc gagacgaaag ggcctcgtga tacgcctatt tttataggtt aatgtcatga 4500
   taataatggt ttcttagacg tcaggtggca cttttcgggg aaatgtgcgc ggaaccccta 4560
   tttgtttatt tttctaaata ctttcaaata tgtatccgct catgagacaa taaccctgat 4620
   aaatgcttca ataatattga aaaaggaaga gtatgagtat tcaacatttc cgtgtcgccc 4680
   ttattccctt ttttgcggca ttttgccttc ctgtttttgc tcacccagaa acgctggtga 4740
   aagtaaaaga tgctgaagat cagttgggtg cacgagtggg ttacatcgaa ctggatctca      4800
   acagcggtaa gatccttgag agttttcgcc ccgaagaacg ttttccaatg atgagcactt      4860
   ttaaagttct gctatgtggc gcggtattat cccgtattga cgccgggcaa gagcaactcg      4920
   gtcgccgcat acactattct cagaatgact tggttgagta ctcaccagtc acagaaaagc      4980
   atcttacgga tggcatgaca gtaagagaat tatgcagtgc tgccataacc atgagtgata      5040
   acactgcggc caacttactt ctgacaacga tcggaggacc gaaggagcta accgcttttt      5100
   tgcacaacat gggggatcat gtaactcgcc ttgatcgttg ggaaccggag ctgaatgaag      5160
   ccataccaaa cgacgagcgt gacaccacga tgcctgtagc aatggcaaca acgttgcgca      5220
   aactattaac tggcgaacta cttactctag cttcccggca acaattaata gactggatgg      5280
   aggcggataa agttgcagga ccacttctgc gctcggccct tccggctggc tggtttattg      5340
   cggataaatc tggagccggt gagcgtgggt ctcgcggtat cattgcagca ctggggccag      5400
   atggtaagcc ctcccgtatc gtagttatct acacgacggg gagtcaggca actatggatg      5460
   aacgaaatag acagatcgct gagataggtg cctcactgat taagcattgg taactgtcag      5520
   accaagttta ctcatatata ctttagattg atttaaaact tcatttttaa tttaaaagga      5580
   tctaggtgaa gatccttttt gataatctca tgaccaaaat cccttaacgt gagttttcgt      5640
   tccactgagc gtcagacccc gtagaaaaga tcaaaggatc ttcttgagat cctttttttc      5700
   tgcgcgtaat ctgctgcttg caaacaaaaa aaccaccgct accagcggtg gtttgtttgc      5760
   cggatcaaga gctaccaact ctttttccga aggtaactgg cttcagcaga gcgcagatac      5820
   caaatactgt ccttctagtg tagccgtagt taggccacca cttcaagaac tctgtagcac      5880
   cgcctacata cctcgctctg ctaatcctgt taccagtggc tgctgccagt ggcgataagt      5940
   cgtgtcttac cgggttggac tcaagacgat agttaccgga taaggcgcag cggtcgggct      6000
   gaacgggggg ttcgtgcaca cagcccagct tggagcgaac gacctacacc gaactgagat      6060
   acctacagcg tgagcattga gaaagcgcca cgcttcccga agggagaaag gcggacaggt      6120
   atccggtaag cggcagggtc ggaacaggag agcgcacgag ggagcttcca gggggaaacg      6180
   cctggtatct ttatagtcct gtcgggtttc gccacctctg acttgagcgt cgatttttgt      6240
   gatgctcgtc aggggggcgg agcctatgga aaaacgccag caacgcggcc tttttacggt      6300
   tcctggcctt ttgctggcct tttgctcaca tgttctttcc tgcgttatcc cctgattctg      6360
   tggataaccg tattaccgcc tttgagtgag ctgataccgc t                          6401
<210> 26
   <211> 687
   <212> PRT
   <213> Homo sapiens
<400> 26
   Met Val Ser Tyr Trp Asp Thr Gly Val Leu Leu Cys Ala Leu Leu Ser
   1               5                   10                  15
   Cys Leu Leu Leu Thr Gly Ser Ser Ser Gly Ser Lys Leu Lys Asp Pro
               20                  25                  30
   Glu Leu Ser Leu Lys Gly Thr Gln His Ile Met Gln Ala Gly Gln Thr
           35                  40                  45
   Leu His Leu Gln Cys Arg Gly Glu Ala Ala His Lys Trp Ser Leu Pro
       50                  55                  60
   Glu Met Val Ser Lys Glu Ser Glu Arg Leu Ser Ile Thr Lys Ser Ala
   65                  70                  75                  80
   Cys Gly Arg Asn Gly Lys Gln Phe Cys Ser Thr Leu Thr Leu Asn Thr
                   85                  90                  95
   Ala Gln Ala Asn His Thr Gly Phe Tyr Ser Cys Lys Tyr Leu Ala Val
               100                 105                 110
   Pro Thr Ser Lys Lys Lys Glu Thr Glu Ser Ala Ile Tyr Ile Phe Ile
           115                 120                 125
   Ser Asp Thr Gly Arg Pro Phe Val Glu Met Tyr Ser Glu Ile Pro Glu
       130                 135                 140
   Ile Ile His Met Thr Glu Gly Arg Glu Leu Val Ile Pro Cys Arg Val
   145                 150                 155                 160
   Thr Ser Pro Asn Ile Thr Val Thr Leu Lys Lys Phe Pro Leu Asp Thr
                   165                 170                 175
   Leu Ile Pro Asp Gly Lys Arg Ile Ile Trp Asp Ser Arg Lys Gly Phe
               180                 185                 190
   Ile Ile Ser Asn Ala Thr Tyr Lys Glu Ile Gly Leu Leu Thr Cys Glu
           195                 200                 205
   Ala Thr Val Asn Gly His Leu Tyr Lys Thr Asn Tyr Leu Thr His Arg
       210                 215                 220
   Gln Thr Asn Thr Ile Ile Asp Val Gln Ile Ser Thr Pro Arg Pro Val
   225                 230                 235                 240
   Lys Leu Leu Arg Gly His Thr Leu Val Leu Asn Cys Thr Ala Thr Thr
                   245                 250                 255
   Pro Leu Asn Thr Arg Val Gln Met Thr Trp Ser Tyr Pro Asp Glu Lys
               260                 265                 270
   Asn Lys Arg Ala Ser Val Arg Arg Arg Ile Asp Gln Ser Asn Ser His
           275                 280                 285
   Ala Asn Ile Phe Tyr Ser Val Leu Thr Ile Asp Lys Met Gln Asn Lys
   290 295 300
   Asp Lys Gly Leu Tyr Thr Cys Arg Val Arg Ser Gly Pro Ser Phe Lys
   305 310 315 320
   Ser Val Asn Thr Ser Val His Ile Tyr Asp Lys Ala Phe Ile Thr Val
   325 330 335
   Lys His Arg Lys Gln Gln Val Leu Glu Thr Val Ala Gly Lys Arg Ser
   340 345 350
   Tyr Arg Leu Ser Met Lys Val Lys Ala Phe Pro Ser Pro Glu Val Val
   355 360 365
   Trp Leu Lys Asp Gly Leu Pro Ala Thr Glu Lys Ser Ala Arg Tyr Leu
   370 375 380
   Thr Arg Gly Tyr Ser Leu Ile Ile Lys Asp Val Thr Glu Glu Asp Ala
   385 390 395 400
   Gly Asn Tyr Thr Ile Leu Leu Ser Ile Lys Gln Ser Asn Val Phe Lys
   405 410 415
   Asn Leu Thr Ala Thr Leu Ile Val Asn Val Lys Pro Gln Ile Tyr Glu
   420 425 430
   Lys Ala Val Ser Ser Phe Pro Asp Pro Ala Leu Tyr Pro Leu Gly Ser
   435 440 445
   Arg Gln Ile Leu Thr Cys Thr Ala Tyr Gly Ile Pro Gln Pro Thr Ile
   450 455 460
   Lys Trp Phe Trp His Pro Cys Asn His Asn His Ser Glu Ala Arg Cys
   465 470 475 480
   Asp Phe Cys Ser Asn Asn Glu Glu Ser Phe Ile Leu Asp Ala Asp Ser
   485 490 495
   Asn Met Gly Asn Arg Ile Glu Ser Ile Thr Gln Arg Met Ala Ile Ile
   500 505 510
   Glu Gly Lys Asn Lys Met Ala Ser Thr Leu Val Val Ala Asp Ser Arg
   515 520 525
   Ile Ser Gly Ile Tyr Ile Cys Ile Ala Ser Asn Lys Val Gly Thr Val
   530 535 540
   Gly Arg Asn Ile Ser Phe Tyr Ile Thr Asp Val Pro Asn Gly Phe His
   545                 550                 555                 560
   Val Asn Leu Glu Lys Met Pro Thr Glu Gly Glu Asp Leu Lys Leu Ser
                   565                 570                 575
   Cys Thr Val Asn Lys Phe Leu Tyr Arg Asp Val Thr Trp Ile Leu Leu
               580                 585                 590
   Arg Thr Val Asn Asn Arg Thr Met His Tyr Ser Ile Ser Lys Gln Lys
           595                 600                 605
   Met Ala Ile Thr Lys Glu His Ser Ile Thr Leu Asn Leu Thr Ile Met
       610                 615                 620
   Asn Val Ser Leu Gln Asp Ser Gly Thr Tyr Ala Cys Arg Ala Arg Asn
   625                 630                 635                 640
   Val Tyr Thr Gly Glu Glu Ile Leu Gln Lys Lys Glu Ile Thr Ile Arg
                   645                 650                 655
   Gly Glu His Cys Asn Lys Lys Ala Val Phe Ser Arg Ile Ser Lys Phe
               660                 665                 670
   Lys Ser Thr Arg Asn Asp Cys Thr Thr Gln Ser Asn Val Lys His
           675                 680                 685